# EUROPEAN PATENT APPLICATION

(11) **EP 2 157 095 A2**
(43) Date of publication of application: **24.02.2010**
(21) Application number: 09075403.7
(22) Date of filing: 30.06.2004
(51) Int. Cl.: C07H 9/04, C07H 9/06, C07H 13/04, C07H 13/08, C07H 15/203, C07H 19/06, C07H 19/09, C07H 19/073

(54) **Synthesis of beta-L-2-Deoxy nucleosides**

(30) Priority: 30.06.2003 US 483711 P; 01.04.2004 US 558616 P
(62) Divisional of application: 04777433.6
(71) Applicant: Novartis AG, 4056 Basel (CH)
(72) Inventor: Storer, Richard, Folkstone, Kent, CT20 2JE (GB); Moussa, Adel, Burlington, MA 01803 (US); Wang, Jing Yang, Acton, MA 01718 (US); Chaudhuri, Narayan, Acton, MA 01720 (US); Mathieu, Steven, Windham, NH 03087 (US); Stewart, Alistair, Somerville, MA 02144 (US)
(74) Representative: Hallybone, Huw George

(57) **Abstract**

An improved process for the preparation of 2'-modified nucleosides and 2'-deoxynucleosides, such as, β-L-2'-deoxy-thymidine (LdT), is provided. In particular, the improved process is directed to the synthesis of a 2'-deoxynucleoside that may utilize different starting materials but that proceeds via a chloro-sugar intermediate or via a 2,2' anhydro-1-furanosyl-nucleobase intermediate. Where an 2,2'-anhydro-1-furanosyl base intermediate is utilized, a reducing agent, such as Red-Al, and a sequestering agent, such as 15-crown-5 ether, that cause an intramolecular displacement reaction and formation of the desired nucleoside product in good yields are employed. An alternative process of the present invention utilizes a 2,2'-anhydro-1-furanosyl base intermediate without a sequestering agent to afford 2'-deoxynucleosides in good yields. The compounds made according to the present invention may be used as intermediates in the preparation of other nucleoside analogues, or may be used directly as antiviral and/or antineoplastic agents.

## Description

### CROSS-REFERENCE TO RELATED APPLICATIONS

This invention claims priority to U.S. Provisional No. 60/483,711, filed June 30, 2003, and 60/558,616, filed April 1, 2004.

### FIELD OF THE INVENTION

This invention is in the field of processes for preparing 2'-deoxy- or 2'-modified-nucleosides and particularly β-L-2'-deoxythymidine. The present invention is an improved process that is easily scalable for purposes of industrial manufacture. The compounds prepared according to the process of the present invention are important as antiviral agents, antineoplastic agents, and intermediates in the synthesis of pharmaceutical compounds and compositions.

### BACKGROUND OF THE INVENTION

HBV is second only to tobacco as a cause of human cancer. The mechanism by which HBV induces cancer is unknown, although it is postulated that it may directly trigger tumor development, or indirectly trigger tumor development through chronic inflammation, cirrhosis, and cell regeneration associated with the infection.

Hepatitis B virus has reached epidemic levels worldwide. After a two to six month incubation period in which the host is unaware of the infection, HBV infection can lead to acute hepatitis and liver damage, that causes abdominal pain, jaundice, and elevated blood levels of certain enzymes. HBV can cause fulminant hepatitis, a rapidly progressive, often fatal form of the disease in which massive sections of the liver are destroyed.

Patients typically recover from acute hepatitis. In some patients, however, high levels of viral antigen persist in the blood for an extended, or indefinite, period, causing a chronic infection. Chronic infections can lead to chronic persistent hepatitis. Patients infected with chronic persistent HBV are most common in developing countries. By mid-1991, there were approximately 225 million chronic carriers of HBV in Asia alone, and worldwide, almost 300 million carriers. Chronic persistent hepatitis can cause fatigue, cirrhosis of the liver, and hepatocellular carcinoma, a primary liver cancer.

WO 96/40164 filed by Emory University, UAB Research Foundation, and the Centre National de la Recherche Scientifique (CNRS) discloses a number of β-L-2',3'-dideoxynucleosides for the treatment of hepatitis B.

WO 95/07287 also filed by Emory University, UAB Research Foundation, and the Centre National de la Recherche Scientifique (CNRS) discloses 2'- or 3'-deoxy and 2',3'-dideoxy-β-L-pentofuranosyl nucleosides for the treatment of HIV infection.

WO96/13512 filed by Genencor International, Inc., and Lipitek, Inc., discloses the preparation of L-ribofuranosyl nucleosides as antitumor agents and virucides.

Idenix Pharmaceuticals, Ltd. discloses 2'-deoxy--L-erythropentofurano-nucleosides, and their use in the treatment of HBV in US Patent Nos. 6,395,716; 6,444,652; 6,566,344 and 6,539,837. See also WO 00/09531. A method for the treatment of hepatitis B infection in humans and other host animals is disclosed that includes administering an effective amount of a biologically active 2'-deoxy-β-L-erythro-pentofuranonucleoside (alternatively referred to as β-L-dN or a β-L-2'-dN) or a pharmaceutically acceptable salt, ester or prodrug thereof, including β-L-deoxyribothymidine (β-L-dT), β-L-deoxyribocytidine (β-L-dC), β-L-deoxyribouridine (β-L-dU), β-L-deoxyribo-guanosine (β-L-dG), β-L-deoxyriboadenosine (β-L-dA) and β-L-deoxyriboinosine (β-L-dI), administered either alone or in combination, optionally in a pharmaceutically acceptable carrier. The 5' and N⁴ (cytidine) or N⁶ (adenosine) acylated or alkylated derivatives of the active compound, or the 5'-phospholipid or 5'-ether lipids were also disclosed.

von Janta-Lipinski et al. J. Med. Chem., 1998, 41 (12), 2040-2046 disclose the use of the L-enantiomers of 3'-fluoro-modified β-2'-deoxyribonucleoside 5'-triphosphates for the inhibition of hepatitis B polymerases. Specifically, the 5'-triphosphates of 3'-deoxy-3'-fluoro-β-L-thymidine (β-L-FTTP), 2',3'-dideoxy-3'-fluoro-β-L-cytidine (β-L-FdCTP), and 2',3'-dideoxy-3'-fluoro-β-L-5-methylcytidine (β-L-FMethCTP) were disclosed as effective inhibitors of HBV DNA polymerases. In addition, von Janta-Lipinski et al. discloses the biological activity of the triphosphate of β-L-thymidine (but not β-L-2'-dC) as a nucleoside inhibitor of endogenous DNA polymerases of HBV and DHBV. However, only triphosphorylated β-L-thymidine was evaluated, not the claimed unphosphorylated form, and there is no comment in the article on whether those β-L-nucleosides are phosphorylated in cells or *in vivo* or, more importantly, there is no comment on the efficacy of phosphorylation of β-L-thymidine *in vivo*. Because of this, the article does not teach that β-L-thymidine would have any hepatitis B activity in a cell or *in vivo*. See also WO 96/1204.

European Patent Application No. 0 352 248 A1 to Johansson et al. discloses the use of L-ribofuranosyl compounds for the treatment of hepatitis B.

Verri et al. disclose the use of 2'-deoxy-β-L-erythro-pentofuranonucleosides as antineoplastic agents and as anti-herpetic agents (Mol. Pharmacol. (1997), 51(1), 132-138 and Biochem. J. (1997), 328(1), 317-20). Saneyoshi et al. demonstrate the use of 2'-deoxy-L-ribonucleosides as reverse transcriptase (I) inhibitors for the control of retroviruses and for the treatment of AIDS, Jpn. Kokai Tokkyo Koho JP06293645 (1994).

Giovanni et al. tested 2'-deoxy-β-L-erythro-pentofuranonucleosides against partially pseudorabies virus (PRV), Biochem. J. (1993), 294(2), 381-5.

Chemotherapeutic uses of 2'-deoxy-β-L-erythro-pentofuranonucleosides were studied by Tyrsted et al. (Biochim. Biophys. Acta (1968), 155(2), 619-22) and Bloch, et al. (J. Med. Chem. (1967), 10(5), 908-12).

Morris S. Zedeck et al. first disclosed β-L-dA for the inhibition of the synthesis of induced enzymes in Pseudomonas testosteroni, Mol. Phys. (1967), 3(4), 386-95.

In addition, cytosine derivatives are useful as intermediates for production of drugs such as cytidine diphosphate choline whose generic name is Citicoline.

US Patent Publication No. 20030083306 to Idenix Pharmaceuticals, Ltd. discloses 3'-prodrugs of 2'-deoxy-β-L-nucleosides for the treatment of HBV. See also WO 01/96353.

U.S. Patent No. 4,957,924 to Beauchamp discloses various therapeutic esters of acyclovir.

In the April 17-21, 2002 European Association for the Study of the Liver meeting in Madrid, Spain, Sühnel et al. of Gilead Sciences, Inc. presented a poster indicating that combinations of adefovir with β-L-2'deoxythymidine produce additive antiviral effects against HBV *in vitro.*

### Nucleoside Synthesis

Processes for preparing nucleosides and furanosyl intermediates are well known in the prior art. In 1952, Pratt et al. reported the synthesis of L-deoxythymidine (LdT) from arabinose (J.W. Pratt et al., J. Am. Chem. Soc., 1952, 74:2200-2205). The synthetic route disclosed by Pratt included the formation of a methyl glycoside from L-arabinose, with subsequent conversion to methylthio-thiocarbonate, and reduction to the deoxy-sugar. Alternatively, the 2-hydroxy group was converted to its corresponding mesylate group, which then was subjected to a reductive cleavage in order to provide the final LdT product (J.W. Pratt et al., J. Am. Chem. Soc., 1952, 74:2200-2205; H. Urata et al., Nucleic Acids Res., 1992, 20:3325-3332).

Variations in the synthesis of LdT were made by Shull et al., Sznaidman et al., Wang et al., and Stick et al., each of whom converted L-arabinose to methyl 2'-deoxy-ribofuranoside via a glycal intermediate (B.K. Shull et al., J. Carbohydr. Chem., 1996, 15:955-64; M.L. Sznaidman et al., Nucleosides, Nucleotides & Nucleic Acids, 2002, 21:155-63; Z.X. Wang et al., Nucleosides, Nucleotides & Nucleic Acids, 2001, 20:11-40; and R.V.Stick et al, Aust. J. Chem., 2002, 55:83-85).

In 1969 Niedballa and Vorbruggen described a process for preparing β-nucleosides by coupling a silylated N-heterocyclic compound and, in particular a pyrimidine, with a 1-O-alkyl or preferably a 1-acyl-protected sugar such as a 1-acyl-protected ribose, deoxyribose, arabinose or glucose. The reaction utilized a Friedel-Crafts reagent as a catalyst and proceeded at ambient temperatures (DE 1 919 307 to Schering Aktiengesellschaft). The inventors noted that this process surprisingly provided the β-anomeric product almost exclusively, and would work for curacil and cytosine but not as well for thymidine (DE 1 919 307, Examples 1-10 and 12-15).

In their exemplified species, Niedballa and Vorbruggen reported only 1-O-acetyl, 1-acetyl, and 1-O-methyl ribose, deoxyribose and arabinofuranose derivative compounds as starting reagents (DE 1 191 307, Examples 1-16). Nowhere was a 1-halo sugar used. In fact, the inventors noted that use of a 1-halo sugar as a reactant was not favored based upon its instability (DE 1 191 307; JP 63026183 to Sato et al.). In the single example where a cytosine base was reacted with a 2'-deoxyribose sugar, the starting compound was 1-O-methyl-2-desoxy-3,5-di-toluylribose (DE 1 919 307, Example 7). It is not surprising that this reaction formed the β-anomer to the near exclusion of the α-anomer, because it is known that 3'-ester derivatives of ribose normally form the β-anomer in preference to the α-anomer product.

In subsequent patents, Vorbruggen et al. referred to their earlier (1969) synthetic method as being "particularly disadvantageous", because the separation of the Lewis acid salts or Friedel-Crafts catalysts formed during the reaction resulted in the need for numerous, labor-intensive steps in the final work-up, and provided lower per cent yields of the final product (DE 2508312, British equivalent GB 1 542 442). In GB 1 542 442, the process replacement of Lewis acids by trimethylsilyl esters of mineral acids and starting reagents that were a 1-halo, 1-O-alkyl, or 1-O-acyl sugar, were reported. As before, all exemplified species utilized a 1-O-acetyl-β-D-ribofuranose starting reagent, and so, not surprisingly, produced the β-anomeric product to the near exclusion of the α-anomer (GB 1 542 442, Examples 1-13).

Likewise, in U.S. 4,209,613, Vorbruggen disclosed a single step nucleoside synthesis that included reacting a silylated nucleoside base with a 1-O-acyl, 1-O-alkyl, or 1-halo derivative of a protected sugar in the presence of a Friedel-Crafts catalyst selected from any of a group of Lewis acids (U.S. 4,209,613). As before, all exemplified species utilized a 1-O-acetyl-β-D-ribofuranose starting reagent, and again, not surprisingly, produced the β-anomeric product to the near exclusion of the α-anomer (U.S. 4,209,613, Examples 1-16).

In U.S. 5,750,676, Vorbruggen et al. reported a process that comprised reacting a free sugar with an N-heterocyclic base in the presence of a silylating agent and an inert solvent having a Lewis acid, wherein the improvement resided in the persilylation of the free sugar. No comment was made regarding product anomeric ratios, and no preference for a single Lewis acid was stated. However, the examples indicated that numerous preparatory steps were required in order to obtain the final products, a distinct disadvantage for industrial scalability (U.S. 5,750,676, Examples 1-3).

Yet another process for preparing nucleosides reported by Vorbruggen et al. included a one-pot synthesis utilizing a trialkylsilyl ester of an inorganic or strong organic acid, especially a Friedel-Crafts catalyst, a nucleoside base, and a 1-O-acyl, 1-O-alkyl, or 1-halo derivative of a protected sugar derivative (U.S. 4,209,613).

### Chloro-Sugar Intermediate

Chloro-sugar is an important intermediate in the formation of LdT, and numerous routes to its synthesis exist. Nonlimiting examples of syntheses for making chloro-sugars include the following.

Isbell, Bock et al., and Lundt et al. each reported the synthesis of LdT from D-xylose in a process that involved a 1,4-lactone intermediate (H.S. Isbell, Methods in Carbohydrate Research, 1963, 2:13-14; K. Bock et al., Carbohydrate Research, 1981, 90:17-26; K. Bock et al., Carbohydrate Research, 1982, 104:79-85; and I. Lundt and R. Madsen, Topics in Current Chemistry, 2001, 215:177-191).

Bock et al. and Humphlett utilized D-galactose as a starting material, which was oxidatively cleaved and brominated to produce D-lyxonolactone. Subsequent steps of selective hydrolysis and transformations provided a chloro-sugar intermediate that could then be used to prepare LdT (K. Bock et al., Carbohydrate Research, 1981, 90:17-26; K. Bock et al., Carbohydrate Research, 1979, 68:313-319; K. Bock et al., Acta Chem. Scand. B, 1984, 38:555-561; and W.J. Humphlett, Carbohydrate Research, 1967, 4:157-164).

Bock et al. also prepared LdT from D-gluconolactone by treating the latter with aqueous bromine and hydrazine, and then with excess aqueous potassium hydroxide to form a primary epoxide. Next, they effected a Payne rearrangement of the primary epoxide to a secondary epoxide on the lactone, and oxidatively cleaved the lactone to form a chloro-sugar intermediate, which then could be used to prepare LdT (K. Bock et al., Carbohydrate Research, 1979, 68:313-316; K. Bock et al., Acta Chem. Scand B, 1984, 38:555-561). In the same journal articles referenced, Bock et al. disclosed the formation of a chloro-sugar from D-galactonolactone, and by the bromination of D-mannono-1,4-lactone.

Liotta and Hager reported the synthesis of a chloro-sugar from a commercially available lactone in a synthesis that involved a stereoselective cyclization step, as well as a synthesis that utilized an aldehyde intermediate and the Horner-Emmons modification of the Wittig reaction (D.C. Liotta et al., Tetrahedron Letters, 1992, 33:7083-7086; and U.S. 5,414,078).

Schinazi et al., Ravid et al., and Taniguchi et al. disclosed processes for preparing chloro-sugar intermediates from hydroxy glutamic acid, which is cyclized to a ribonolactone derivative that can then be converted to a chloro-sugar (U.S. 6,348,587 B1 to R.F. Schinazi et al.; U. Ravid et al., Tetrahedron, 1978, 34:1449-1452; and M. Taniguchi et al., Tetrahedron, 1974, 30:3547-3552).

Jung et al. reported using a Sharpless epoxidation on a commercially available alcohol to provide an epoxide that was then treated with alcohol to prepare a diol, which was then converted to an acetonide. The acetonide was acidified to give the desired ribofuranose, which was then converted to a chloro-sugar. Alternatively, an epoxyalcohol was subjected to hydroboration using the Swern oxidation, and the cloro-sugar was formed from a di-toluoyl derivative (M.E. Jung et al., Tetrahedron Letters, 1998, 39:4615-4618).

Yadav et al. and Harada et al. disclosed syntheses that employed allyl bromide and ozonolysis, or 2-bromomethyl-[1,3]-dioxolane without ozonolysis, to prepare chloro-sugars (J.S. Yadav et al., Tetrahedron Letters, 2002, 43:3837-3839; T. Harada et al., Chem. Lett., 1981, 1109-1110), while Ohuri et al., Cheng et al., and Abramski et al. reported treating a glycal with acidic methanol to produce 2-deoxy-ribofuranose that could then be converted to the desired chloro-sugar.

JP 09059292 to Takeya Mori disclosed a one-pot synthesis of a 4-aminopyrimidine nucleoside from a 4-hydroxypyrimidine nucleoside by protection of the reactant's hydroxy groups with trimethylsilyl groups, subsequent reaction with phosphorus oxychloride or 4-chlorophenyl phosphorodichloridate, and amination with aqueous ammonia.

Chu reported a process for preparing 2'-deoxynucleosides that included reacting a nucleoside having 2'- and 3'-hydroxyl groups with a mixture of acyl bromide or acyl chloride and hydrobromic or hydrochloric acid at moderate temperatures to provide a haloacyl nucleoside derivative that was deprotected to form a desired nucleoside product (U.S. 5,200,514).

In Nucleosides and Nucleotides, 1996, 15(1-3):749-769, Kamaike et al. disclosed the formation of 2'-deoxyribonucleosides via nucleophilic substitution reactions of 4-azolyl-1-β-D-ribofuranosyl-pyrimidin-2(1*H*)-one converted from uridine with [¹⁵N]phthalimide in the presence of triethylamine or DBU to give *N*⁴-phthaloyl[4-¹⁵N]cytidine in high yields.

JP 71021872 to Sankyo Co. Ltd. taught the reaction of a silylated cytosine, uracil, thymine or azauracil base with a sugar halide, such as a halogenized ribose or glucose, in the presence of a solvent and mercuric halide.

### D-Xylose

Utilizing D-xylose as a starting material, 2'-deoxynucleosides can be synthesized according to methods taught in the prior art.

Okabe et al. disclosed a synthesis of 2-deoxy-3,5-di-O-p-toluoyl-α-L-erythro-pentofuranosyl chloride, which may be further reacted to produce β-L-2'-deoxythymidine (LdT) (Okabe et al., J. Org. Chem., 1991, 56(14):4392; Bock et al., Carbohydr. Res., 1981, 90:17-26; Bock et al., Carbohydr. Res., 1982, 104:79-85).

The following is a non-limiting list of processes used to prepare intermediates in the synthesis of 2'-deoxynucleosides and 2'-deoxythymidine in particular, from D-xylose.

Takahata et al. and Graf et al. reported the formation of 2,5-dibromo-2,5-dideoxy-D-lyxo-1,4-lactone by reaction of the lyxo-1,4-lactone with potassium iodide in acetone (Takahata et al., J. Org. Chem., 1994, 59:7201-7208; Graf et al., Liebigs Ann. Chem., 1993, 1091-1098).

Lundt et al., Bock et al., and Choi et al. disclosed the inversion of 5-bromo-2,5-dideoxy-D-threo-pentono-1,4-lactone to form 2-deoxy-L-ribono-1,4-lactone (Lundt et al., Topics in Current Chemistry, 2001, 215:177-191; Bock et al., Carbohydr. Res., 1981, 90:17-26; WO 01/72698 to Y-R.Choi et al.).

Urata et al. and Zhang et al. reported the conversion of 2-deoxy-3,5-di-O-toluoyl-α,β-L-ribose to 2-deoxy-3,5-di-O-*para*-toluoyl-α-L-erythro-pentofuranosyl chloride either directly from lactol by reaction with hydrochloric and acetic acids, or indirectly via a 2-deoxy-7-methoxy-3,5-di-O-toluoyl-α,β-L-ribose intermediate by reaction with acetic and hydrochloric acids (H. Urata et al., Nucleic Acids Res., 1992, 20(13):3325-3332; Zhang et al., Nucleosides and Nucleotides, 1999, 18(11-12):2357.

Urata et al. also disclosed the preparation of 2'-deoxy-3',5'-di-O-*para*-toluoyl L-thymidine from 2-deoxy-3,5-di-O-*p*-toluoyl-α-L-erythro-pentofuranosyl chloride and silylated thymine in the presence of chloroform, followed by deprotection to form 2'-L-deoxythymidine (H. Urata et al., Nucleic Acids Res., 1992, 20(13):3325).

### 2,2'-Anhydro-1-Furanosyl Nucleoside Intermediate

2'-Deoxy- and 2'-substituted nucleosides, and particularly 2'-deoxy- or 2'-substituted nucleosides that have pyrimidine bases, have been shown to stabilize oligonucleotides against nuclease degradation. Nuclease degradation is a problem in the field of oligonucleotide therapeutics (Huryn et al., (1992), Chem. Rev. 92:1745-88; English et al., (1991), Angew. Chem. 30:613-722). However, to date, modification of pyrimidine nucleosides at the 2'-position has been accomplished only under harsh conditions and by syntheses that are inefficient with generally low product yields (Verheyden et al., (1971), J. Org. Chem. 36:250-254).

Tronchet et al. disclosed a reduction of the oxime derivative of 2'-ketouridine by BH₃ that provides predominantly 2'-hydroxy- or 2'-amino-nucleosides in the arabino-configuration (Tronchet et al., (1990), Tetrahedron Lett. 31:351). This work by Tronchet is one of only a few attempts at stereo-selective synthesis of 2'-ribofuranosyl-amino or 2'-ribofuranosyl-hydroxyl pyrimidines.

Early approaches at syntheses of 2'-deoxy- or 2'-substituted pyrimidine nucleosides focused on appropriate protective groups for ribose, xylose and arabinose that were the starting reagents in the syntheses. For example, numerous approaches to the synthesis of peracylated ribofuranose as an intermediate in processes for preparing nucleosides were attempted. These included i) a 7-step stereospecific process that started with D-ribose and provided β-D-2'-deoxyribofuranosyl thymidine in an approximate 40% final product yield (M. Jung and Y. Xu, Tetrahedron Lett. (1997), 38:4199); ii) a 3-step process starting from L-ribose and resulting in a 56% product yield (E.F. Recondo and H. Rinderknecht, Helv. Chim. Acta, (1959) 42:1171; iii) an 8-step process utilizing L-arabinose as a starting material and providing about a 20% product yield (J. Du et al., Nucleosides and Nucleotides, (1999), 18:187; iv) a 6-step process starting with L-xylose (% yield of final product unknown) (E. Moyroud and P. Strazewski, Tetrahedron (1999) 55:1277; and v) a multi-step process beginning with D-ribose that initially was converted to tri-O-acetyl thymidine (U.S. Patent No. 4, 914, 233).

In 1959, E.F. Recondo reported a 5-step process for preparing toluoyl-, benzoyl-and acetyl-protected ribofuranosyl in an approximate 70-80% yield from D-ribose (E.F. Recondo, Helv. Chim. Acta, (1959) 121:1171). Codington, Doerr and Fox disclosed the synthesis of 2,2'-anhydro-1-(5-*O*-trityl-β-D-arabinofuranosyl)thymine from β-D-thymidine by reacting β-D-thymidine with tritylchloride and pyridine for 24 hours at room temperature, and then at about 70°C. for 3 hours, to protect the 5'-OH on β-D-thymidine; then reacting 5'-protected β-D-thymidine with tosyl chloride (TsCl) and pyridine at 0 °C, which provides a tosyl-protected 2'-group; and finally reacting the 5'-trityl-O-protected, 2'-tosyl-O-protected- β-D-thymidine with sodium benzoate (NaOBz) and acetamide at 100°C for 1 hour to provide 2,2'-anhydro-1-(5'-*O*-trityl- β-D-arabinofuranosyl)thymine in 61% yield (Codington et al., J. Org. Chem., (1963) 29:558-64).

Enzymatic synthesis of β-D-thymidine was reported using E.coli and hypoxanthine in a first step, and reacting the resulting 2-mono-phosphorylated ribofuranosyl compound with uridine phosphorylase and recovering the desired β-D-thymidine product in 45% yield by column chromatography (A. I. Zinchenko, Khimiya Prirodnykh Soedinenii, (1989), 4:587-88).

Another approach to nucleoside synthesis involved formation of a 5-methyl-2,2'-anhydrouridine intermediate from an "open nucleoside". The open nucleoside is formed by an intramolecular nucleophilic displacement reaction that provides 2,2'-anhydro-1-(β-D-arabino-furanosyl)nucleoside from the ring opening of 2,2'-anhydronucleoside. Synthesis of anhydronucleosides was described in Japanese Kokai No. 81 49 398 (laid open on 2 May 1981), which required as an intermediate, an acylated iminoarabino[1', 2':4,5] oxazoline acid addition salt. The use of an available amino-oxazoline carbohydrate derivative as an anhydronucleoside precursor was reported in 1971 (J. Mol. Biol., (1970) 47:537).

Rao et al. reported a 6-step synthesis that utilized D-xylose as a starting reagent to form 1-β-D-xylofuranosyl-thymine, which then was treated with PhOCOOPh (diphenylcarbonate) and NaHCO₃ catalyst in the presence of DMF at 140-150 °C for about 4 hours to provide 2,2'-anhydro-1-(β-arabinofuranosyl)thymine in 55% yield (A.V. Rama Rao et al., J. Chem. Soc. Comm., (1994), p.1255; EP 0 683 171 B1). Both Schinazi et al. and Manfredi et al. disclosed a synthesis similar to that of Rao et al. that employed the same reagents except for utilizing 1-β-D-arabinofuranosyl thymine rather than 1-β-D-xylofuranosyl thymine (Schinazi et al., J. Med. Chem., (1979) 22:1273; Manfredi et al., Bioorg. Med. Chem. Letters, (2001) 11:1329-32).

An early attempt at formation of 3', 5'-dibenzoyl protected 2,2'-anhydro-1-(β-ribonofuranosyl)thymine was taught by Anton Holy et al. Holy et al. used β-D-ribonofuranosyl-thymine as a starting compound, reacted it with 1.4 eq. of PhOCOOPh and NaHCO₃ catalyst in HMPA for approximately 20 minutes at about 150 °C to form 2,2'-anhydro-1-(β-D-ribofuranosyl)thymine (5-methyluridine), which was reacted with PhCOCN in DMF to protect the 3'- and 5'-OH groups by forming 2,2'-anhydro-1-(β-3',5'-diO-benzoyl)ribofuranosyl thymine in approximately 87% yield (A. Holy et al., Collect. Czech. Commun., (1974), 39:3157-67). Holy et al. also--reported the unsuccessful attempt to convert 2-amino-β-D-arabinofurano-[1', 2':4,5]-2-oxazoline into O², 2'-anhydro-1-(β-D-arabinofuranosyl)thymine (Id. at 1377).

Fraser et al. improved upon Holy's process by using the same starting reagent and reacting it with 1.2 eq. of PhOCOOPh and NaHCO₃ catalyst in the presence of HMPA at about 150°C for about 2 hours to provide 2,2'-anhydro-1-β-D-ribofuranosyl thymine. However, the process of Fraser et al. produced a decreased percent yield of product of about 77% as compared to about 87% yield given in the Holy et al. synthesis (Allister Fraser et al., J. Heterocycl. Chem., (1993) 30(5):1277-88).

Yukio Aoyama et al. disclosed formation of a silyl-protecting ring that embraces both the 3'- and 5'-positions on β-1-D-(2-Br-ribofuranosyl) thymine in about 96% yield (Aoyama et al., Nucleosides & Nucleotides, (1996), 15(1-3):733-8). 1-β-D-ribofuranosyl-thymine was used as a starting material and was reacted with TPDSCl₂ and pyridine at room temperature to provide the 3'-, 5'-silyl- protected ring structure. Next the silyl-protected structure was reacted with TfCl and DMAP in CH₂Cl₂ at room temperature to form the 2,2'-anhydro intermediate, and finally reacting the 2,2'-anhydro intermediate with LiBr, BF₃-OEt in 1,4-dioxane at about 60 °C to afford the final product, 1-β-D-2'-Br, 3',5'-tri-O-di-(dimethyl)silyl)-ribofuranosyl-thymine.

Mitsui Chemicals, Inc., reported methods for preparing 2,2'-anhydro-1-(β-L-arabinofuranosyl)thymine and 2,2'-anhydro-5,6-dihydrocyclouridine, which are useful as intermediates in the synthesis of L-nucleic acids (PCT Publication No. WO 02/044194; EP 1 348 712 A1). The 7-step Mitsui process includes: a) reacting L-arabinose with cyanamide to provide L-arabino-amino-oxazoline; b) reacting L-arabinoaminooxazoline with an acrylic acid derivative to form a derivative of the L-arabinoaminooxazoline having a methyl acrylic acid ester bound to the N-atom of the oxazoline moiety; c) reacting the product of the (b) with a base such as, for example, an alkali metal, alkali metal alkoxide, alkali metal carbonate, alkali metal bicarbonate, alkali metal hydroxide, alkali metal hydride, organic base, base ion exchange resin, and the like, any of which thereby form a tricyclic ring that is an L-2,2'-anhydro-nucleic acid derivative; d) isomerizing the L-2,2'-anhydro-nucleic acid derivative from step (c) to provide 2,2'-anhydro-1-( β-L-arabinofuranosyl)thymine; e) subjecting the 2,2'-anhydro-1-( β-L-arabinofuranosyl)thymine from step (d) either to halogenation and subsequent protection, or to protection and subsequent halogenation, or to simultaneous halogenation and protection, to form a 2'-position halogenated L-thymidine derivative; f) dehalogenating the halogenated L-thymidine derivative from step (e); and g) deblocking the 3'- and 5'-positions of the product from step (f) to provide L-thymidine. While Mitsui reported good product yields from this synthesis, it is desireable to have a process that requires fewer steps so that it is more easily adapted to large scale production for industry.

A second, closely related process found in the prior art is that reported by Pfizer in EP 0 351 126 B1. Pfizer's process included a new route to the formation of O², 2'-anhydro-1-(β-D-arabinofuranosyl)thymine nucleosides (anhydronucleosides), which can easily be converted to β-thymine derivatives. The process includes a condensation reaction between 2-amino- β-D-arabinorurano[1',2':4,5]-2-oxazoline, or a 5'-trityl- or silyl-protected form thereof, preferably with methyl-2-formylpropionate in H₂O and NaOH at pH 8.1 for 48 hours at room temperature, followed by treatment with aqueous acid to afford the O²,2'-anhydro-1-(β-D-arabinofuranosyl)thymine in approximately 42% yield. Alternatives to using methyl-2-formylpropionate include the use of methyl-3-bromomethylacrylate in the presence of DMAP and Et₃N at about 80 °C for 4 days, which provided an approximate 25% yield of final anhydro-thymidine product; the use of ethyl-2-formylpropionate in aqueous MeOH and Et₃N at room temperature for about 24 hours and then at about 60 °C for another 24 hours for an approximate 8% yield of the anhydro-thymidine product; and the use of methyl-3-methoxymethacrylate in DMSO at about 80 °C for 4 days to provide the anhydro-thymidine product in approximately 32% yield.

The Pfizer condensation reaction includes the use of basic catalysts in its preferred embodiment. Such catalysts are tertiary amines and inorganic salts, and preferred among these are dimethylaminopyridine, triethylamine, N-methylmorpholine, and combinations thereof. Pfizer reported that its preferred method for converting O²,2'-anhydro-1-(β-arabinofuranosyl)thymine to β-thymidine was by reaction of the anhydrothymidine with HBr, followed by the removal of Br by reaction with BaSO₄-poisoned Pd catalyst. It is desireable to have an industrially-scalable synthesis that would eliminate the need for using a poisoned catalyst of this type.

Boehringer-Ingelheim Pharma GMBH reported a 4-step process for preparing β-L-2'-deoxythymidine that used L-arabinose as a starting-material (PCT Publication No. WO 031087118). The process comprised a) reacting L-arabinose with cyanamide in aqueous or aqueous alcohol solution, or in another polar solvent such as, for example, DMF, pyridine, or N-methyl-pyrrolidine, at a temperature of from 80-100 °C, in the presence of a base catalyst, such as NH₃, Et₃N, or tri-ethyl carbonate, alkali carbonate, or di-alkali carbonate, to form an L-arabinofuranosyl-amino-oxazoline derivative; b) reacting the L-arabinofuranosyl-amino-oxazoline derivative from step (a) with a 2-methyl-C-3-acid, or an activated derivative thereof, in inert solvent under water-precipitating conditions such as, for example, in the presence of DMF, DMSO, NMP, acetone, benzene, toluene, or cyclohexane, and a tertiary amine base or inorganic salt catalyst like DMAP, Et₃N, or N-methyl-morpholine at about 20-80 °C; c) reacting the β-L-2,2'-anhydrothymidine from step (b) with a nucleophilic reagent such as an acidic-halogen like HCl, HI, or HBr, toluene sulfonic acids or thioacetic acid, in DMF or trifluoroacetic acid solvent, to rupture the C-O bond at the 2'-position; and d) reacting the β-L-2'-halo-thymidine with a catalyst, preferably either Pd or Raney-Nickel, to remove the halo group from the 2'-position and to provide β-L-thymidine as a final product.

Preferably, prior to performing steps (a) or (b) of the synthesis, any free hydroxyl groups are protected to prevent their reaction with the amino-oxazoline derivative, or with the 2-methyl-C-3-acid.

In this Boehringer synthesis, preferred protective groups include benzyl, diphenyl-methyl, triphenylmethyl, or silyl, where the three substituents on silyl may be C₁₋₆ alkyl or phenyl, and the phenyl groups optionally may be further substituted. Any protective groups can be removed as a final step in the synthesis, and crystallization or purification steps may also be added.

Unfortunately, the first step in the process disclosed by Boehringer required a minimum of two extraction, filtration, and crystallization steps; the second step in the process required the use of boiling cyclohexane, and final purification by chromatography; and the fourth step in the process required the use of a Pd or Raney-Nickel catalyst. The reported yield of the β-L-2,2'-anhydroarabinofuranosyl-thymine intermediate was approximately 49%. Thus, there exists a need for a synthetic method that avoids the use of a Pd or Raney-Nickel catalyst and that provides higher percent yields of the 2,2'-anhydro-thymidine intermediate.

Holy and Pragnacharyulu et al. disclosed the use of L-arabinose as a starting material that is reacted with cyanamide to produce a 1,2-oxazoline derivative; the oxazolidine derivative is reacted with propionic acid ethyl ester to provide an O^{2,2'}-anhydro-L-thymidine intermediate that is benzoylated and reductively cleaved or treated with hydrogen chloride to provide the desired chloro-sugar. (A. Holy, Coll. Czech. Chem. Commun. 1972, 37, 4072-4087).

Abushanab et al. reported a chloro-sugar synthesis that includes reacting a methyl-oxirane carboxylic acid ester with an oxazoline to provide O^{2,2'}-anhydro-L-thymidine intermediate (E. Abushanab, and P. V. P Pragnacharyula, U.S. Patent 5,760,208, June 2, 1998), while Asakura et al., Hirota et al. and A. Holy disclosed the reaction of ethyl propiolate with oxazoline to provide O^{2,2'}-anhydro-L-uridine, which is then protected at its 3' and 5' positions and reacted with hydrogen chloride to produce 2'-deoxy-2'-chloro sugar as an intermediate (J.-I. Asakura, and M. J. Robins, J. Org. Chem. 1990, 55, 4928-4933; J.-I. Asakura, and M. J. Robins, Tetrahedron Lett. 1988, 29, 2855-2858; K. Hirota, Y. Kitade, Y. Kanbe, Y. Isobe, and Y. Maki, Synthesis, 1993, 210, 213-215; and A. Holy, Coll. Czech. Chem. Commun. 1972, 37, 4072-4087).

In 2003, Abushanab and Pragnacharyulu reported a process for preparing pyrimidine nucleosides that involved a Michael-type condensation reaction between an arabinoribofuranosyl-amino-oxazoline and a substituted epoxy-methylate derivative; subsequent acylation of the condensed product by treatment with pivaloyl chloride to place a chloro group at the 2'-position of the thymidine; and finally, dehalogenation to remove the chloro substituent if a 2'-deoxy-thymidine was the desired product (U.S. Patent No. 6,596,859).

However, pivaloyl chloride is known to cause anhydro-ring opening, and its placement of a chloro group at the 2'-position on thymidine then requires an additional synthetic step to remove the chloro group. Also, it would be advantageous to avoid the use of the costly reagent methyl-2-methyl glycidate that Abushanab and Pragnacharyulu employ in the condensation reaction of their method, as well as the use of acetonitrile used in the second step of the process, and the chromatographic separations required for each step in the synthesis.

Pragnacharyulu et al. also reported the formation of a 2,2'-anhydro-amino-oxazoline from L-arabinose by reacting L-arabinose with H₂NCN, which permitted an intramolecular elimination of one terminal OH and one H to afford a 2,2'-anhydro-amino-oxazoline product intermediate (Pragnacharyulu et al., (1995), J. Org. Chem. 60:3096-99).

Sawai et al. disclosed a direct cyclization step in the formation of 2,2'-anhydro-(arabino-furanosyl)thymine from D-arabinose. Their synthesis comprised (1) preparing D-arabino-furanosyl-amino-oxazoline from D-arabinose by methods known in the art; (2) reacting the D-arabino-furanosyl-amino-oxazoline with ethyl-α-(bromo-methyl)-acrylate in dimethyl acetamide to provide an oxazolino-N-branched intermediate in approximately an 88% yield; and (3) reacting the intermediate formed in step (2) with KO^{t}Bu and *t*-BuOH to afford 2,2'-anhydro-(arabinofuranosyl)thymine in about a 30% yield, or alternatively, using hydrogen iodide to open the O^{2,2'}-anhydro-L-thymidine linkage, and then reacting the acyclic product with potassium iodide to produce di-O-benzoyl-2'-deoxythymidine (Sawai et al., (1994), Nucleosides & Nucleotides, 13(6-7):1647-54; Sawai et al., Chem. Lett., 1994, 605-606). This process advantageously avoids the use of catalysts like poisoned Pd/BaSO₄, but results in rather low % yields of products.

U.S. Patent No. 4,914,233 to Freskos et al. disclosed the selective separation of β-thymidine from a mixture of α- and β-anomers by a 5-step process involving formation of tri-O-acyl-β-ribothymidine, and conversion of 2,2'-anhydro-β-thymidine to 2'-halo-2'-deoxy-5-methyluridine followed by conversion of the latter to β-thymidine.

U.S. Patent No. 5,212,293 to Green et al., reported the synthesis of 2',3'-dideoxynucleosides by reacting a protected anhydrothymidine with a halo-generating agent that contained an organo-aluminum compound for increased reactant solubility.

U.S. Patent No. 5,596,087 to Alla et al. included the formation of 2,2'-anhydrothymidine that was brominated and then reduced by methods known to those skilled in the art, to produce β-thymidine.

U.S. Patent No. 6,369,040 to Acevedo et al. disclosed a 3',5'-protected -2,2'-anhydro-uridine to synthesize corresponding arabinosides.

McGee and Murtiashaw each reported preparing a chloro-sugar intermediate from L-arabinose as a starting material that includes an O^{2,2'}-anhydro-L-thymidine intermediate prepared from different reagent compounds than were used by Holy or Pragnacharyulu et al. (D. McGee, Boehringer Ingelheim Proposal to Novirio Pharmaceuticals, Inc., May 17, 2002; C. W. Murtiashaw, *Eur. Patent,* 0,351,126 B1, January 18, 1995).

McGee et al. disclosed a method for preparing 2'-modified nucleosides by an intramolecular displacement reaction (U.S. Patent No. 6, 090, 932). McGee et al. reported the introduction of a substituent at the 2'-position of a 2,2'-anhydro-uridine by the careful selection of a 3'-substituent that could be activated to cause stereospecific reduction at the 2'-position. The synthesis comprised protecting the 5'-OH of uridine by reaction with DMT to form 5'-O-(4,4'-dimethoxytrityl)uridine, and afforded the final product, 2'-deoxythymidine, in approximately 24% yield.

Even though McGee et al. reported that their process could be scaled for industrial purposes, it is known that dioxane is flammable and prone to peroxide formation, and is therefore contraindicated for industrial processes. In addition, McGee et al. is silent with respect to whether their process produced the D- or L-enantiomer of 2'-deoxythymidine, or whether separation of enantiomers was required.

Thus, there exists a need for a simple, cost-effective, and safe process for making 2'-deoxynucleosides, salts, analogs and prodrugs thereof, including β-L-2'-deoxynucleosides, such as β-L-2'-deoxythymidine, that avoids the use of hazardous, toxic, dangerous, and/or difficult to handle reagents that do not lend themselves to industrial production.

There is also a need to provide a synthesis for preparing 2'-deoxynucleosides, salts, analogs and prodrugs thereof, including β-L-2'-deoxynucleosides, such as P-L-2'-deoxythymidine, that utilizes safe materials and reagents.

There is also a need to provide a synthesis for preparing 2'-deoxynucleosides, salts, analogs and prodrugs thereof, including β-L-2'-deoxynucleosides, such as β-L-2'-deoxythymidine, under mild reaction conditions.

There is also a need to provide an efficient and cost-effective procedure for synthesizing 2'-deoxynucleosides, salts, analogs and prodrugs thereof, including β-L-2'-deoxynucleosides, such as β-L-2'-deoxythymidine, under mild reaction conditions.

There is also a need to provide a synthesis that is efficient by requiring a minimal number of steps.

There is also a need to provide a process that requires few or no steps for product separation.

There is also a need to provide an industrially scalable process for the synthesis of 2'-deoxynucleosides, salts, analogs and prodrugs thereof, including β-L-2'-deoxynucleosides, such as β-L-2'-deoxythymidine, that is cost-effective and affords the final product in high yield.

There is also a need to provide an industrially-scalable synthesis for β-2'-deoxynucleosides, salts, analogs and prodrugs thereof, including β-L-2'-deoxynucleosides, such as β-L-2'-deoxythymidine, that produces the β-anomeric form of the desired compound in excess of the α-anomeric form in good yields.

There is also a need to provide a synthesis for amino-acid prodrugs of 2'-deoxynucleosides, salts, and analogs thereof, including β-L-2'-deoxynucleosides, such as β-L-2'-deoxythymidine.

### SUMMARY OF THE INVENTION

The present invention discloses novel, efficient synthetic processes for preparing 2'-, 3'- and/or 5'-substituted-nucleosides and 2'-, 3'- and/or 5'-deoxy-nucleosides, such as 2'-substituted and 2'-deoxy-nucleosides derived from natural and non-natural carbocyclic, heterocyclic and heteroaromatic nucleoside bases, and, in particular, β-L-2'-deoxy-thymidine (LdT) and salts, prodrugs, stereroisomers and enantiomers thereof. Processes for the production of the stereoisomeric, diastereoisomeric, and enantiomeric forms of the compounds of the present invention, based on the appropriate starting materials are also provided. The compounds made according to the present invention may be used as intermediates in the preparation of a wide variety of other nucleoside analogues, or may be used directly as antiviral and/or antineoplastic agents.

In one embodiment, the 2'-deoxy-nucleosides and 2'-substituted nucleosides have naturally-occurring pyrimidine nucleoside bases. In a particular embodiment, the process is directed to the synthesis of β-L-2'-deoxythymidine (LdT). In another embodiment, the 2'-deoxy-nucleosides and 2'-substituted nucleosides have non-naturally occurring pyrimidine-like nucleoside bases. In one particular embodiment, the non-naturally occurring pyrimidine-like nucleoside base can be prepared by a synthetic process disclosed in the present invention.

In one embodiment, the process of the present invention requires no separation of isomers, and therefore is an improvement over the prior art.

In one embodiment, the introduction of functionalities at the 2'-position or elimination of such functionalities to give a 2'-deoxy nucleoside is accomplished by selective reactions that utilize D-xylose, L-arabinose, L-ribose, D-galactose, D-gluconolactone, D-galactonolactone, D-glucose, D-hydroxy-glutamic acid (for ribonolactone), alcohol or epoxyalcohol, isopropylidene glyceraldehydes, or substituted dioxolane as a starting reagent.

In one particular embodiment of the invention, the syntheses proceed via a chloro-sugar intermediate. Therefore, one particular intermediate of the synthetic processes set forth herein, which does not involve intramolecular rearrangements, is a chloro-sugar compound.

In another particular embodiment of the invention, the syntheses proceed via an intramolecular nucleophilic displacement. Therefore, one particular intermediate of the synthetic processes set forth herein is a 2,2'-anhydro-1-furanosyl nucleoside ring.

In one embodiment of the invention, one of the critical intermediates is obtained by reduction of the lactone with a reducing agent, such as Red-Al, as follows:

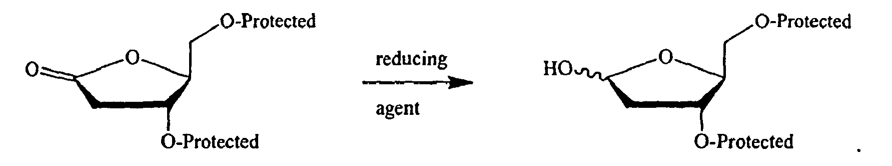

In one particular embodiment, the oxygen protecting groups are toluoyl.

In another particular embodiment, the intermediate is obtained as follows:

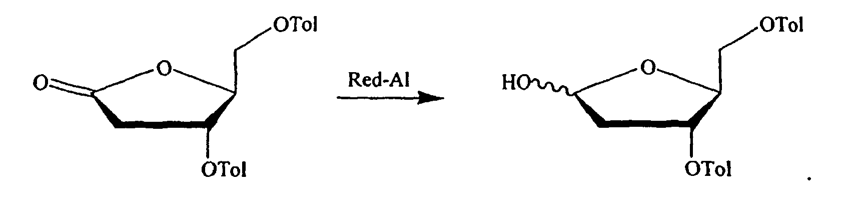

Therefore, in an embodiment of the present invention, the synthetic method includes the steps of:

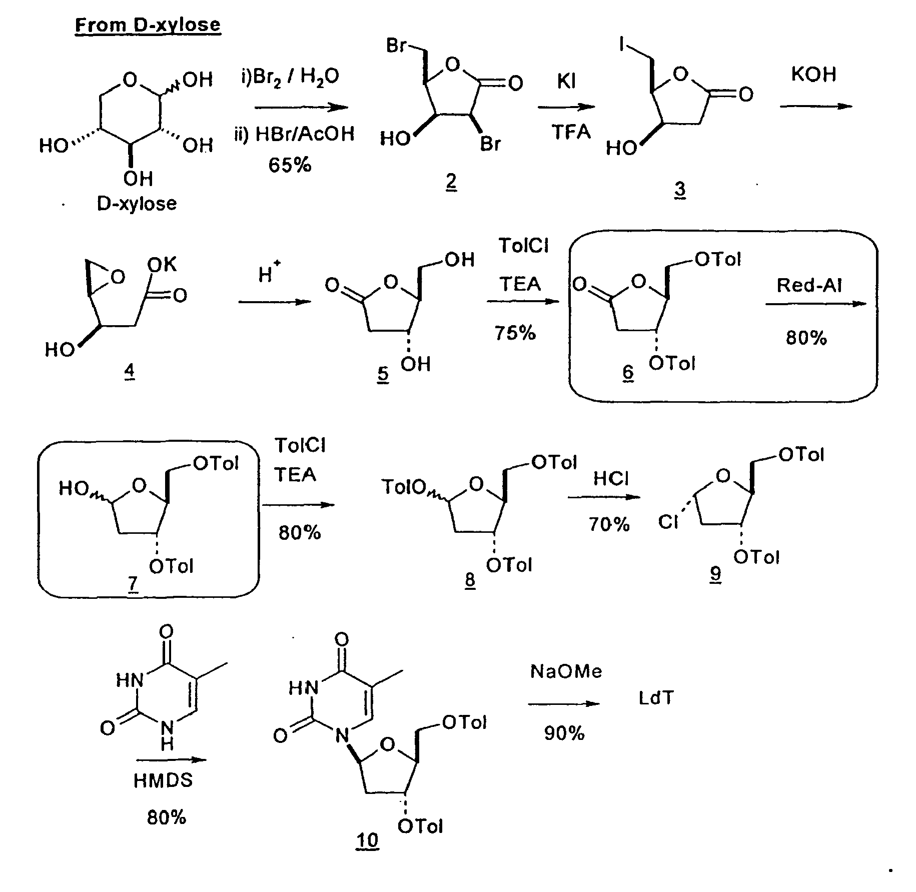

An alternative synthesis of the present invention for the preparation of 2'-deoxythymidine includes the following method steps:

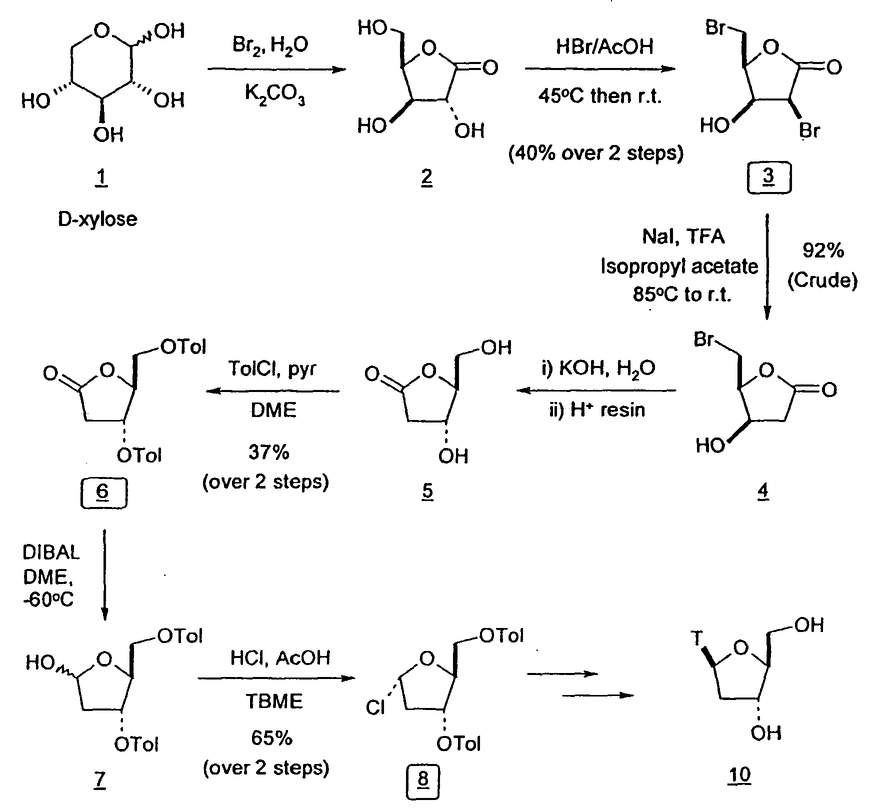

In still another embodiment of the present invention there is provided a process for preparing 2'-deoxythymidine from D-xylose that includes 2-deoxy-3,5-di-O-*para-*toluoyl-α-L-erythro-pentofuranosyl chloride as a key intermediate.

In an alternate embodiment, a synthesis is provided using a mesylate intermediate: 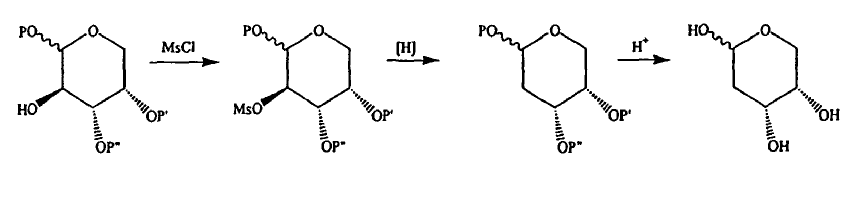 wherein P, P' and P" are independently H, alkyl, or a suitable oxygen protecting group. In one embodiment, P is methyl. In another embodiment P' and P" come together to form an isopropylidine.

Therefore, in one particular embodiment, the synthesis is provided using a mesylate intermediate:

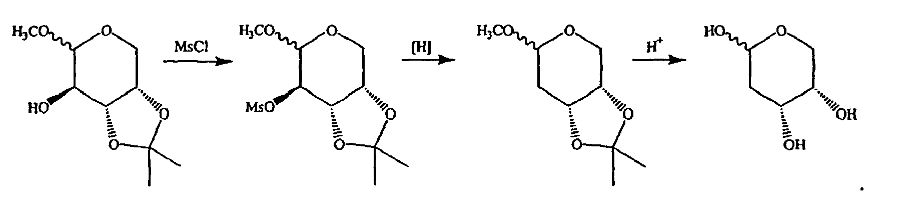

In an alternate embodiment, one of the critical intermediates is obtained by the following method:

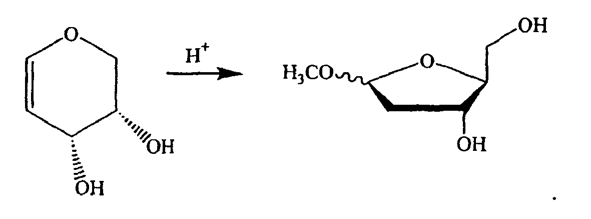

In an alternate embodiment, one of the critical intermediates is obtained cis-oxidation of an alkene using an appropriate oxidizing agent capable of cis-oxidation, such as OsO₄, by the following method:

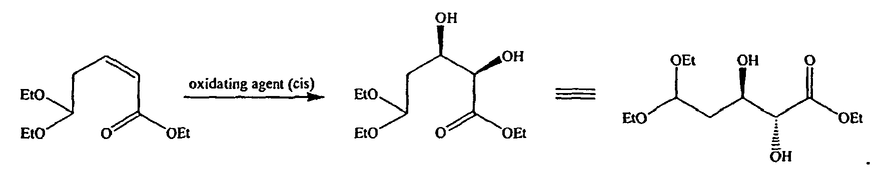

Therefore, in one particular embodiment, the critical intermediate is obtained cis-oxidation of an alkene using OsO₄, by the following method:

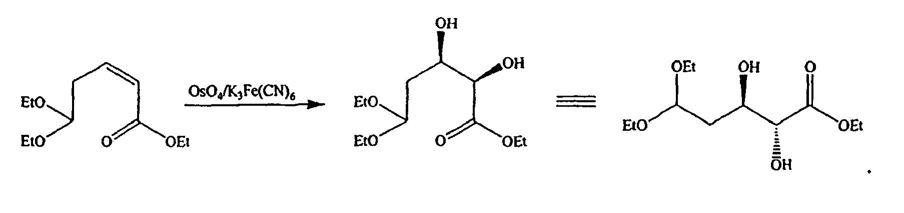

In an alternate embodiment, one of the critical intermediates is obtained by the following method:

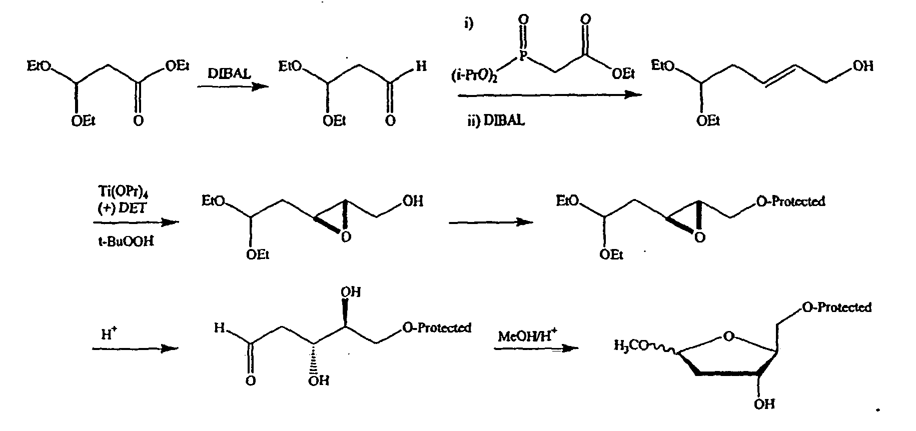

In an alternate embodiment, one of the critical intermediates is obtained by the following method:

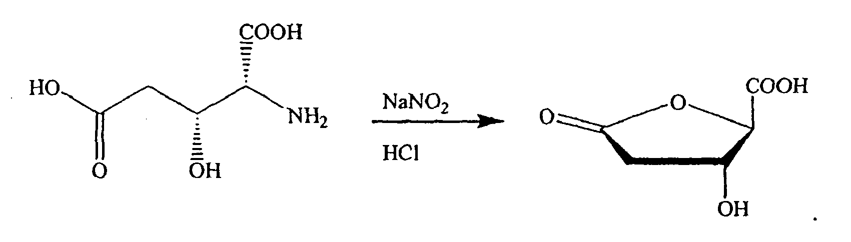

In an alternate embodiment, one of the critical intermediates is obtained via reaction with an alcohol/acid solution, by one of the following methods: 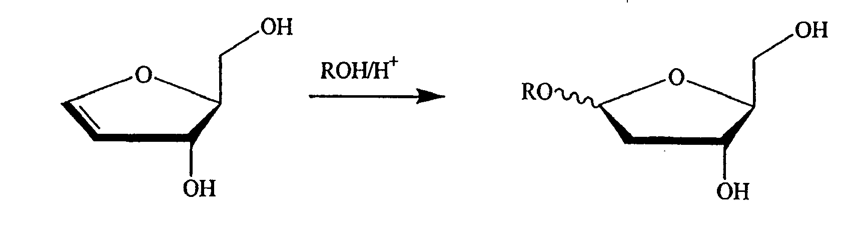 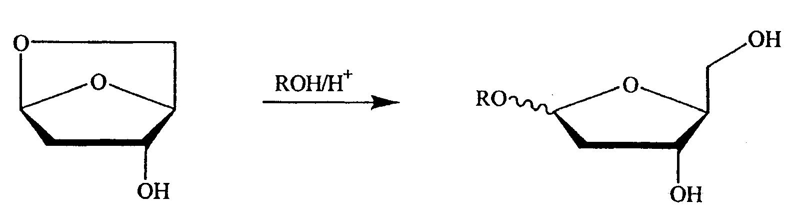 wherein R is an alkyl, preferably a lower alkyl, such as methyl or ethyl, and in particular methyl.

In one embodiment of the invention, the alcohol is selected from the group consisting of methanol, ethanol, propanol, isopropanol, butanol, isobutanol, t-butanol, s-butanol, pentanol, hexanol, or a mixture thereof. In a particular embodiment, the alcohol is methanol or ethanol. In another particular embodiment, the alcohol is methanol.

Therefore, in a particular embodiment of the invention, the critical intermediate is obtained via reaction with an alcohol/acid solution, by one of the following methods:

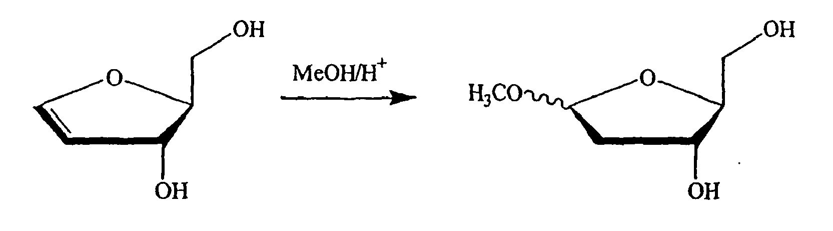

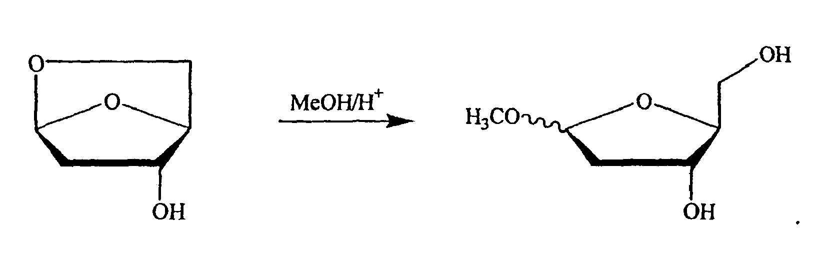

Another representative process of the present invention includes using a reducing agent, such as Red-Al, in combination with a sequestering agent, such as 15-crown-5 ether, to rupture a 2,2'-anhydro-1-furanosyl-nucleoside ring intermediate to produce the desired nucleoside product.

It has been unexpectedly found that the use of a sequestering agent, such as 15-crown-5 ether, affords a higher percent product yield when dimethoxy trityl is the protecting group of choice, but a lower percent product yield when trityl alone is used as a protecting group. Therefore, in one embodiment of the invention, a process is provided that includes the step of rupturing a 2,2'-anhydro-1-furanosyl nucleoside ring intermediate to form a desired nucleoside product in the absence of a sequestering agent. In a particular embodiment of the present invention, a process is provided that includes the step of rupturing a 2,2'-anhydro-1-furanosyl nucleoside ring intermediate to form a desired nucleoside product in the absence of a sequestering agent when trityl is the protecting group.

Processes are provided for using an appropriate nucleophilic agent, e.g. an organometallic agent (e.g. a Grignard reagent or an alkyl lithium reagent) if an alkyl substituent is desired, to rupture a 2,2'-anhydro-1-furanosyl-nucleoside ring intermediate to produce the desired 2'-substituted nucleoside product.

In one embodiment, the present invention is directed to a process for preparing a 2'-deoxynucleoside or 2'-modified nucleoside that comprises (a) optionally protecting one or more hydroxyl groups on a furanosyl ring, such as a ribo-, arabino-, or xylo-furanosyl, by reaction with a protecting group; (b) condensing the furanosyl ring from step (a) with an optionally substituted natural or non-natural nucleoside base to form a nucleoside; (c) reacting the nucleoside from step (b) with a condensing agent at an elevated temperature to afford a 2,2'-anhydro-1-furanosyl-nucleoside; (d) reacting the 2,2'-anhydro-1-furanosyl-nucleoside from step (c) with a reducing agent, such as Red-Al, and a sequestering agent, such as 15-crown-5 ether, preferably in a polar solvent at a low temperature, to afford an optionally protected 2'-deoxynucleoside or 2'-substituted nucleoside; and (e) deprotecting the optionally protected hydroxyl groups, if necessary or desired, for example by the addition of acids or acid resins at a temperature of about 50°C.

In another embodiment, a process is provided for preparing a 2'-deoxythymidine that comprises (a) optionally protecting one or more hydroxyl groups on a furanosyl ring by reaction with a protective group; (b) reacting the optionally protected furanosyl ring with cyanamide to form an optionally protected furanosylaminooxazoline; (c) reacting the optionally protected furanosylaminooxazoline with a cyclization or condensation agent to afford an optionally protected 2,2'-anhydro-1-furanosyl-thymidine; (d) reacting the optionally protected 2,2'-anhydro-1-furanosyl-thymidine with a reducing agent, such as Red-Al, and a sequestering agent, such as 15-crown-5 ether, preferably in a polar solvent at a low temperature to provide an optionally protected, 2'-deoxythymidine; and (e) deprotecting the optionally protected 2'-deoxythymidine, if necessary or desired, for example, by reaction with acids or acid resins at about 50°C to provide 2'-deoxythymidine.

In yet another embodiment, the present invention is directed to a process for preparing a 2'-deoxythymidine that embraces steps (a) - (e) given above, but does not include the use of a sequestering agent as given in step (d).

In yet another embodiment, the present invention is directed to a process for preparing a 2'-deoxynucleoside or 2'-modified nucleoside that comprises (a) optionally protecting one or more hydroxyl groups on a furanosyl ring, such as a ribo-, arabino-, or xylo-furanosyl, by reaction with a protecting group; (b) condensing the furanosyl ring from step (a) with an optionally substituted natural or non-natural nucleoside base to form a nucleoside; (c) reacting the nucleoside from step (b) with a condensing agent at an elevated temperature to afford a 2,2'-anhydro-1-furanosyl-nucleoside; (d) reacting the 2,2'-anhydro-1-furanosyl-nucleoside from step (c) with a reducing agent, such as Red-Al, in the absence of a sequestering agent, such as 15-crown-5 ether, preferably in a polar solvent at a low temperature, to afford an optionally protected 2'-deoxynucleoside or 2'-substituted nucleoside; and (e) deprotecting the optionally protected hydroxyl groups, if necessary or desired, for example by the addition of acids or acid resins at a temperature of about 50°C.

In another embodiment, a process is provided for preparing a 2'-deoxythymidine that comprises (a) optionally protecting one or more hydroxyl groups on a furanosyl ring by reaction with a protective group; (b) reacting the optionally protected furanosyl ring with cyanamide to form an optionally protected furanosylaminooxazoline; (c) reacting the optionally protected furanosylaminooxazoline with a cyclization or condensation agent to afford an optionally protected 2,2'-anhydro-1-furanosyl-thymidine; (d) reacting the optionally protected 2,2'-anhydro-1-furanosyl-thymidine with a reducing agent, such as Red-Al, in the absence of a sequestering agent, such as 15-crown-5 ether, preferably in a polar solvent at a low temperature to provide an optionally protected, 2'-deoxythymidine; and (e) deprotecting the optionally protected 2'-deoxythymidine, if necessary or desired, for example, by reaction with acids or acid resins at about 50 °C to provide 2'-deoxythymidine.

Included within the scope of the present invention are processes for the production of 2'-modified nucleosides, phosphoramidites of 2'-modified nucleosides, 3'-and 5'-mono, di-, and tri-phosphates of 2'-modified nucleosides, and oligonucleotides that comprise at least one nucleoside modified according to the process of the present invention. Also included are processes for the production of intramolecular functionalities that involve anhydronucleosides at positions other than the 2'-position on the furanose ring, e.g. the 3' and/or 5'-position. Processes of the present invention also include functional group modification to produce, for example, the corresponding 5'-diacylglycerophosphate or 5'-dialkylglycerolphosphate derivatives that can be used as prodrugs.

Yet other embodiments of the present invention are provided in the disclosure and Examples contained herein.

### BRIEF DESCRIPTION OF THE SCHEMES

**Figure 1** is a schematic of a process of the present invention for preparing LdT from L-arabinose via a mesylate intermediate.
**Figure 2** is a schematic of a process of the present invention for preparing LdT from L-arabinose via a glycal intermediate.
**Figure 3** is a schematic of a process of the present invention for preparing LdT from L-arabinose via a glycal intermediate and a reductive elimination step.
**Figure 4** is a schematic of a process of the present invention for preparing LdT from L-xylose via a di-O-toluoyl derivative.
**Figure 5** is a schematic of a process of the present invention for preparing LdT from D-galactose.
**Figure 6** is a schematic of a process of the present invention for preparing LdT from D-gluconolactone.
**Figure 7** is a schematic of a process of the present invention for preparing LdT from D-galactonolactone.
**Figure 8** is a schematic of a process of the present invention for preparing LdT from a furonolactone, a non-carbohydrate, achiral starting material.
**Figure 9** is a schematic of a process of the present invention for preparing LdT from ethyl-3,3-diethoxypropanoate.
**Figure 10** is a schematic of a process of the present invention for preparing LdT from hydroxy glutamic acid.
**Figure 11** is a schematic of a process of the present invention for preparing LdT from a commercially available alcohol via an epoxidation.
**Figure 12** is a schematic of a process of the present invention for preparing LdT from an epoxyalcohol.
**Figure 13** is a schematic of a process of the present invention for preparing LdT from 1,2-O-isopropylidine-L-glyceraldehyde.
**Figure 14** is a schematic of a process of the present invention for preparing LdT from 2-bromomethyl-[1,3]-dioxolane.
**Figure 15** is a schematic of a process of the present invention for preparing LdT from a glycal treated with acidic methanol.
**Figure 16** is a schematic of a process of the present invention for preparing LdT from L-arabinose and cyanamide.
**Figure 17** is a schematic of a process of the present invention for preparing LdT from L-arabinose via a hydrogen chloride opening of the O^{2,2'}-linkage of the compound.
**Figure 18** is a schematic of a process of the present invention for preparing LdT from L-arabinose as in **Figure 17** using alternative reagents for opening the O^{2,2'}-linkage of the compound.
**Figure 19** is a schematic of a process of the present invention for preparing LdT from L-arabinose as in **Figure 17** using hydrogen iodide for opening the O^{2,2'}-linkage of the compound.
**Figure 20** is a schematic of a process of the present invention for preparing LdT from L-arabinose that includes reacting 2-methyl-oxirane-2-carboxylic acid ester with 1,2-oxazoline.
**Figure 21** is a schematic of a process of the present invention for preparing LdT from L-arabinose via an O^{2,2'}-anhydro-L-uridine intermediate.
**Figure 22** is a schematic of a process of the present invention for preparing LdT from L-arabinose as in **Figure 21** proceeding via a 2'-deoxy-5-ethoxymethyl-L-uridine intermediate.
**Figure 23** is a schematic of a process of the present invention for preparing LdT from D-xylose via a 2-deoxy-3,5-di-O-*para*-toluoyl-α-L-erythro-pentofuranosyl chloride intermediate.
**Figure 24** is a schematic of a process of the present invention for preparing β-L-deoxy-thymidine where the 5'-OH of the arabinofuranosyl-amino-oxazoline intermediate is protected by a trityl group prior to formation of the 2,2'-anhydro-1-(β-arabinofuranosyl)-thymidine intermediate and its reductive cleavage by Red-Al and 15-Crown-5 ether.
**Figure 25** is a schematic of a process of the present invention for preparing β-L-deoxy-thymidine where protection of the 5'-OH of the L-arabinofuranosyl moiety occurs after formation of 2,2'-anhydro-1-(β-arabinofuranosyl)-thymidine intermediate and its reductive cleavage by Red-Al and 15-Crown-5 ether.
**Figure 26** is a schematic of a process of the present invention for preparing β-D-deoxy-thymidine from D-ribose that involves protection and deprotection with an OH-protective group at 2'-, 3'- and 5'-positions of the ribofuranosyl, and then utilizes trityl as a protective group at the 5'-position alone prior to reductive cleavage by Red-Al and 15-Crown-5 ether.
**Figure 27** is a schematic of a process of the present invention in which a 2,2'-anhydro-1-(β-ribofuranosyl)-thymidine intermediate is formed directly from thymidine, then protected at its 5'-OH by a trityl group, and finally reductively cleaved by Red-Al and 15-Crown-5 ether.
**Figure 28** is a schematic of a process of the present invention that utilizes L-ribose as a starting material and proceeds via protection and deprotection of its hydroxyl groups with any appropriate protecting group prior to formation of a 2,2'-anhydro-1-(β-ribofuranosyl)-thymidine intermediate, which then is protected at its 5'-OH position before reductive cleavage with Red-A1 and 15-Crown-5 ether.
**Figure 29** is a schematic of a process of the present invention for preparing β-D-deoxy-thymidine from 2,2'-anhydro-1-β-D-arabinofuranosyl thymine without the use of a sequestering agent during reduction.

### DETAILED DESCRIPTION OF THE INVENTION

The present invention discloses novel, efficient synthetic processes for preparing 2'-, 3'- and/or 5'-substituted-nucleosides and 2'-, 3'- and/or 5'-deoxy-nucleosides, such as 2'-substituted and 2'-deoxy-nucleosides derived from natural and non-natural carbocyclic, heterocyclic and heteroaromatic nucleoside bases, and, in particular, β-L-2'-deoxy-thymidine (LdT) and salts, prodrugs, stereroisomers and enantiomers thereof. Included herewith are processes for the production of the stereoisomeric, diastereoisomeric, and enantiomeric forms of the compounds of the present invention, based on the appropriate starting materials. The compounds made according to the present invention may be used as intermediates in the preparation of a wide variety of other nucleoside analogues, or may be used directly as antiviral and/or antineoplastic agents.

In one embodiment, the 2'-deoxy-nucleosides and 2'-substituted nucleosides have naturally-occurring pyrimidine nucleoside bases. In a particular embodiment, the process is directed to the synthesis of β-L-2'-deoxythymidine (LdT). In another embodiment, the 2'-deoxy-nucleosides and 2'-substituted nucleosides have non-naturally occurring pyrimidine-like nucleoside bases. In one particular embodiment, the non-naturally occurring pyrimidine-like nucleoside base can be prepared by a synthetic process disclosed in the present invention.

In one embodiment, the process of the present invention requires no separation of isomers, and therefore is an improvement over the prior art.

In one embodiment, the introduction of functionalities at the 2'-position or elimination of such functionalities to give a 2'-deoxy nucleoside is accomplished by selective reactions that utilize D-xylose, L-arabinose, L-ribose, D-galactose, D-gluconolactone, D-galactonolactone, D-glucose, D-hydroxy-glutamic acid (for ribonolactone), alcohol or epoxyalcohol, isopropylidene glyceraldehydes, or substituted dioxolane as a starting reagent.

In one particular embodiment of the invention, the syntheses proceed via a chloro-sugar intermediate. Therefore, one particular intermediate of the synthetic processes set forth herein, which does not involve intramolecular rearrangements, is a chloro-sugar compound.

In another particular embodiment of the invention, the syntheses proceed via an intramolecular nucleophilic displacement. Therefore, one particular intermediate of the synthetic processes set forth herein is a 2,2'-anhydro-1-furanosyl nucleoside ring.

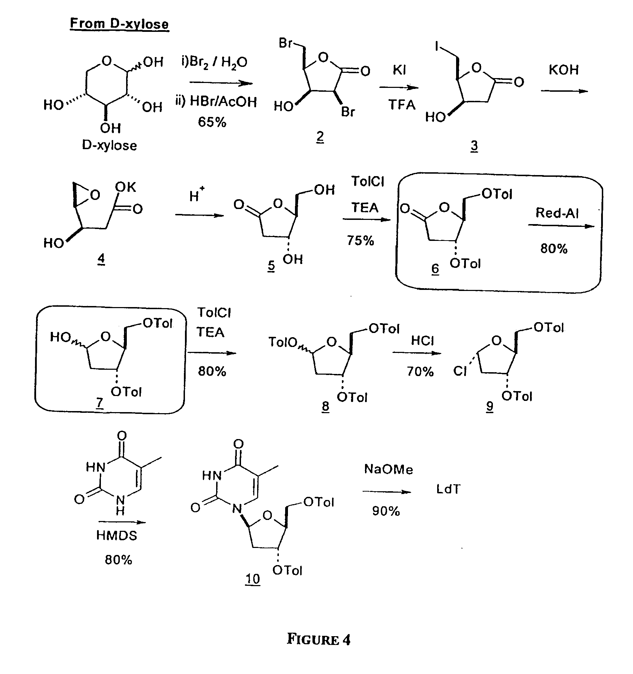

In a first embodiment, 2'-deoxythymidine is prepared from D-xylose as a starting material (**Figure 4**). This synthesis comprises: (a) oxidizing D-xylose first with an aqueous solution of bromine, and then with acetic and hydrobromic acid to form 2,5-dibromo-2,5-dideoxy-D-lyxono-1,4-lactone **(2);** (b) reacting the lactone product from step (a) with potassium iodide in trifluoroacetic acid (TFA), to provide the corresponding 5-iodo compound with selective removal of the bromine atom at C-2 to give 5-iodo-2-deoxylactone (3); (c) subjecting the 5-iodo-2-deoxylactone to aqueous potassium hydroxide to provide the 4,5-epoxide derivative **(4);** (d) treating the 4,5-epoxide derivative with an aqueous acid to produce the corresponding 2-deoxy-L-ribonolactone via a stereospecific inversion at C-4 **(5);** (e) protecting the C-3 and C-5 positions by reaction with any protecting group, such as toluoyl chloride in TEA **(6);** (f) selectively reducing the protected 2-deoxy-L-ribonolactone with Red-Al reducing agent to give the corresponding lactol **(7);** and (g) converting the lactol from step (f) to the desired chloro sugar intermediate **(9).**

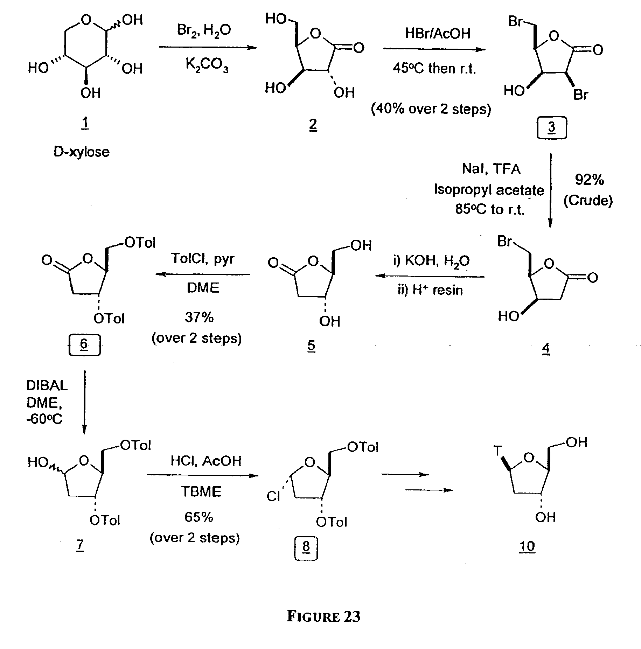

In a second embodiment, an alternative synthesis for preparing 2'-deoxythymidine is provided that also utilizes D-xylose as a starting material using alternative reagents and advantageously eliminates three chromatographic purifications that involve highly polar, water soluble, UV-inactive reagents (**Figure 23**). The process comprises: (a) oxidizing D-xylose first with bromine/water and potassium carbonate to provide D-lyxono-1,4-lactone **(2);** (b) reacting the lactone of step (a) with acetic and hydrobromic acid, for example at 45 °C for 1 hour and then at room temperature with stirring for about 1.5 hours, to provide 2,5-dibromo-2,5-dideoxy-D-lyxono-1,4-lactone **(3);** (c) reacting the lactone of step (b) with isopropyl acetate and sodium iodide in TFA, and, for example heating the reaction mixture to about 85°C. for about 1.5 hours, to form 5-bromo-2,5-dideoxy-D-threo-pentono-1,4-lactone **(4);** (d) reacting the lactone from step (c) with potassium hydroxide and water and, for example, after 3 hours, heating the reaction mixture to about 80°C for 30 minutes, then cooling the mixture to room temperature with stirring overnight, to provide 2-deoxy-L-ribono-1,4-lactone **(5);** (e) adding toluoyl protecting groups to C-3 and C-5 by reacting the lactone of step (d) with *para*-toluoyl chloride, for example with pyridine in DME, (**6**); (f) reacting the 2-deoxy-3,5-di-O-*para*-toluoyl-L-ribono-1,4-lactone with DIBAL and, for example DME at approximately -60 °C for about 1 hour, to provide 2-deoxy-3,5-di-O-*para*-toluoyl-L-ribose (7); (g) reacting the product of step (f) with dry HCI gas in acetic acid to prepare 2-deoxy-3,5-di-O-*para*-toluoyl-a-L-erythro-pentofuranosyl chloride (**8**), which can then be reacted by means known to those of skill in the art to provide 2'-deoxythymidine as the final, desired product.

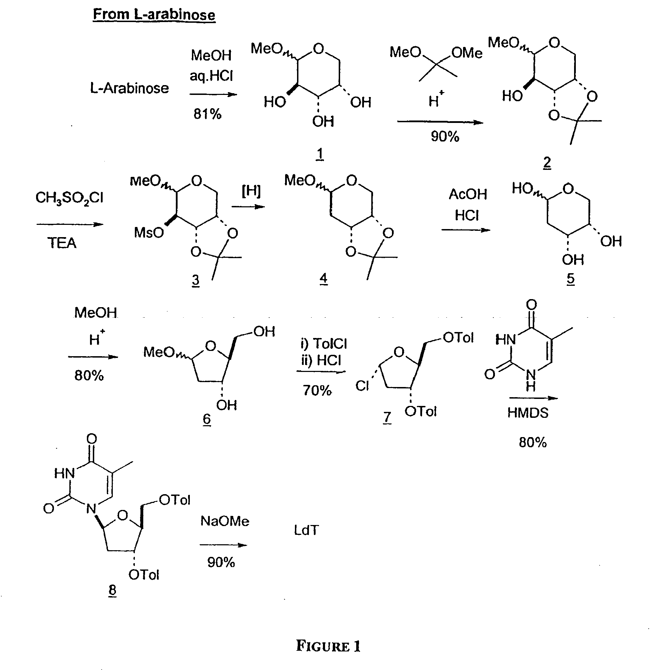

In certain embodiments, L-arabinose is used as a starting material for the preparation of 2'-deoxynucleosides and 2'-deoxythymidine in particular. These processes include the steps of (a) converting L-arabinose to its corresponding methyl glycoside, thereby protecting the C-3 and C-4 hydroxyl groups as acetonide derivatives **(2),** (b) deoxygenating the C-2 hydroxyl group by converting it to the corresponding mesylate group **(3),** and then (c) subjecting the mesylate intermediate to reductive cleavage **(5)** with an additional two process steps to afford the key chlorosugar intermediate (**Figure 1**).

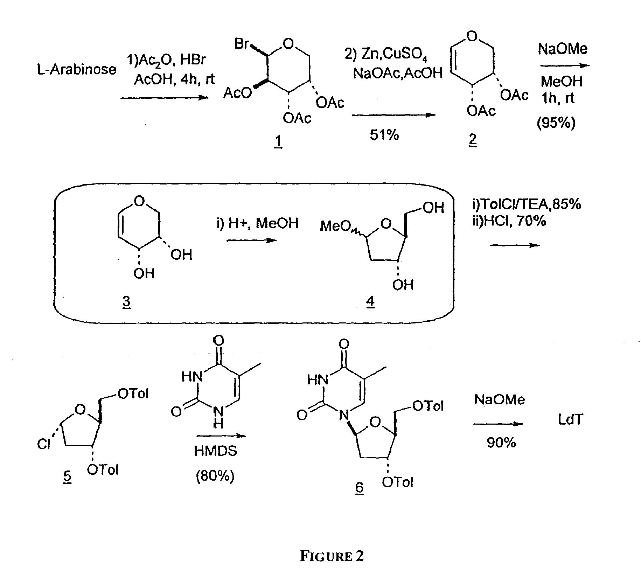

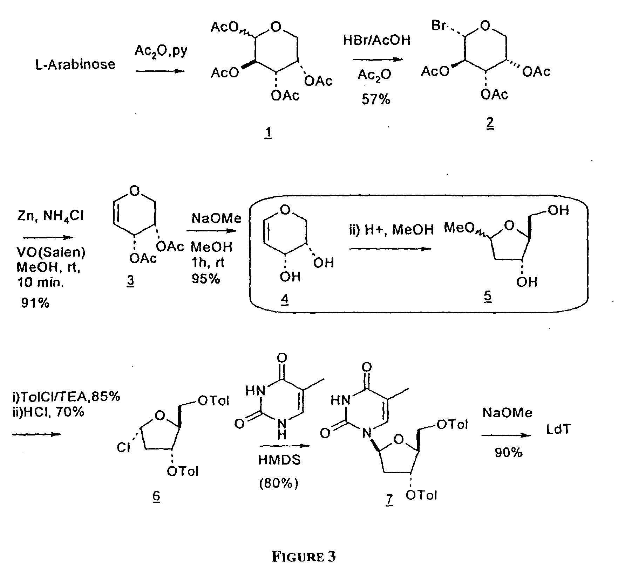

Alternatively, L-arabinose may be converted to its corresponding glycal derivative via a reductive elimination step, see for example **Figures 2** and **3**, steps **(1)** and **(2)** respectively, and the resulting glycal intermediate may then be converted to methyl 2-deoxy-ribofuranoside, steps **(4)** and **(5)** respectively.

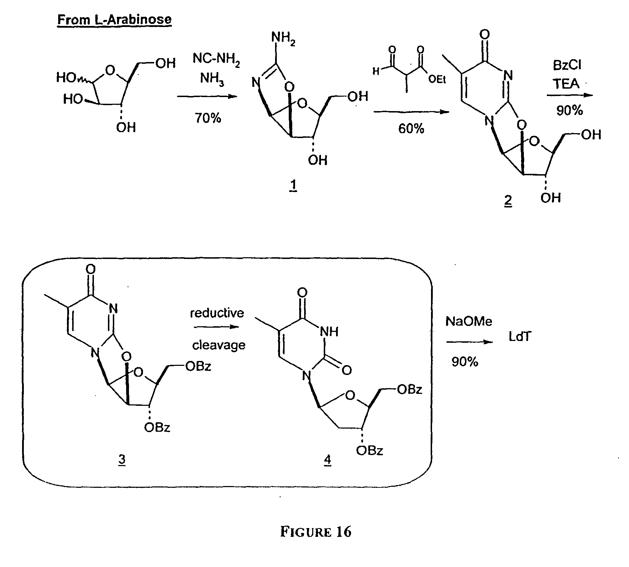

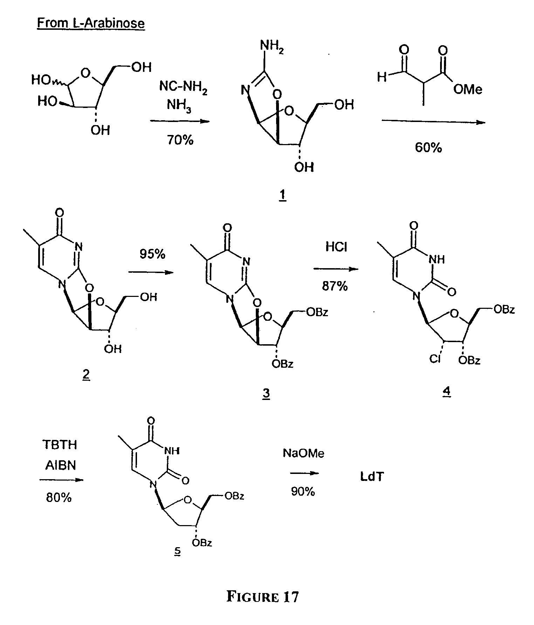

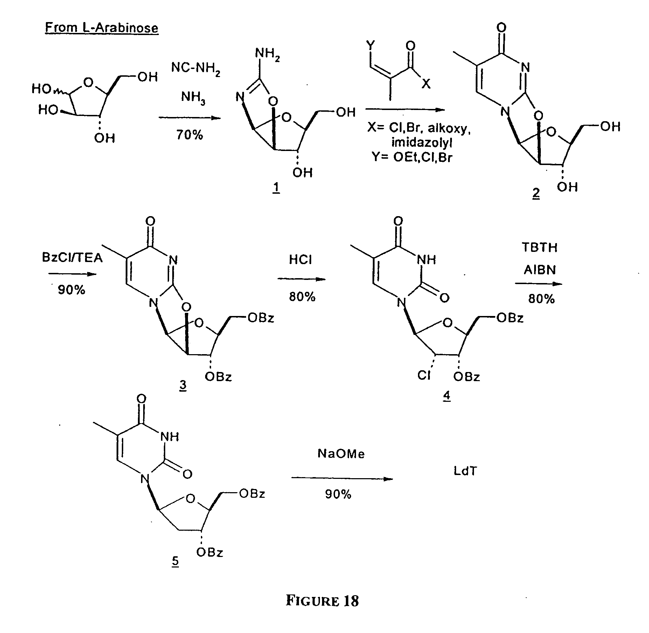

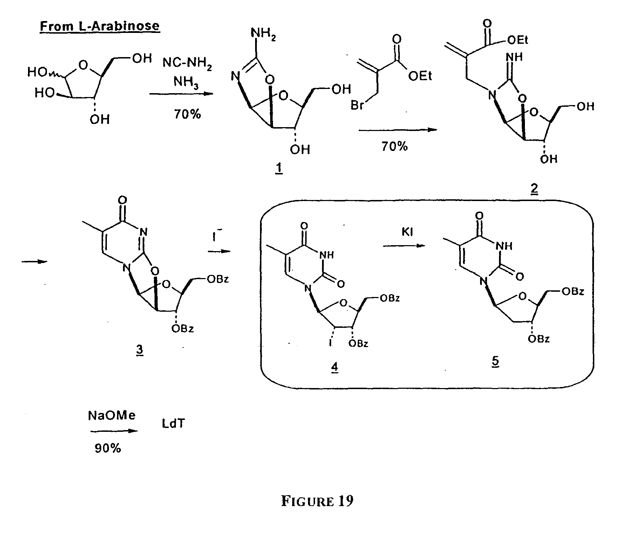

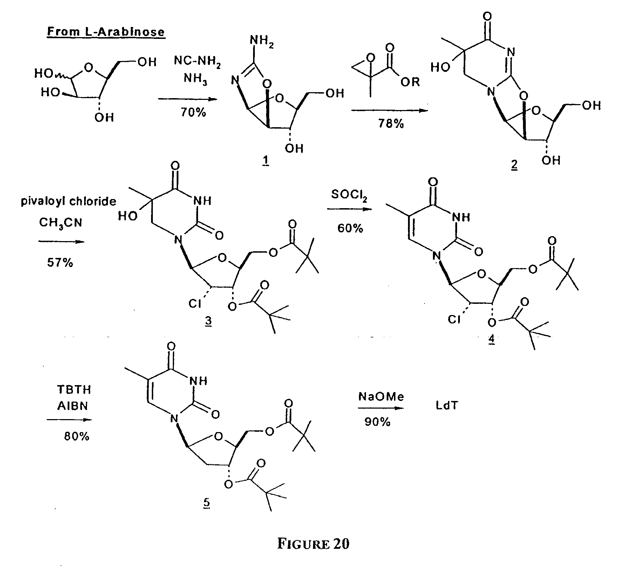

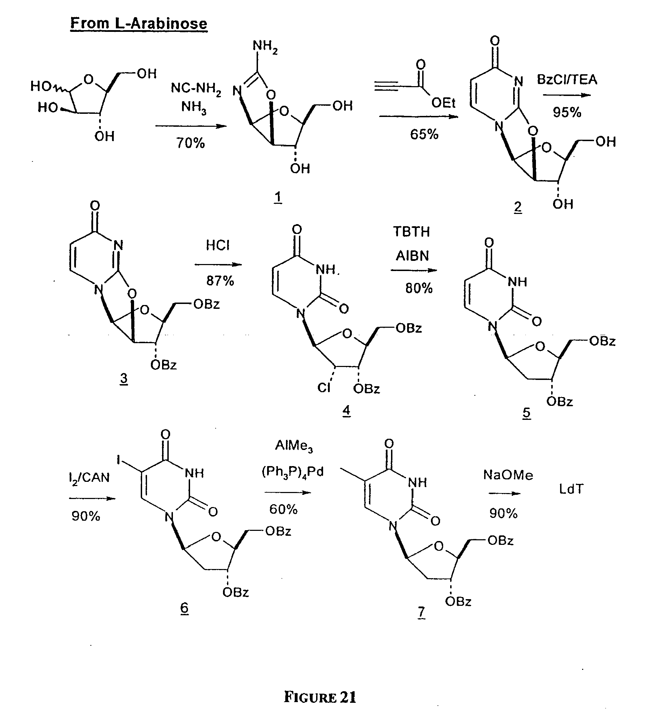

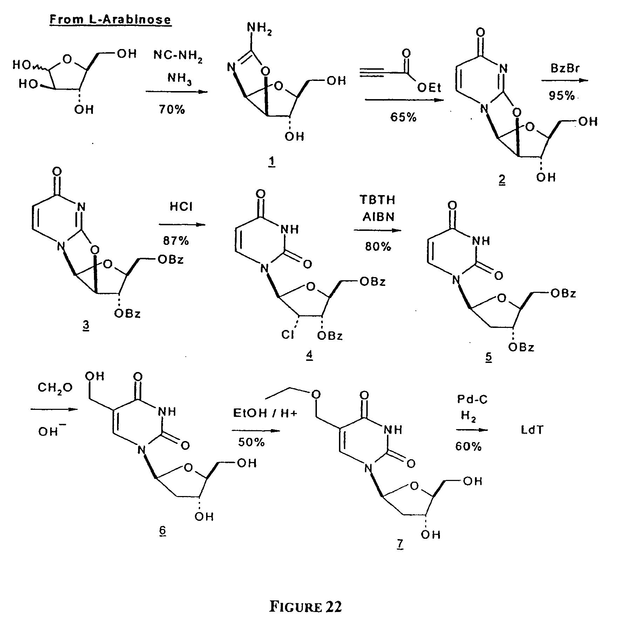

In other embodiments of the present invention, L-arabinose is utilized as a starting material. Such processes include the steps of (a) reacting L-arabinose with cyanamide to afford a 1,2-oxazoline intermediate **(1),** (b) reacting the intermediate of step (a) with a 3-oxo-propionic acid ester derivative or ethyl propiolate to provide a 2,2'-anhydro-1-furanosyl nucleoside ring **(2),** and (c) rupturing the ring of step (b) using various reactants and under different reaction conditions to provide LdT **(****Figures 16-22**).

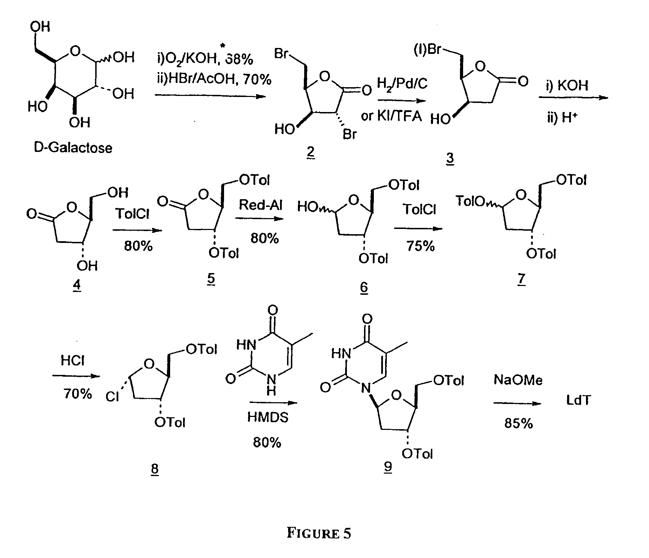

Alternatively, 2'-deoxynucteosides also may be formed from galactose as a starting material. When D-galactose is utilized as the starting material, it is oxidatively cleaved and brominated to provide 2,5-dibromo-2,5-dideoxy-D-lyxono-1,4-lactone, and this lactone undergoes selective hydrogenolysis to afford 5-bromo-2-deoxylactone that undergoes a sequence of transformations to provide the key chlorosugar intermediate (**Figure 5**).

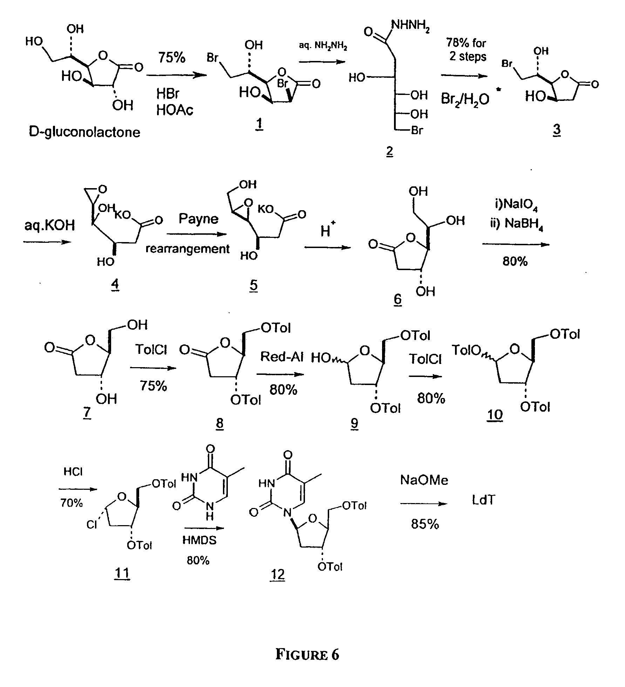

Likewise, gluconolactones may serve as starting materials for the synthesis of 2'-deoxynucleotides. Gluconolactone is converted to 2,6-dibromo-2,6-dideoxy-D-mannono-1,4-lactone **(1),** successively treated with hydrazine and aqueous potassium hydroxide, acidified to cause inversion at C-4 and C-5 that affords a 2-deoxy-lactone **(6),** subjected to a Payne rearrangement of the epoxide **(5),** oxidatively cleaved and reduced to provide a lactone **(7)** that easily can be converted to the desired chlorosugar **(11)** (**Figure 6**).

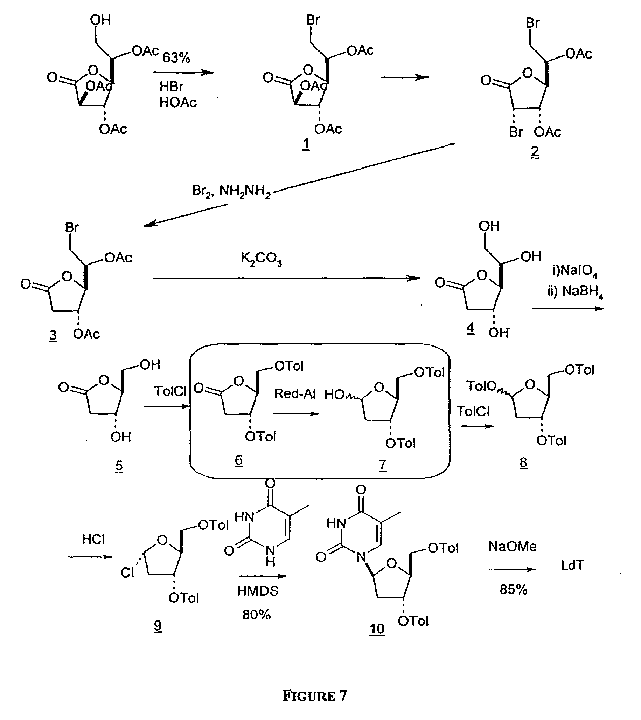

Alternatively, 2'-deoxynucleosides also may be formed from galactonolactones as a starting material. Where galactonolactone is utilized as the starting material, it is converted to the acetylated dibromolactone **(2),** treated with hydrazine and brominated to provide 2-deoxylactone **(3),** which is then de-acetylated, oxidatively cleaved and reduced by NaBH₄ to provide 2-deoxy-L-ribono-1,4-lactone **(5),** and this lactone is protected by reaction with toluoyl chloride, subjected to reduction by Red-Al and chlorination to afford the final, desired chlorosugar product **(9) (****Figure 7****).**

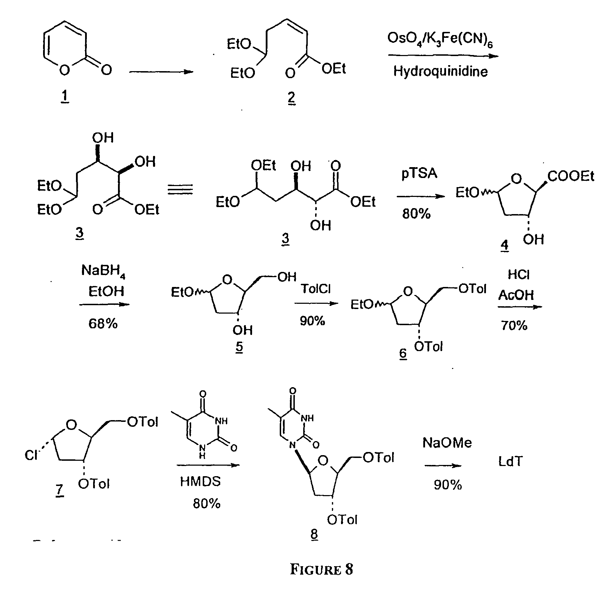

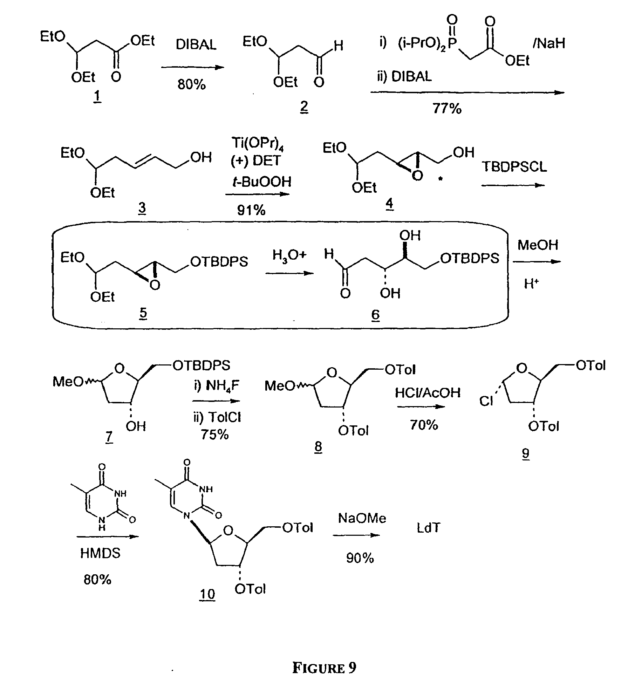

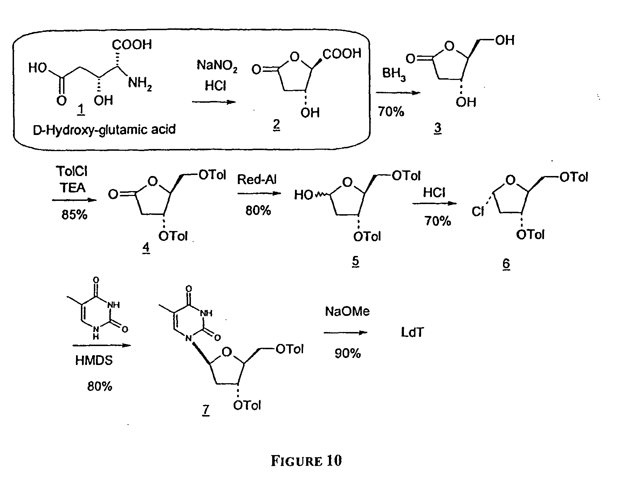

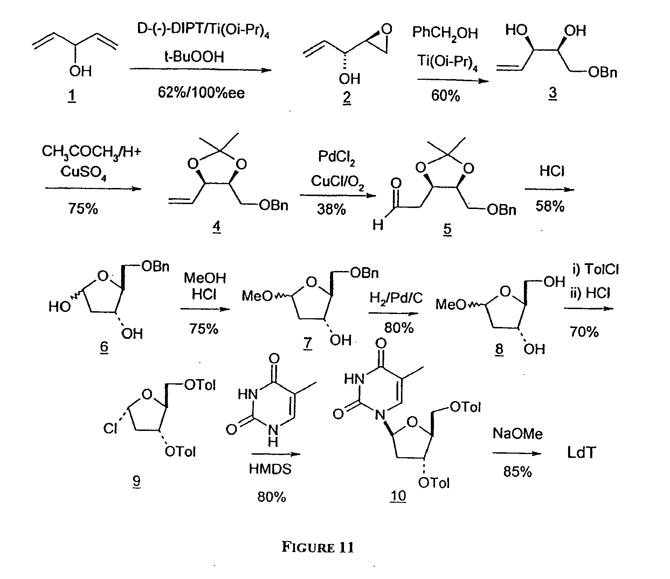

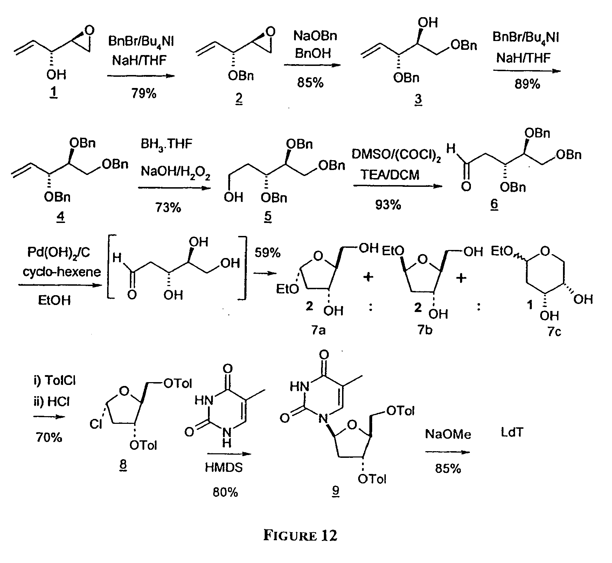

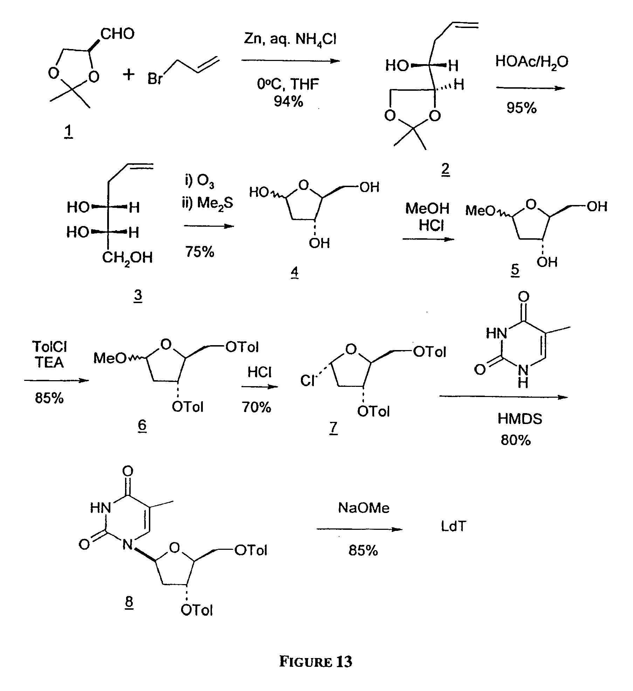

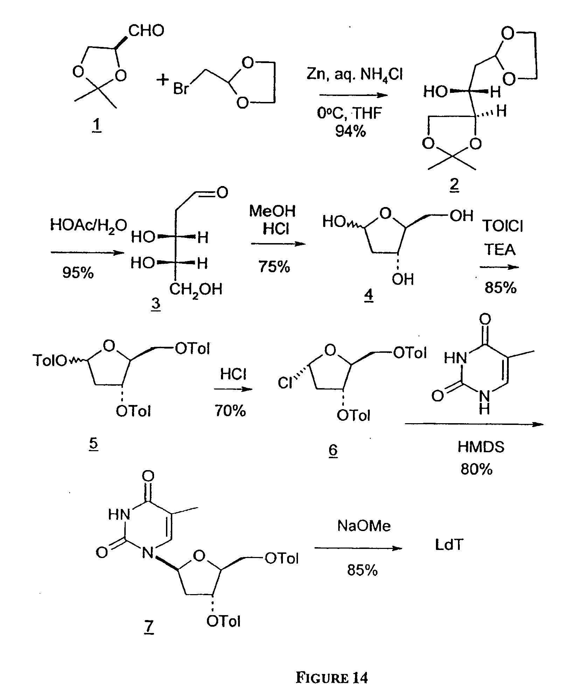

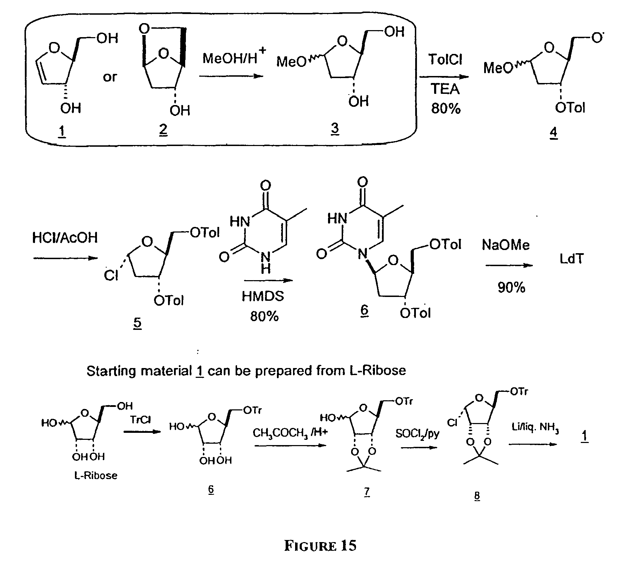

The present invention also provides additional processes for preparing 2'-deoxynucleosides and 2'-deoxythymidine in particular, from starting materials that are non-carbohydrates **(****Figure 8****),** dioxolanyl derivatives (**Figure 14**), acids, esters and aldehydes (**Figures 9****,** **10****,** **13**), glycal (**Figure 15**), and alcohols (**Figures 11** **and** **12**). Details of these syntheses may be found in the Examples contained herein, which are the preferred embodiments (see **Figures 1-23**).

Processes also are provided for using a reducing agent, such as Red-Al, in combination with a sequestering agent, such as 15-crown-5 ether, to rupture a 2,2'-anhydro-1-furanosyl-nucleoside ring intermediate to produce the desired 2'-deoxy nucleoside product. Alternatively, processes are provided for using a reducing agent, such as Red-Al, in the absence of a sequestering agent, to rupture a 2,2'-anhydro-1-furanosyl-nucleoside ring intermediate to produce the desired 2'-deoxy nucleoside product. Alternatively, a 2,3'-anhydro-1-furanosyl-nucleoside ring intermediate can be used to form the corresponding 3'-deoxy nucleoside.

Any reducing agents known in the art which provide the necessary chemoselective and regioselective reduction may be used. Suitable reducing agents include Red-Al, Red-Al (sodium bis[2-methoxyethoxy]-aluminum hydride), NaHTe, SmI₂, H₂ + Pd-phosphine catalyst, and LiAl(O^{t}Bu)₃H (lithium tri-tertiary butyoxy aluminum hydride).

The ring-opening reaction can be carried out at any temperature that achieves the desired results, i.e., that is suitable for the reaction to proceed at an acceptable rate without promoting decomposition or excessive side products, preferably at reduced temperatures, such as from about 0-5 °C.

Any reaction solvent can be selected that can achieve the necessary temperature and that can solubilize the reaction components. Non-limiting examples are any polar aprotic solvent including, but not limiting to, dichloromethane (DCM) or dichloroethane, acetone, ethyl acetate, dithianes, THF, 1,2-dimethoxyethane (DME), dioxane, acetonitrile, diethyl ether, pyridine, dimethylformamide (DMF), dimethylsulfoxide (DMSO), dimethylacetamide, or any combination thereof, though preferably THF and/or DME.

Alternatively, processes are provided for using an appropriate nucleophilic agent, e.g. an organometallic agent (e.g. a Grignard reagent or an alkyl lithium reagent) if an alkyl substituent is desired, to open the 2,2'-anhydro-1-furanosyl-nucleoside ring intermediate to produce the desired 2'-substituted nucleoside product. In another embodiment, a 2,3'-anhydro-1-furanosyl-nucleoside ring intermediate or 2,5'-anhydro-1-furanosyl-nucleoside ring intermediate may be used to form the desired 3'-substituted or 5'-substituted nucleoside product.

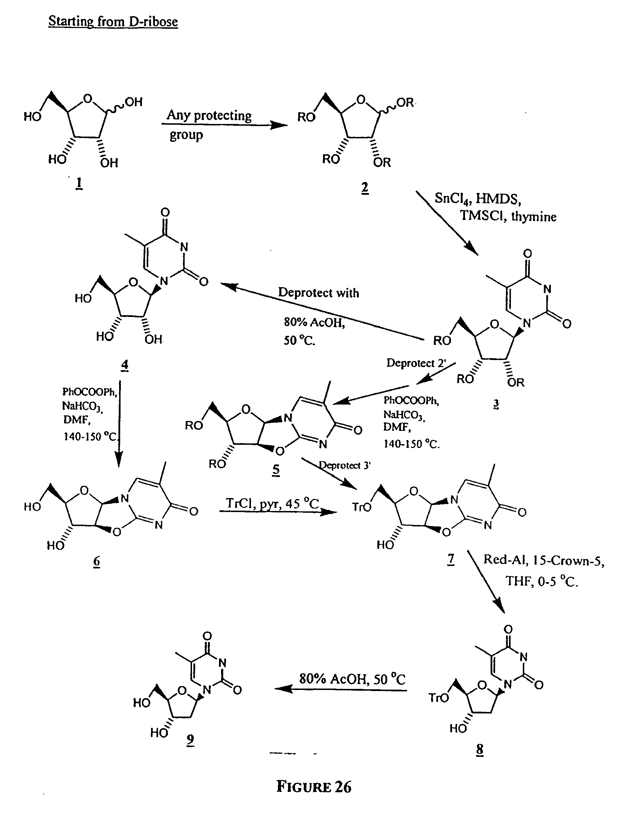

Specifically, in one embodiment, the present invention is directed to a process for preparing a 2'-deoxynucleoside or 2'-modified nucleoside that comprises (a) optionally protecting one or more hydroxyl groups on a furanosyl ring, such as a ribo-, arabino-, or xylo-furanosyl ring, by reaction with a protecting group **(2);** (b) condensing the optionally protected furanosyl ring from step (a) with an optionally substituted natural or non-natural nucleoside base to form a nucleoside **(3);** (c) reacting the nucleoside from step (b) with a condensing agent at an elevated temperature to afford a 2,2'-anhydro-1-furanosyl-nucleoside **(5);** (d) reacting the 2,2'-anhydro-1-furanosyl-nucleoside from step (c) with a reducing agent, such as Red-Al, and a sequestering agent, such as 15-crown-5 ether, preferably in a polar solvent at a low temperature, to afford an optionally protected 2'-deoxynucleoside or 2'-substituted nucleoside (**8**); and (e) deprotecting the optionally protected hydroxyl groups, if necessary or desired, for example by the addition of acids or acid resins at a temperature of about 50°C **(9) (****Figure 26****).**

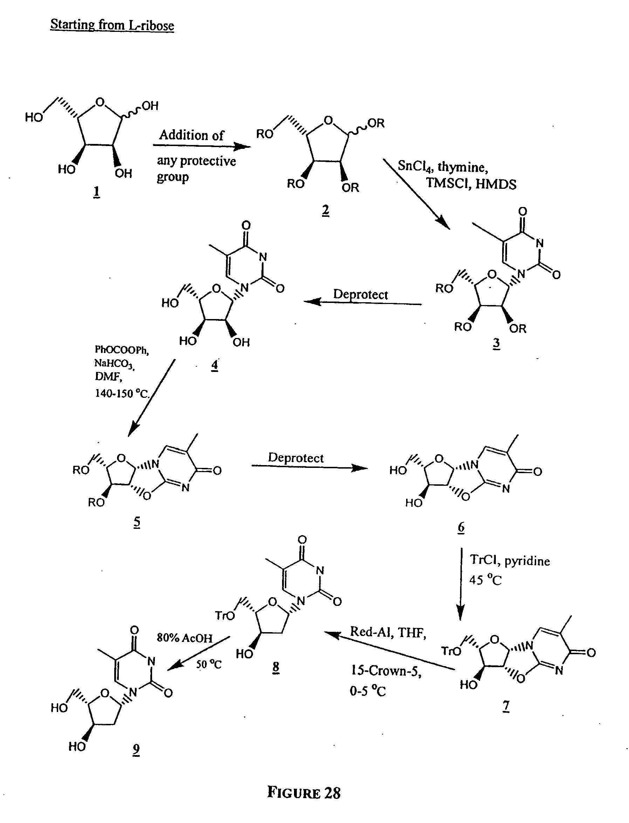

In another embodiment, a process for preparing a 2'-deoxythymidine is provided that comprises (a) optionally protecting one or more hydroxyl groups on a furanosyl ring by reaction with a protective group **(2);** (b) reacting the optionally protected furanosyl ring with cyanamide to form an optionally protected furanosylaminooxazoline **(3);** (c) reacting the optionally protected furanosylaminooxazoline with a cyclization or condensation agent to afford an optionally protected 2,2'-anhydro-1-furanosyl-thymidine **(5);** (d) reacting the optionally protected 2,2'-anhydro-1-furanosyl-thymidine with a reducing agent, such as Red-Al, and a sequestering agent, such as 15-crown-5 ether, preferably in a polar solvent at a low temperature to provide an optionally protected, 2'-deoxythymidine **(8);** and (e) deprotecting the optionally protected 2'-deoxythymidine, if necessary or desired, for example, by reaction with acids or acid resins at about 50°C to provide 2'-deoxythymidine **(9). (****Figure 28****)**

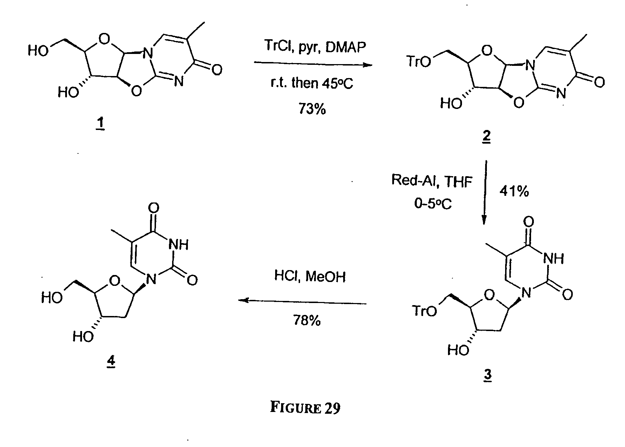

In yet another embodiment, the present invention is directed to a process for preparing a 2'-deoxynucleoside or 2'-modified nucleoside that comprises (a) condensing a furanosyl ring with an optionally substituted natural or non-natural nucleoside base to form a nucleoside; (b) reacting the nucleoside from step (a) with a condensing agent at an elevated temperature to afford a 2,2'-anhydro-1-furanosyl-nucleoside **(1);** (c) reacting the 2,2'-anhydro-l-furanosyl-nucleoside from step (b) with a protecting agent such as a trityl protecting group to protect the 5'-position on the nucleoside **(2)**; (d) adding a reducing agent, such as Red-Al, preferably in a polar solvent at a low temperature, to afford an optionally protected 2'-deoxynucleoside or 2'-substituted nucleoside **(3);** and (e) deprotecting the optionally protected hydroxyl groups, if necessary or desired, for example by the addition of acids or acid resins at a temperature of about 50°C. **(4) (****Figure 29****).**

Preferred embodiments are contained in **Figures 1-29****.**

### Definitions

In the present invention, the term "isolated" refers to a nucleoside composition that includes at least 85% or 90% by weight, preferably 95% to 98% by weight, and even more preferably 99% to 100% by weight, of the nucleoside, the remainder comprising other chemical species or enantiomers.

The term "protected", as used herein and unless specified otherwise, refers to a group that is added to an oxygen, nitrogen or phosphorus atom to prevent its further reaction or for other purposes. A wide variety of oxygen, nitrogen and phosphorus protecting groups are known to those skilled in the art of organic synthesis.

Examples of suitable protecting groups include, but not limited to, benzoyl; substituted or unsubstituted alkyl groups, substituted or unsubstituted aryl groups, substituted or unsubstituted silyl groups; substituted or unsubstituted aromatic or aliphatic esters, such as, for example, aromatic groups like benzoyl, toluoyls (e.g. p-toluoyl), nitrobenzoyl, chlorobenzoyl; ether groups such as, for example, -C-O-aralkyl, - C-O-alkyl, or -C-O-aryl; and aliphatic groups like acyl or acetyl groups, including any substituted or unsubstituted aromatic or aliphatic acyl, -(C=O)-aralkyl, -(C=O)-alkyl, or-(C=O)-aryl; wherein the aromatic or aliphatic moiety of the acyl group can be straight-chained or branched; all of which may be further optionally substituted by groups not affected by the reactions comprising the improved synthesis (see Greene et al., Protective Groups in Organic Synthesis, John Wiley and Sons, 2nd Edition (1991)). For example, in one embodiment of the invention, the protecting groups are substituted by groups not affected by the reducing agent of choice, such as Red-Al. For the use of ethers as protective groups, attention is directed to U.S. 6,229,008 to Saischek et al., herein incorporated by reference, wherein it is reported that the use of an ether as a protective group may offer significant advantages, particularly at the 5' position of a pentofuranoside, for stability toward reagents and process conditions. This affords an ultimate advantage for separation, isolation, and purification of the desired product and thus, on the product's percent yield.

The sugar hydroxyl protecting groups can be as nonlimiting examples, silyl, benzoyl, p-toluoyl, p-nitrobenzoyl, p-chlorobenzoyl, acyl, acetyl, -(C=O)-alkyl, and -(C=O)-aryl, all of which may be unsubstituted or substituted by one or more groups not affected by the selected reducing agent. In one embodiment, the sugar hydroxyl protecting group is benzoyl. The amino acid protecting groups are preferably BOC (butoxycarbonyl), -(C=O)-aralkyl, -(C=O)-alkyl or -(C=O)-aryl. In one embodiment of the invention, the amino-protecting group is BOC (butoxycarbonyl).

The term "alkyl", as used herein and unless specified otherwise, includes a saturated or unsaturated, straight, branched, or cyclic, primary, secondary or tertiary hydrocarbon of typically C₁ to C₁₀, and specifically includes methyl, trifluoromethyl, ethyl, propyl, isopropyl, cyclopropyl, butyl, isobutyl, *t*-butyl, pentyl, cyclopentyl, isopentyl, neopentyl, hexyl, isohexyl, cyclohexyl, cyclohexylmethyl, methylpentyl and dimethylbutyl. The term includes both substituted and unsubstituted alkyl, alkylene, alkenyl, alkenylene, alkynyl, and alkynylene groups. Moieties with which the alkyl group can be substituted in one or more positions are selected from the group consisting of halo (including fluorine, chlorine, bromine or iodine), hydroxyl (eg. CH₂OH), amino (eg., CH₂NH₂, CH₂NHCH₃ or CH₂N(CH₃)₂), alkylamino, arylamino, alkoxy, aryloxy, nitro, azido (eg., CH₂N₃), cyano (CH₂CN), sulfonic acid, sulfate, phosphonic acid, phosphate or phosphonate, any or all of which may be unprotected or further protected as necessary, as known to those skilled in the art and as taught, for example, in Greene et al., Protective Groups in Organic Synthesis, John Wiley and Sons, 2nd Edition (1991).

The term "aryl", as used herein, and unless specified otherwise, refers to phenyl, biphenyl or naphthyl. The term includes both substituted and unsubstituted moieties. The aryl group can be substituted with one or more moieties including but not limited to hydroxyl, amino, alkylamino, arylamino, alkoxy, aryloxy, nitro, cyano, sulfonic acid, sulfate, phosphonic acid, phosphate, or phosphonate, any or all of which may be unprotected or further protected as necessary, as known to those skilled in the art and as taught, for example, in Greene et al., Protective Groups in Organic Synthesis, John Wiley and Sons, 2nd Edition (1991).

The term "acyl" includes a -C(=O)-R in which the non-carbonyl moiety R is for example, straight, branched, or cyclic alkyl or lower alkyl, alkoxyalkyl including methoxymethyl, aralkyl including benzyl, aryloxyalkyl such as phenoxymethyl, aryl including phenyl optionally substituted with halogen, C₁ to C₄ alkyl or C₁ to C₄ alkoxy, sulfonate esters such as alkyl or aralkyl sulphonyl including methanesulfonyl, the mono-, di- or tri-phosphate ester, trityl or monomethoxytrityl, substituted benzyl, trialkylsilyl such as, for example, dimethyl-*t*-butylsilyl), or diphenylmethylsilyl. Aryl groups in the esters optimally comprise a phenyl group. The term "lower acyl" refers to an acyl group in which the non-carbonyl moiety is lower alkyl.

The term pyrimidine nucleoside base, includes a pyrimidine or pyrimidine analog base. Examples of pyrimidine or pyrimidine analog bases include, but are not limited to, thymine, cytosine, 5-fluorocytosine, 5-methylcytosine, 6-azapyrimidine, including 6-aza-cytosine, 2- and/or 4-mercaptopyrmidine, uracil, 5-halouracil, including 5-fluorouracil, C⁵-alkylpyrimidines, C⁵-benzylpyrimidines, C⁵-halopyrimidines, C⁵-vinylpyrimidine, C⁵-acetylenic pyrimidine, C⁵-acyl pyrimidine, C⁵-amidopyrimidine, C⁵-cyanopyrimidine, C⁵-nitropyrimidine, C⁵-aminopyrimidine, 5-azacytidinyl, 5-azauracilyl, triazolopyridinyl, imidazolopyridinyl, pyrrolopyrimidinyl, and pyrazolo-pyrimidinyl. Functional oxygen and nitrogen groups on the base can be protected as necessary or desired. Suitable protecting groups are well known to those skilled in the art, and include trimethylsilyl, dimethylhexylsilyl, *t*-butyldimethylsilyl and *t*-butyldiphenylsilyl, trityl, alkyl groups, and acyl groups such as acetyl and propionyl, methanesulfonyl, and p-toluenesulfonyl. Alternatively, the pyrimidine base or pyrimidine analog base can optionally substituted such that it forms a viable prodrug, which can be cleaved in vivo. Examples of appropriate substituents include acyl moiety, an amine or cyclopropyl (e.g., 2-amino, 2,6-diamino or cyclopropyl guanosine).

Other reagents used in the process of the present invention or the prior art are defined as: AIBN is azobis(isobutyronitrile; BSA (bis(trimethylsilyl)acetamide); CAN is ceric ammonium nitrate; DIBAL is diisobutylaluminum hydride; TMSCI is chlorotrimethylsilane; TFA is trifluoroacetic acid; TEA is triethylamine; TFAA is trifluoroacetic anhydride; TBDPSC1 is tert-butyldiphenylsilyl chloride; TBDMSC1 is tert-butyldimethylsityl chloride; TBTN is tri-n-butyltin hydride; DET is diethyl tartrate; TBS is *t*-butyldimethylsilyl; DMTrCl is dimethoxytrityl chloride; DME is 1,2-dimethoxyethane; "Pyr" is used as an abbreviation for pyridine; DMAP is 4-dimethylaminopyridine; DIBAL is diisobutylaluminium hydride; PhOCO₂Ph is diphenylcarbonate; HMDS is hexamethyldisilazide; and DCM is dichloromethane.

The process of the present invention is not limited to the use of the nucleoside and reagents exemplified. Suitable alternative reagents for the present invention may be used in place of those given above. For example, DME (1,2-dimethoxyethane) may be replaced by any suitable polar aprotic solvent, such as THF (tetrahydrofuran) or any ether; and Red-Al (sodium bis[2-methoxyethoxy]-aluminum hydride) in toluene can be replaced by NaHTe, SmI₂, H₂ + Pd-phosphine catalyst, or LiAl (O^{t}Bu)₃H (lithium tri-tertiary butyoxy aluminum hydride), all of which produce chemoselective and regioselective reductions.

### Detailed Description of Process Steps

### Anhydro-1-furanosyl-nucleoside ring intermediate

One key compound for the processes for the present invention is a 2,2'-anhydro-1-furanosyl-nucleoside, for example an α or β, D or L, 2,2'-anhydro-1-furanosyl-nucleoside of the general formula: 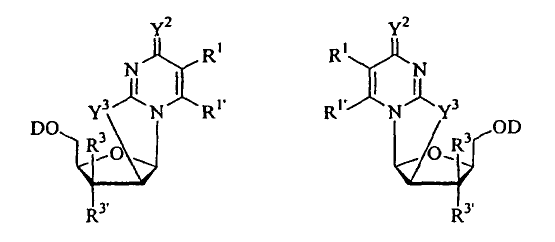 wherein:
each D is a hydrogen or a suitable hydroxyl protecting group, such as a substituted or unsubstituted alkyl, substituted or unsubstituted aryl, substituted or unsubstituted acyl, silyl, or amino acid;
each R¹ and R^{1'} is independently hydrogen, substituted or unsubstituted lower alkyl, substituted or unsubstituted lower alkenyl, substituted or unsubstituted lower alkynyl, substituted or unsubstituted aryl, alkylaryl, halogen (F, Cl, Br or I), NH₂, NHR⁵, NR⁵R^{5'}, NHOR⁵, NR⁵NHR^{5'}, NR⁵NR^{5'}R^{5"}, OH, OR⁵, SH, SR⁵, NO_{2,} NO, CH₂OH, CH₂OR⁵, CO₂H, CO₂R⁵, CONH₂, CONHR⁵, CONR⁵R^{5'} or CN;
each R³ and R^{3'} independently is hydrogen or halogen (F, Cl, Br or I), OH, SH, OCH₃, SCH₃, NH₂, NHCH₃, CH₃, C₂H₅, CH=CH₂, CN, CH₂NH₂, CH₂OH, or CO₂H;
each Y² is O, S, NH or NR⁶;
each Y³ is O, S, NH or NR⁷; and
each R⁵, R^{5'}, R⁶ and R⁷ is independently hydrogen, substituted or unsubstituted lower alkyl of C₁-C₆, arylalkyl or substituted or unsubstituted aryl.

The 2,2'-anhydro-1-furanosyl-nucleoside can be purchased or synthesized by any means known in the art, including from furanyl sugars with a 2'-hydroxyl using standard sugar coupling techniques followed by condensation to form the 2,2'-anhydro compound, or alternatively, coupling the sugar with a cyanamide to obtain an oxazoline intermediate, then building the base with requisite cyclization or condensation agent.

In particular embodiments of the present invention, the β- or α, D- or L-, 2'-deoxy or 2'-substituted nucleoside are prepared via the 2,2'-anhydro-1-furanosyl-nucleoside according to the following protocols.

### From Arabino-Furanose

In one process of the present invention, a furanose, such as an L-furanose, and in particular L-arabinose, can be used as the starting material to prepare the 2,2'-anhydro-1-furanosyl-nucleoside, which is then reduced according to the present invention to give a 2'-deoxynucleoside, such as a β-L-2'-deoxynucleoside, and in particular, β-L-2'-deoxythymidine. Alternatively, the 2,2'-anhydro-1-furanosyl-nucleoside can be reacted with a nucleophilic agent, e.g. an organometallic agent (e.g. a Grignard reagent or an alkyl lithium reagent), to give the desired 2'-substituted nucleoside.

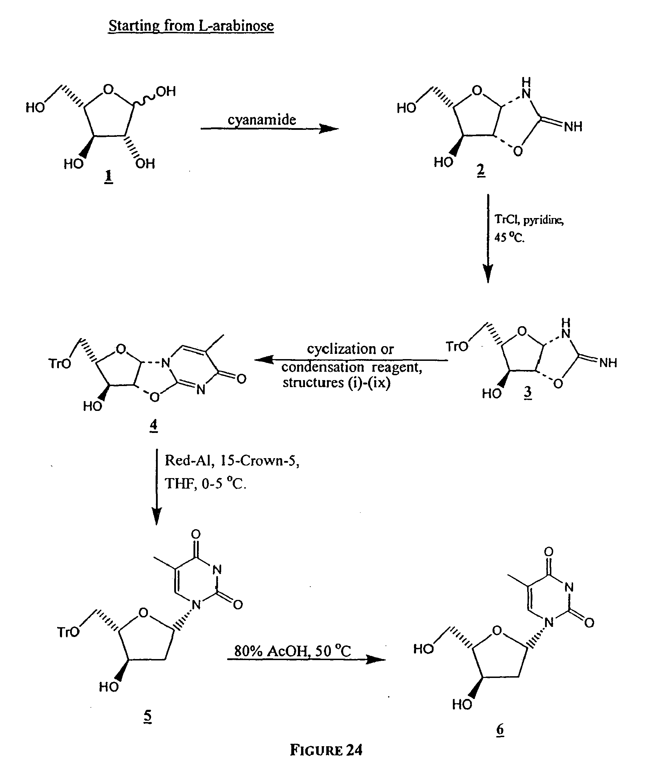

**Figure 24** of the present invention utilizes L-arabinose **(1)** as a starting material to prepare β-L-2'-deoxy-thymidine in a 5-step synthesis. L-arabinose **(1)** initially is reacted with cyanamide under conditions taught in the prior art to form the intermediate, L-arabinofuranosyl amino oxazoline **(2)** (see WO 02/44194). Next, the 5'-OH on the arabinose moiety of the L-arabinofuranosyl amino oxazoline intermediate **(2)** is protected by its reaction with trityl chloride (TrCl) and pyridine at a temperature of about 45 °C **(3).** The addition of an OH-protecting group under these conditions is also well known to those skilled in the art (Greene et al., Protective Groups in Organic Synthesis, (1991) John Wiley and Sons, 2nd Edition).

Step 3 of the process depicted in **Figure 24** shows the reaction under appropriate conditions as taught in the prior art of the 5'-trityl-protected L-arabinofuranosyl amino oxazoline **(3)** with a cyclization or condensation agent selected from any of the following:

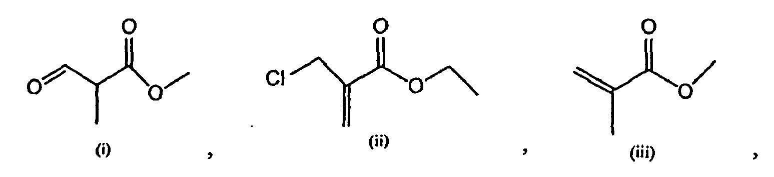

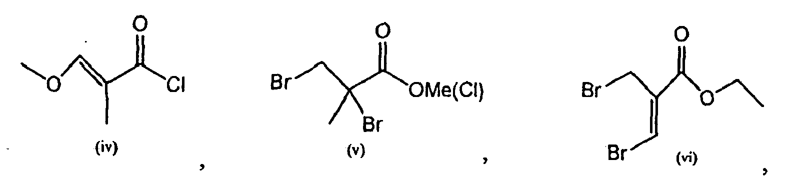

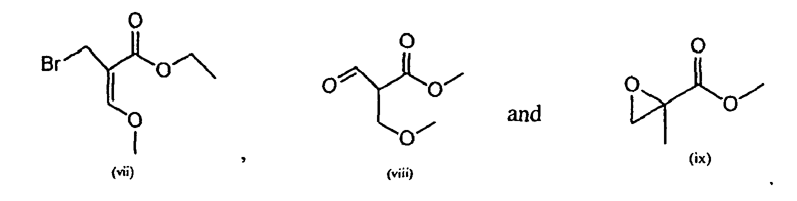

For example, if structure (ii) shown above is used as a condensing or cyclization agent, the reaction is carried out in the presence of Na₂CO₃/H₂O and the subsequent isomerization is effected by the addition of Pd/Al₂O₃/H₂O (see WO 02/44194). However, if the condensing or cyclization agent (i) is used, the reaction is carried out in methyl 2-formylpropionate at reflux for 1 hour (see EP 0 351 126). Cyclization results in the formation of 2,2'-anhydro-1-(L-arabinofuranosyl)thymidine **(4).**

The next step of the present invention involves the reduction of 2,2'-anhydro-1-(L-arabinofuranosyl)thymidine **(4)** with a reducing agent such as Red-Al, and a sequestering agent such as 15-crown-5 ether, in the presence of a polar solvent such as THF and/or DME, preferably at reduced temperatures such as from about 0-5 °C to provide β-L-5'-trityl-2'-deoxythymidine **(5).**

Using a sequestering agent such as 15-crown-5 ether at this step is advantageous due to an increase in solubility of the 2,2'-anhydro-1-(L-arabinofuranosyl)thymidine that results in higher percent yield of product, and the avoidance of using reagents such as palladium catalysts whose removal requires labor-intensive efforts. Moreover, the use of 15-crown-5 ether circumvents the use of HBr to open the anhydro-ring structure, necessitating the use of H₂ with poisoned catalyst Pd-BaSO₄ to remove bromide (see EP 0 351 126), thereby avoiding the use of dangerous reagents found in certain prior art processes. Finally, the process of the present invention avoids the use of dioxane as a reagent. This is advantageous because dioxane is flammable and inappropriate for an industrially scalable synthesis.

The final step in the process shown in **Figure 24** is removal of the trityl-protective group from the 5'-position on β-L-2'-deoxythymidine **(5)** by treatment with 80% AcOH at a temperature of about 50°C to form L-2'-deoxythymidine **(6).**

Alternatively, selective protection of L-arabinose **(1)** at the C-5 position using trityl is possible by reacting L-arabinose **(1)** with TrCl (trityl chloride) and pyridine at a temperature of about 45°C to form 5-TrO-L-arabinose (structure not shown). Next the 5-TrO-L-arabinose is reacted with cyanamide under conditions taught in the prior art to form the intermediate, 5-TrO-L-arabinofuranosyl amino oxazoline **(3)** (see WO 02/44194). The remainder of the steps in the process are as given in **Figure 24** for formation of structures **(4), (5),** and **(6).**

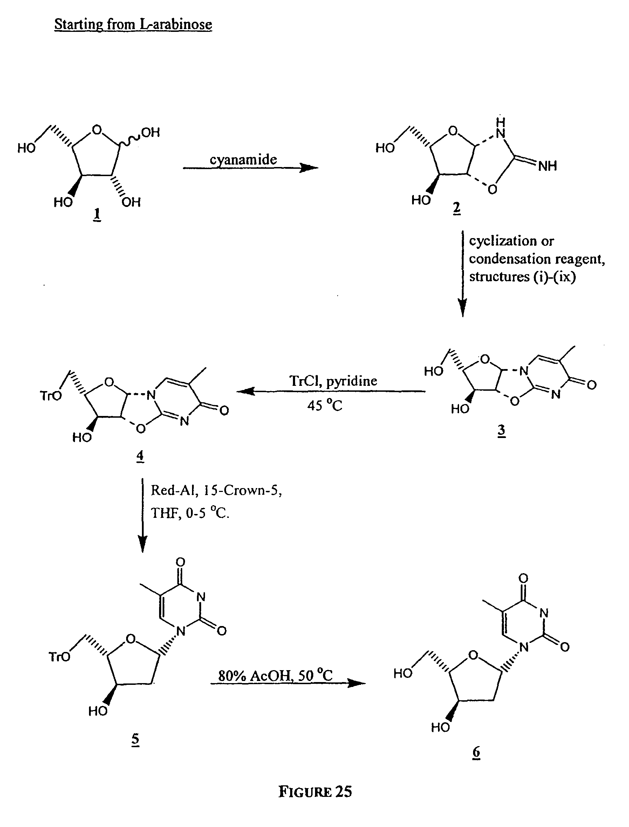

**Figure 25** shows a synthesis of the present invention that is similar to that depicted in **Figure 24** but differs in the steps in which intermediates are OH-protected. As in **Figure 24****,** L-arabinose **(1)** is used as a starting material and is reacted with cyanamide to afford L-arabinofuranosyl amino oxazoline as an intermediate **(2).** L-Arabinofuranosyl amino oxazoline **(2)** is then reacted with any one of the cyclization/condensation reagents given above in structures (i)-(ix) under appropriate conditions as taught in the prior art to provide 2,2'-anhydro-1-(L-arabinofuranosyl)thymidine **(3).** The 2,2'-anhydro-l-(L-arabinofuranosyl)thymidine **(3)** next is reacted with TrCl and pyridine at a temperature of about 45 °C to protect the 5'-OH on the arabinose moiety of the 2,2'-anhydro compound **(4).** This step should be compared to **Figure 24****,** step 2, where a trityl group was added to the 5'-of the arabino moiety of the arabinofuranosyl amino oxazoline prior to reacting it with a cyclization or condensation reagent. The last 2 steps of the 5-step process shown in **Figure 25** are identical to the last 2 steps provided in **Figure 24** and provide 5'-trityl-protected thymidine **(5)** and deprotected 2'-deoxythymidine **(6).**

Applicants concluded that the process described in **Figure 24** is more efficient than that depicted in **Figure 25** based on the addition of the trityl-protective group at the 5'-position in a step that occurs earlier in the synthesis than heretofore seen in the prior art.

### From Ribo-Furanose

In another process of the present invention, a furanose, such as an L-furanose, and in particular L-ribose, can be used as the starting material to prepare the 2,2'-anhydro-1-furanosyl-nucleoside, which is then reduced according to the present invention to give a 2'-deoxynucleoside, such as a β-L-2'-deoxynucleoside, and in particular, β-L-2'-deoxythymidine. Alternatively, the 2,2'-anhydro-1-furanosyl-nucleoside can be reacted with a nucleophilic agent, e.g. an organometallic agent (e.g. a Grignard reagent or an alkyl lithium reagent), to give the desired 2'-substituted nucleoside.

**Figure 26** shows a 7-step synthesis for making 2'-deoxythymidine utilizing D-ribose as a starting material. In the first step of this process, all OH groups on D-ribose are protected such as, for example, with acetyl or benzoyl groups as known by those skilled in the art. The protected D-ribose next is reacted with thymine in the presence of, for example, SnCl₄, HMDS, and TMSCI as known in the prior art to provide thymidine that has protective groups at the 2'-, 3'- and 5'-positions on the nucleoside. The protective groups are removed in step 3 by reagents and under conditions appropriate for removal of the particular protective group attached. The intermediate produced in step 3 is thymidine.

Step 4 in **Figure 26** introduces a cyclization/condensation step directly from thymidine rather than from the furanosyl amino oxazoline as shown m **Figures 24** and **25**. Here, thymidine is reacted with PhOCOOPh and NaHCO₃ catalyst in the presence of DMF at a temperature of about 150 °C to afford 2,2'-anhydro-1-(ribofuranosyl)-thymidine.

The 2,2'-anhydro-1-(ribofuranosyl) thymidine structures **5** and **6** represent two separate embodiments of the present invention, in that structure **5** is derived from the thymidine structure **4** with protective groups at the 3'- and 5'-positions on the ribo moiety of thymidine, and structure **6** is derived from a thymidine structure wherein the 3'- and 5'-positions on the ribo moiety of thymidine are unprotected. In either instance, the 2'-OH of thymidine must be a free OH group so that it can participate in the reaction providing the 2,2'-anhydro-1-(ribofuranosyl)thymidine structure. If the synthesis proceeds via structure **5**, in one embodiment, the 5'-protective group is trityl; an additional step may then be performed to remove the protective group from the 3'-position of the ribo moiety prior to reduction with a reducing agent such as Red-Al, and a sequestering agent such as 15-crown-5 ether, to form structure **(8).**

In an embodiment of the present invention depicted in **Figure 26****,** the synthesis proceeds via structure **6,** where TrCl and pyridine are reacted with 2,2'-anhydro-1-(ribofuranosyl)thymidine at about 45°C to provide 5'-tritylated 2,2'-anhydro-1-(ribofuranosyl)thymidine that is structure **(7).** 5'-Tritylated 2,2'-anhydro-1-(ribofuranosyl)thymidine then is reduced by reacting it with a reducing agent such as Red-Al, and a sequestering agent such as 15-crown-5 ether, in a polar solvent, such as THF and/or DME, preferably at a temperature of about 0-5 °C. This step affords 5'-tritylated-2'-deoxythymidine **(8),** which is then deprotected by reacting it with 80% AcOH at about 50°C to provide D-2'-deoxythymidine **(9).** The process shown in **Figure 26** provides a means for preparing a 2,2'-anhydro-furanosyl-thymidine directly from thymidine or protected thymidine without involving a furanosyl-amino-oxazolidine intermediate and an accompanying condensation or cyclization step.

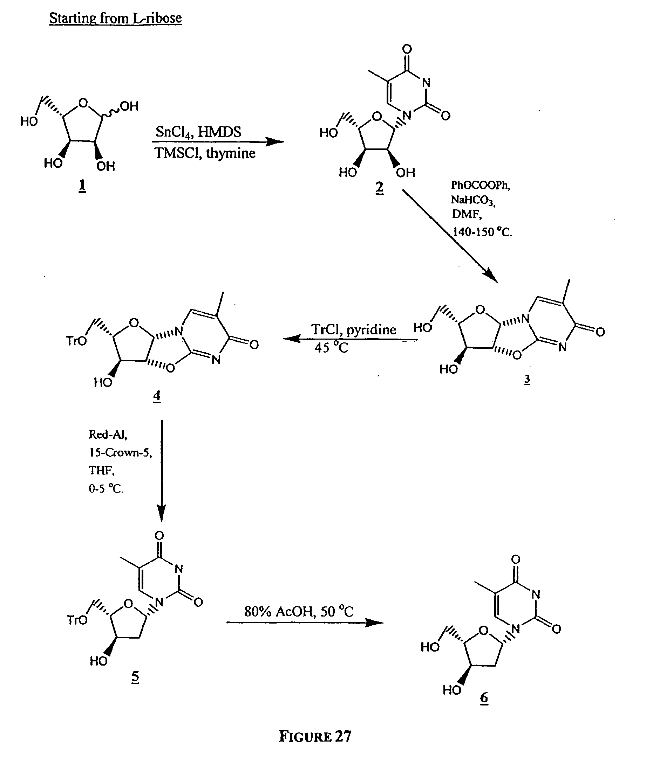

**Figure 27** shows a 5-step process starting from L-ribose to prepare L-2'-deoxy-thymidine. In this synthesis, L-ribose is reacted with thymine and SnCl₄ in TMSCl and HMDS to form thymidine **(2).** Thymidine next is reacted with PhOCOOPh and NaHCO₃ catalyst in DMF at about 150°C to provide L-2,2'-anhydro-ribofuranosyl-thymidine **(3).** This L-2,2'-anhydro-ribofuranosyl-thymidine is reacted with TrCl and pyridine at about 45°C to afford 5'-trityl-protected L-2,2'-anhydro-ribofuranosyl-thymidine **(4),** and this, in turn, is reduced by reaction with a reducing agent such as Red-Al, and a sequestering agent such as 15-crown-5 ether, in a polar solvent such as THF and/or DME, preferably at temperatures of from about 0-5 °C to produce 5'-trityl-L-2'-deoxy-thymidine **(5).** Finally compound **(5)** is deprotected by reacting it with 80% AcOH at about 50°C to form L-2'-deoxythymidine **(6).** This synthesis is both efficient in the number of steps required, and also avoids formation of a ribofuranosyl-amino-oxazolidine.

**Figure 28** depicts an 8-step process for preparing 2'-deoxythymidine from L-ribose. L-ribose **(1)** initially is protected by any protecting group under conditions appropriate for using that protective group **(2)** as known by one skilled in the art. Protected L-ribose **(2)** is reacted with thymine and SnCl₄ in the presence of TMSCI and HMDS, a step known in the prior art, to form thymidine that has protective groups at its 2'-, 3'-, and 5'-positions **(3).** The protected thymidine **(3)** next is deprotected **(4)** by using reagents and conditions appropriate for the removal of the particular protective group utilized, and the unprotected thymidine **(4)** is reacted with PhOCOOPh and NaHCO₃ catalyst in the presence of DMF at about 140-150 °C to form 2,2'-anhydro-1-ribofuranosyl-thymidine **(5)** or **(6).** It is to be noted that if 2,2'-anhydro-1-ribofuranosyl-thymidine **(5)** is the intermediate prepared, an additional step is required after the formation of intermediate **(4)** in which the 3'- and 5'-positions on thymidine are reacted to place protective groups at those positions. Trityl groups are the preferred protective groups for this intermediate. If intermediate **(6)** is prepared, it can be prepared directly from the thymidine structure **(4).**

Next, intermediate **(5),** if used, must undergo deprotection at its 3'-position with reagents and under conditions appropriate to remove the protective group from this position, in order to provide the 5'-trityl-protected 2,2'-anhydro-l-ribofuranosyl-thymidine **(7).** However, if intermediate **(6)** is used, it can be reacted with TrCl and pyridine at about 45 °C to provide 5'-trityl-protected 2,2'-anhydro-1-ribofuranosyl-thymidine **(7).**

5'-Trityl-protected 2,2'-anhydro-1-ribofuranosyl-thymidine **(7)** then is reduced with a reducing agent such as Red-Al, and a sequestering agent such as 15-crown-5 ether, in a polar solvent such as THF and/or DME, preferably at a temperature of from about 0-5 °C. to afford 5'-trityl-protected thymidine **(8),** which is then deprotected by reaction with 80% AcOH at a temperature of about 50 °C to provide L-ribo-2'-deoxythymidine **(9).**

The synthesis depicted in **Figure 28** avoids proceeding through a furanosyl-amino-oxazolidine intermediate that requires an additional condensation step to form its corresponding 2,2'-anhydro compound. It also permits choices with respect to which intermediates should be protected at different steps in the process.

In another process of the present invention, the synthesis of the desired compounds can be accomplished in the absence of a sequestering agent (see **Figure 29**). The use of a sequestering agent such as, for example, 15-crown-5 ether, affords a higher percent product yield when dimethoxy trityl is the protecting group, however, when trityl alone is used as a protecting group, the use of a sequestering agent such as, for example, 15-crown-5 ether, affords a lower percent product yield. Thus, in some embodiments of the invention, the synthesis of the desired compounds can be accomplished in the absence of a sequestering agent when trityl is used as the protecting group, as in **Figure 29****.**

**Figure 29** depicts a 3-step process for preparing 2'-deoxythymidine. The process comprises:
**(a)** preparing 2,2'-anhydro-1-(S-*O*-trityl-□-D-arabinofuranosyl) thymine **(2)** from 2,2'-anhydro-1-(□-D-arabinofuranosyl) thymine **(1)** by suspending 2,2'-anhydro-1-(□-D-arabinofuranosyl) thymine **(1)** in, for example, pyridine and DMAP, and adding trityl chloride portion-wise, for example at room temperature. The reaction mixture can be maintained at room temperature or heated as need, for example, the reaction mixture can be maintained at room temperature for about 1 hour and then heated to 45°C (internal temperature) for about 15 hours. The reaction can be monitored, for example by t.1.c. (starting material R_{f} 0.15; product R_{f} 0.43). The reaction mixture can then be quenched and the desired product purified, for example by cooling to about 0°C and slowly adding saturated aqueous NaHCO₃ solution over a 15 minute period of time with no change in internal temperature. A white solid can be immediately precipitated from solution and the white suspension can then be stirred for 30 minutes at room temperature. The solid can be isolated by filtration through a Buchner funnel and subsequently washed with water. The residual solid can be taken up into dichloromethane and stirred for about 30 minutes at room temperature. The remaining residue can be isolated by filtration through a Buchner funnel, washed with dichloromethane, and dried under vacuum overnight to yield 2,2'-anhydro-1-(5-*O*-trityl-□-D-arabinofuranosyl) thymine **(2)** in an approximate 73% yield as a white solid;
**(b)** preparing 2'-deoxy-5'-*O*-trityl-□-D-thymidine **(3)** from 2,2'-anhydro-1-(5-*O*-trityl-□-D-arabinofuranosyl) thymine **(2)**_by reducing 2,2-anhydro-1-(5-*O*-trityl-□-D-arabinofuranosyl) thymine **(2),** for example, by suspending **(2)** in anhydrous tetrahydrofuran and cooling the suspension to about 0-5°C in an ice-bath. In a separate flask immersed in the ice-bath, a 65% wt solution of Red-Al in toluene can be diluted with the appropriate solvent, for example, by addition to anhydrous tetrahydrofuran. This diluted Red-Al solution can then be cooled to about 0-5°C and added dropwise *via* syringe to the suspension of 2,2'-anhydro-1-(5-*O*-trityl-□-D-arabinofuranosyl) thymine **(2).** The rate of dropwise addition of the Red-Al solution is critical to the reaction and can be completed in about 1 hour. The resulting clear solution was maintained at about 0-5°C for 1 hour after which time, t.l.c. analysis indicated the presence of starting material (R_{f} 0.34), required product (R_{f} 0.47) and impurities (R_{f} 0.42 and 0.26). HPLC analysis indicated presence of starting material (11.35 mins, 36.5% AUC), product (12.60 mins, 24%) and little of the major impurity (11.7 mins, 2.9%). After a total of about 2 hours at about 0-5°C, an additional portion of an "undiluted" 65% wt solution of Red-Al in toluene was added dropwise *via* syringe over a period of about 20 minutes to the reaction mixture which was maintained at about 0-5°C. After a further 1 hour, t.l.c. and HPLC analysis indicated presence of starting material (11.35 mins, 3.2%). A further portion of a 65% wt solution of Red-Al in toluene was added dropwise and the reaction mixture maintained at about 0-5°C for a further 45 minutes. After this time, t.l.c. analysis indicated only a trace amount of remaining starting material. The reaction was quenched by addition of saturated NH₄Cl solution and the tetrahydrofuran layer was decanted. The aqueous layer was extracted with isopropylacetate, and the resulting emulsion was broken by slow addition of 5N HCl solution. The organic layer was separated, combined with the tetrahydrofuran layer and washed with sat. NH₄Cl solution, and then with brine. The pH of the brine layer was 6.5 to 7 at this point and the organic layer was dried with Na₂SO₄, filtered and concentrated *in vacuo* to yield a foamy solid. The crude residue was co-evaporated with toluene, concentrated *in vacuo* and the resulting residue was taken into toluene by heating to about 45°C. The mixture was cooled to room temp. and stirred at this temperature until a white solid began to precipitate. Water was added dropwise, and the resulting mixture stirred at room temperature for about 3 hours. The solid was isolated by filtration and the filter cake washed with water and toluene. The solid was dried at about 45°C under high vacuum for about 1 hour, and then at room temperature under vacuum overnight to yield 2'-deoxy-5'-*O*-trityl-□-D-thymidine **3** in an approximate 41 % yield;
(c) preparing 2'-deoxy-D-thymidine **(4)** from 2'-deoxy-5'-*O*-trityl-□-D-thymidine **(3)** 2'-deoxy-5'-*O*-trityl-□-D-thymidine **3** (1.215 g, 2.5 mmol) by suspending **(3)** in methanol and heating the reaction mixture to about 45°C in a water bath until **(3)** dissolved. The flask was then cooled to room temperature and concentrated. HCl was added to the mixture and stirred at room temperature. After about 25 minutes, a white solid began to precipitate from the solution. After 1 hour, t.l.c. analysis indicated no remaining starting material (R_{f} 0.53) and formation of major product (R_{f} 0.21). A portion of n-heptane was added to the reaction mixture and stirred at room temperature for about 15 minutes. The white solid was isolated by filtration. The filtrate was split into two layers and the methanol layer was extracted with n-heptane, and then concentrated *in vacuo* to a volume of 2 mL. The residue was combined with the 405 mg of white solid, suspended in TBME, and stirred at room temp for 1 hour. The white solid was isolated by filtration, washed with TBME, and dried under vacuum in an oven to yield 2'-deoxy-D-thymidine **(4)** in approximately_78% yield.

It is to be understood that all synthetic routes described in **Figures 1-29** and all Examples are equally applicable to any stereochemical form, α- or β-, D- or L-, of any starting material, and that starting material compounds are not limited to ribose, xylose, and arabinose as provided herein, but also include 5- and 6-membered rings having S, N, or CH₂ in place of the O shown in the non-limiting examples and in **Figures 1-29****.**

The present invention is best described in the following non-limiting series of examples. Equivalent, similar, or suitable solvents, reagents, and/or reaction conditions may be substituted for those particular solvents, reagents, and/or reaction conditions described herein without departing from the spirit and scope of the invention.

### EXAMPLES

### Example 1

L-arabinose is converted to the corresponding methyl glycoside and the 3- and 4-hydroxyl groups are protected as the acetonide derivative. The scheme below shows a simple approach to deoxygenate the 2-hydroxy group of compound **2** by converting it to the corresponding mesylate group and subjecting this mesylate intermediate to reductive cleavage conditions to produce the 2-deoxy intermediate **4.** See H. Urata, E. Ogura, K. Shinohara, Y. Ueda, and M. Akagi, Nucleic Acids Res. 1992, 20, 3325-3332; and J. W. Pratt, N. K. Richtmyer, and C. S. Hudson, J. Am. Chem. Soc. 1952, 74, 2200-2205.

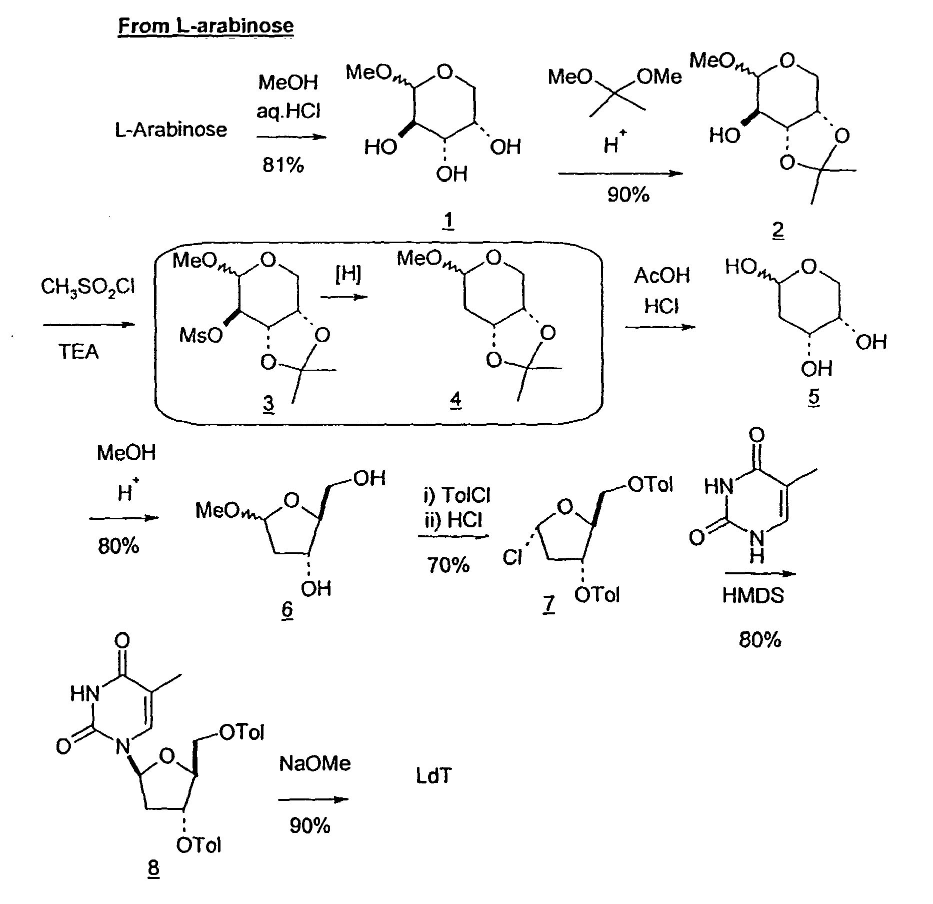

### Example 2

L-arabinose is converted to the corresponding glycal derivative via a key reductive elimination step and converting the resulting glycal intermediate to methyl 2-deoxy ribofuranoside. See B. K. Shull, Z. Wu, and M. Koreeda, J. Carbohydr. Chem. 1996, 15, 955-964; M. L. Sznaidman, M. R. Almond, and A. Pesyan. Nucleosides, Nucleotides & Nucleic Acids 2002, 21, 155-163; and Z.-X. Wang, W. Duan, L. I. Wiebe, J. Balzarini, E. D. Clercq, and E. E. Knaus, Nucleosides, Nucleotides & Nucleic Acids 2001, 20, 11-40.

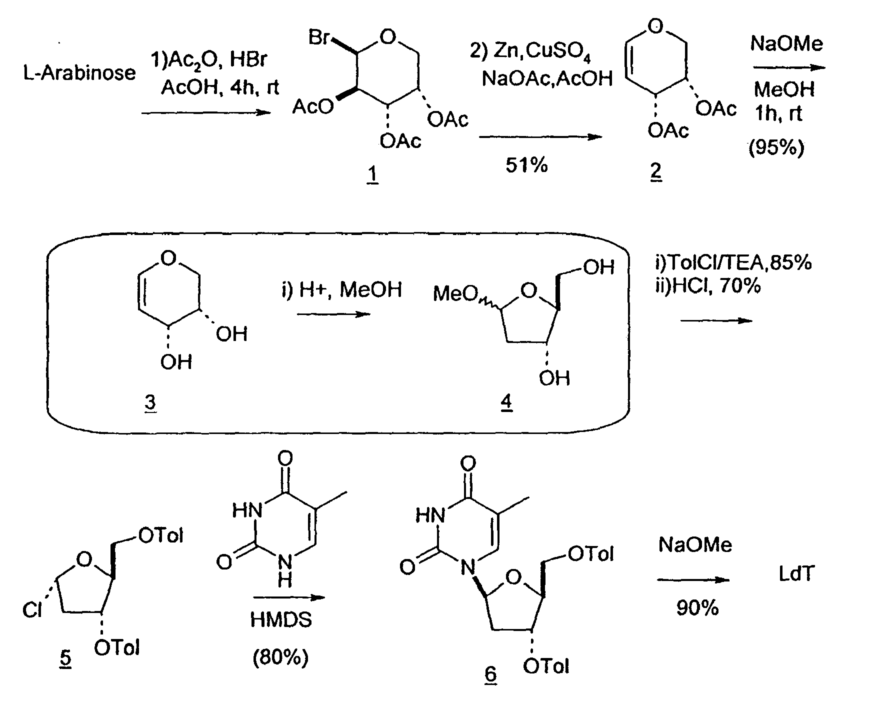

### Example 3

L-arabinose is converted to the corresponding glycal derivative via a key reductive elimination step and converting the resulting glycal intermediate to methyl 2-deoxy ribofuranoside. See M. L. Sznaidman, M. R. Almond, and A. Pesyan. Nucleosides, Nucleotides & Nucleic Acids 2002, 21, 155-163; Z.-X. Wang, W. Duan, L. I. Wiebe, J. Balzarini, E. D. Clercq, and E. E. Knaus, Nucleosides, Nucleotides & Nucleic Acids 2001, 20, 11 ― 40; and R. V. Stick, K. A. Stubbs, D. M. G. Tilbrook, and A. G. Watts, Aust. J. Chem. 2002, 55, 83-85.

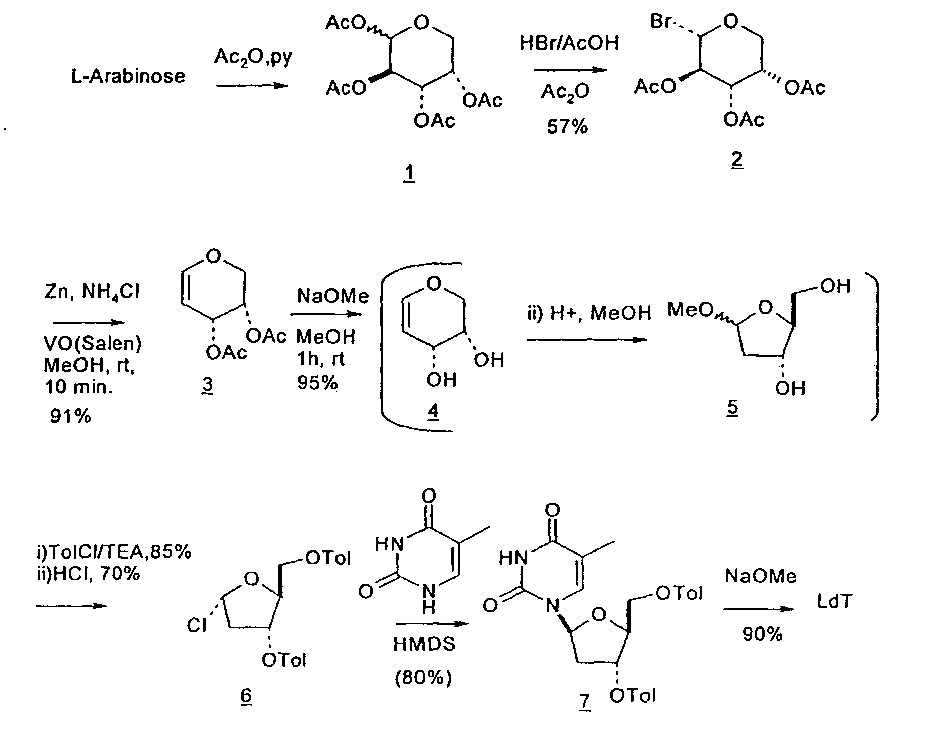

### Example 4

D-Xylose is oxidized with bromine/water and then the resulting 1,4-lactone is subjected to HBr/acetic acid to obtain 2,5-dibromo-2,5-dideoxy-D-lyxono-1,4-lactone **2.** Treatment of the dibromolactone **2** with potassium iodide in TFA gives the corresponding 5-iodo compound and also results in selective removal of the bromine atom at C-2 to give the 5-iodo-2-deoxylactone **3.** Subjecting this 5-iodo-lactone **3** to aqueous potassium hydroxide gives the 4,5-epoxide derivative, which on treatment with aqueous acid gives the corresponding 2-deoxy L-ribonolactone via a stereospecific inversion at C-4. The protected 2-deoxy L-ribonolactone **6** is selectively reduced to the corresponding lactol **7** using Red-Al. Lactol **7** is then converted to the desired chlorosugar **9.** See H. S. Isbell, Methods in Carbohydrate Research 1963, 2, 13-14; K. Bock, I. Lundt, and C. Pedersen, Carbohydrate Research 1981, 90, 17-26; K. Bock, I. Lundt, and C. Pedersen, Carbohydrate Research 1982, 104, 79-85; and I. Lundt, and R Madsen, Topics in Current Chemistry 2001, 215, 177-191.

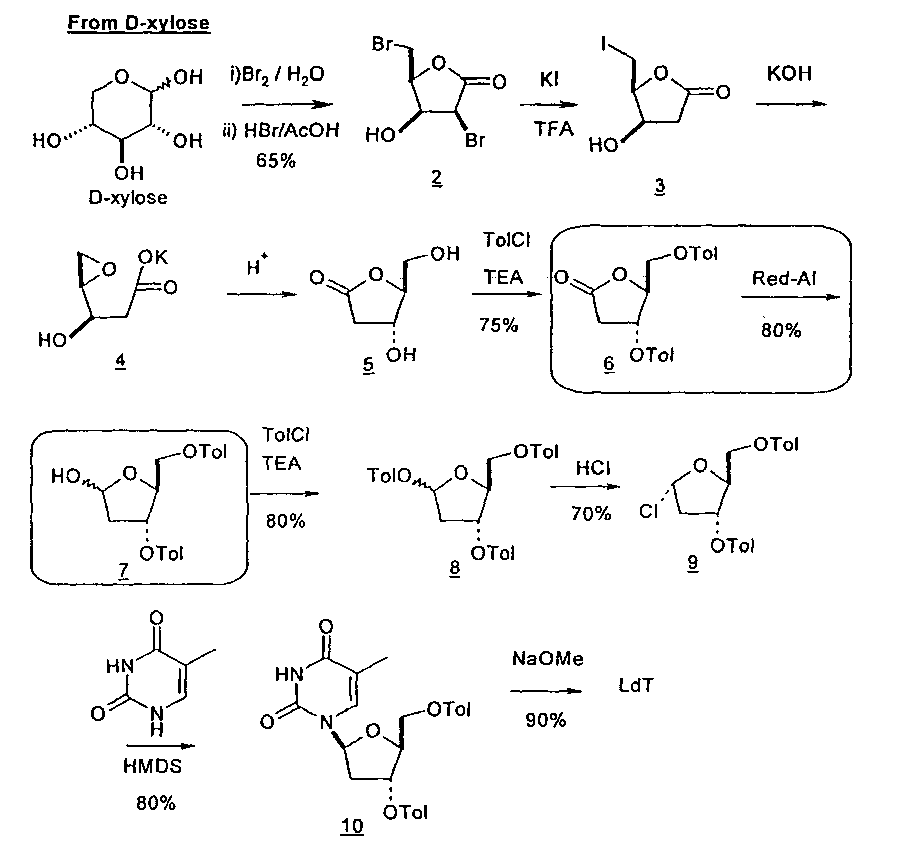

### Example 5

D-galactose is oxidatively cleaved to give a D-lyxonolactone, which is then brominated to produce the 2,5-dibromo-2,5-dideoxy-D-xylono-1,4-lactone **2**. Selective hydrogenolysis of **2** gives 5-bromo-2-deoxylactone **3** which is then subjected to a sequence of transformations, similar to that shown in **Example 7,** to produce the key chlorosugar intermediate **8**. See K. Bock, I. Lundt, and C. Pedersen, Carbohydrate Research 1981, 90, 17-26; K. Bock, I. Lundt, and C. Pedersen, Carbohydrate Research 1979, 68, 313-319; K. Bock, I. Lundt, and C. Pedersen, Acta Chem. Scand. B 1984, 38, 555-561; and W. J. Humphlett, Carbohydrate Research 1967, 4, 157-164.

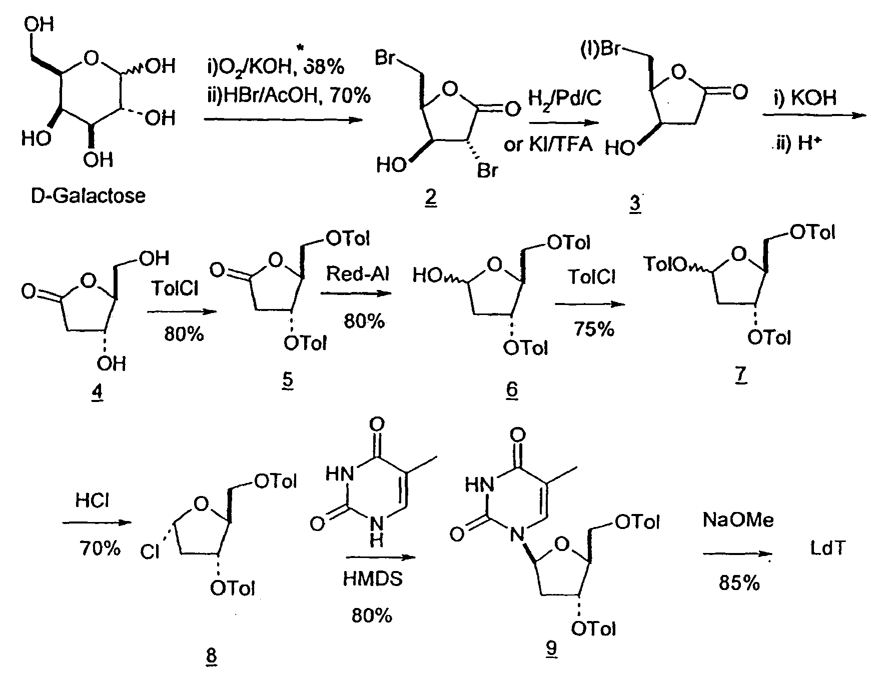

### Example 6

D-glucono-1,4-lactone is converted to 2,6-dibromo-2,6-dideoxy-D-mannono-1,4-lactone **1.** Treatment of lactone **1** with hydrazine followed by aqueous bromine gives 6-bromo-2,6-dideoxy-D-arabino-hexono-1,4-lactone **3**. Reaction of **3** with excess aqueous potassium hydroxide followed by acidification leads to inversion at C-4 and C-5 giving 2-deoxy-L-ribo-hexono-1,4-lactone **6**. This transformation involves ring opening of the lactone via a Payne rearrangement of the primary epoxide **4** to the secondary epoxide **5.** 2-Deoxy-L-ribo-hexono-1,4-lactone **6** is subjected to oxidative cleavage followed by reducing the resulting aldehyde to produce lactone 7 which is converted to the desired chlorosugar using a reaction sequence similar to that shown in **Example 5**. See K. Bock, I. Lundt, and C. Pedersen, Carbohydrate Research 1979, 68, 313-319; and K. Bock, I. Lundt, and C. Pedersen, Acta Chem. Scand. B 1984, 38, 555-561.

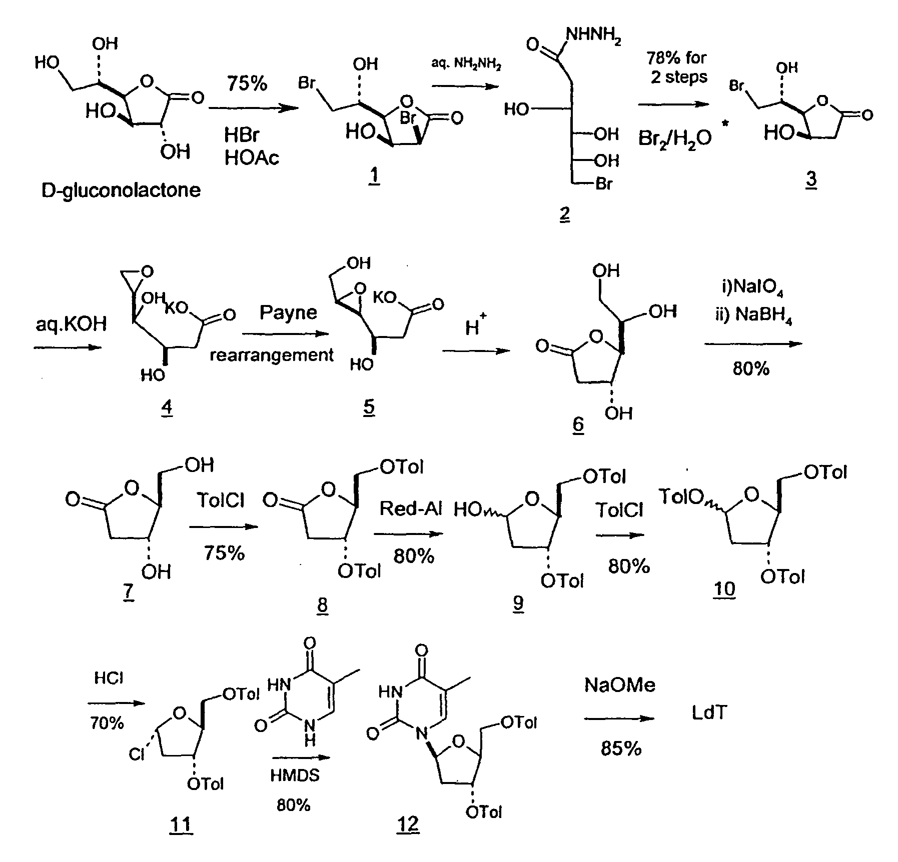

### Example 7

D-Galactono-1,4-lactone is converted to the acetylated dibromolactone **2**, which on treatment with hydrazine followed by bromination, gives 2-deoxylactone **3.** Lactone **3** is de-acetylated and subjected to oxidative cleavage followed by reducing the resulting aldehyde with NaBH₄ to produce the 2-deoxy-L-ribono-1,4-lactone **5**. See K. Bock, I. Lundt, and C. Pedersen, Carbohydrate Research 1979, 68, 313-319; and K. Bock, I. Lundt, and C. Pedersen, Acta Chem. Scand B 1984, 38, 555-561.

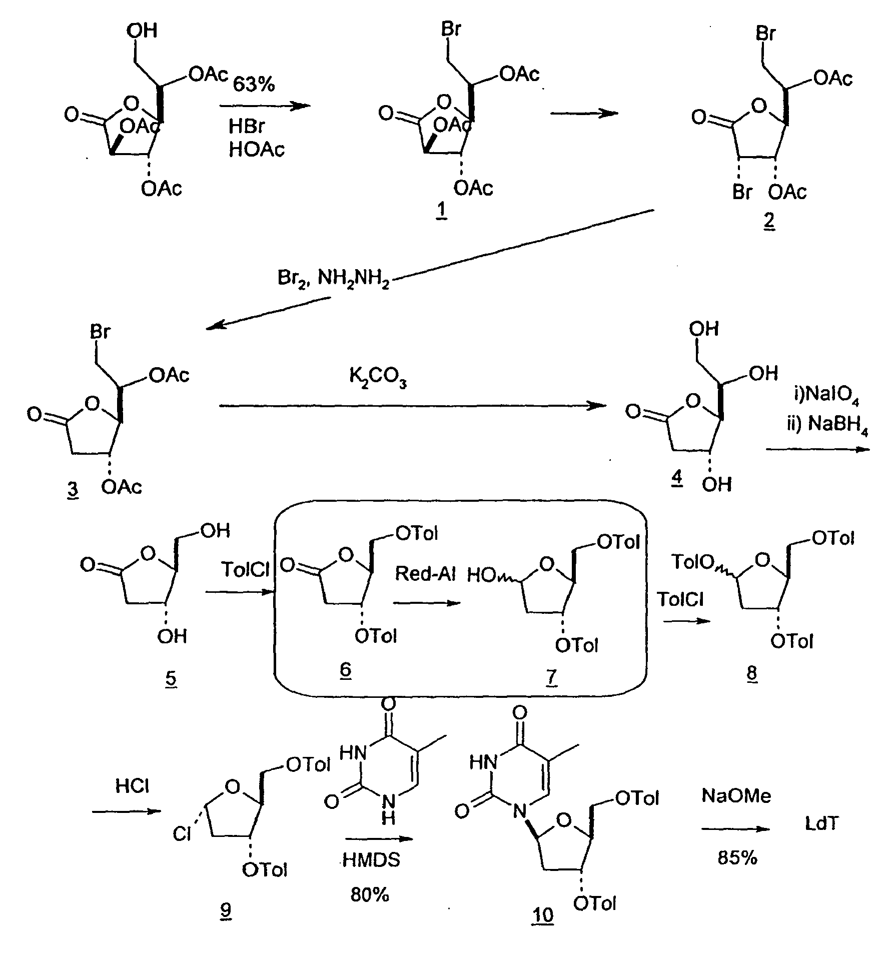

### Example 8

A furanolactone, a commercially available, non-carbohydrate and achiral, is used as starting material to obtain a chlorosugar. The key step in this approach is the asymmetric dihydroxylation of the 2Z-pentenoate ester **2** to form the 2(R),3(R)-pentanoate derivative **3**. Intermediate **3** is subjected to stereoselective cyclization to give the 2-deoxy L-sugar **4**. Compound **4** is converted to the desired protected chlorosugar in three straightforward synthetic transformations. See D.C. Liotta, and M.W. Hager, *U.S. Patent 5,414,078*, May 9,1995.

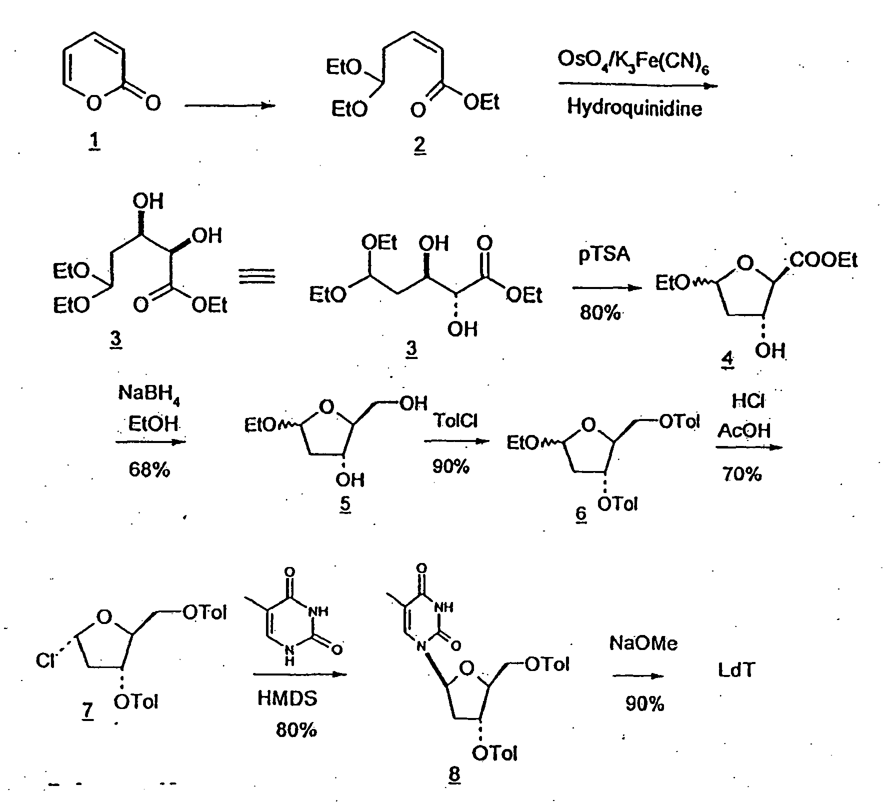

### Example 9

Ethyl-3,3-diethoxypropanoate **1** is an inexpensive, non-carbohydrate, acyclic and achiral starting material. Compound **1** is reduced to the corresponding aldehyde **2** using DIBAL. In the next step aldehyde **2** is converted to the QO-unsaturated ester, 5,5-diethoxy-2E-pentenoate via the Horner-Emmons modification of the Wittig reaction, using diisopropyl(ethoxycarbonyl) methylphosphonate and the resulting ester is reduced to the 2E-penten-1-ol derivative **3**. This prochiral allylic alcohol **3** is converted to the corresponding 2(S),3(S)-epoxy alcohol **4** using Sharpless asymmetric epoxidation conditions. The resulting epoxy alcohol **4** is protected and then subjected to acid hydrolysis to produce the key intermediate **6**. Compound **6** is cyclized to the 2-deoxy L-ribofuranose derivative **7**, which is then converted to the desired L-chlorosugar **9**. See . D. C. Liotta, and M, W. Hager, U.S. Patent 5, 414, 078, May 9, 1995; and M. W. Hager, and D. C. Liotta, Tetrahedron Lett.1992, 33, 7083-7086.

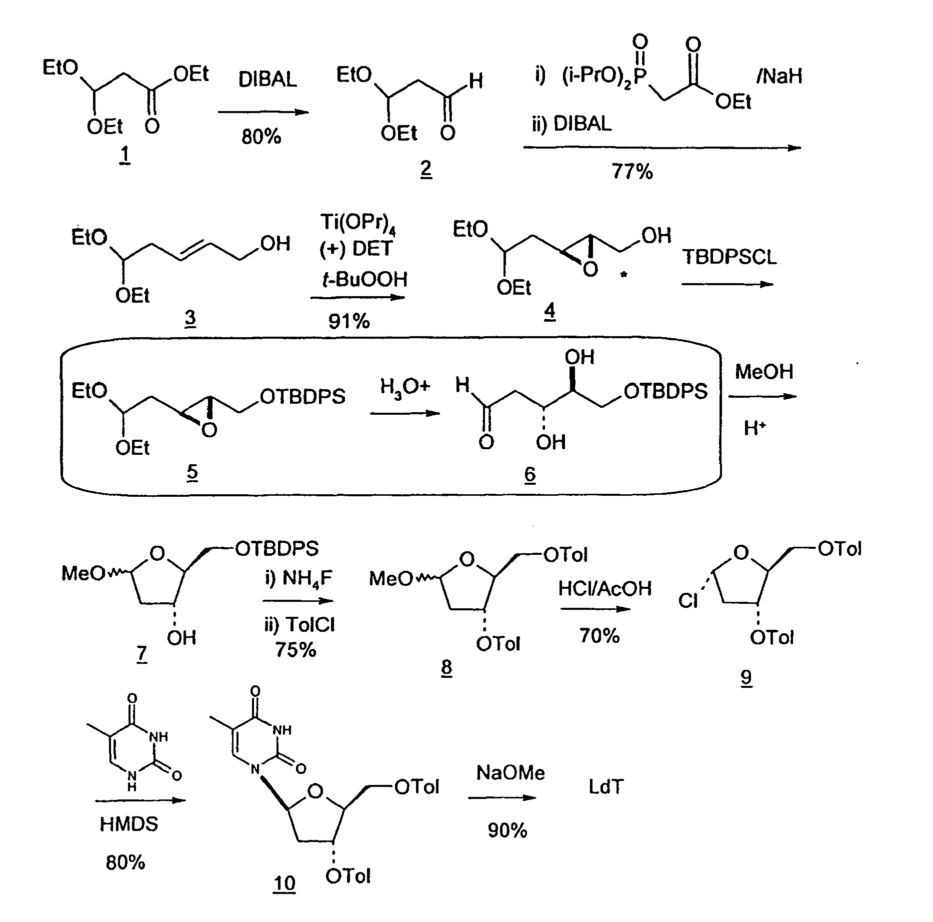

### Example 10

Hydroxy glutamic acid **1** is cyclized to give 2-deoxy L-1,4-ribonolactone derivative **2**, which is then-converted to the desired chlorosugar **5** in four steps. See R. F. Schinazi, D. C. Liotta, C. K. Chu, J. J. McAtee, J. Shi, Y. Choi, K. Lee, and J. H. Hong, U. S. Patent, 6,348,587B1, Feb. 19, 2002**;** U. Ravid, R. M. Silverstein, and L. R. Smith, Tetrahedron 1978, 34, 1449-1452; and M. Taniguchi, K. Koga, and S. Yamada, Tetrahedron 1974, 30, 3547-3552.

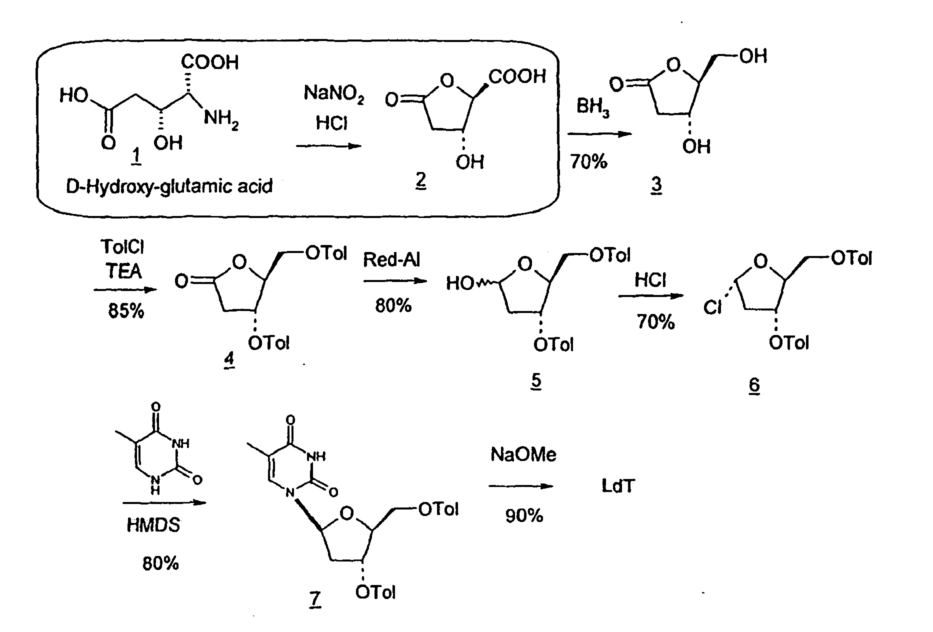

### Example 11

Commercially available alcohol **1** is subject to Sharpless epoxidation conditions to produce epoxide **2**. The epoxy alcohol **2** is treated with benzyl alcohol in presence of Ti(Oi-Pr)₄ to give the diol **3,** which is converted to the corresponding acetonide derivative **4.** Compound **4** is oxidized using the Wacker conditions to produce aldehyde **5,** which is treated with aqueous hydrochloric acid to give 5-O-benzyl-2-deoxy-L-ribofuranose **6.** Compound **6** is converted to the desired chlorosugar **9** in four simple steps. See M. E. Jung, and C. J. Nichols, Tetrahedron Lett. 1998, 39, 4615-4618.

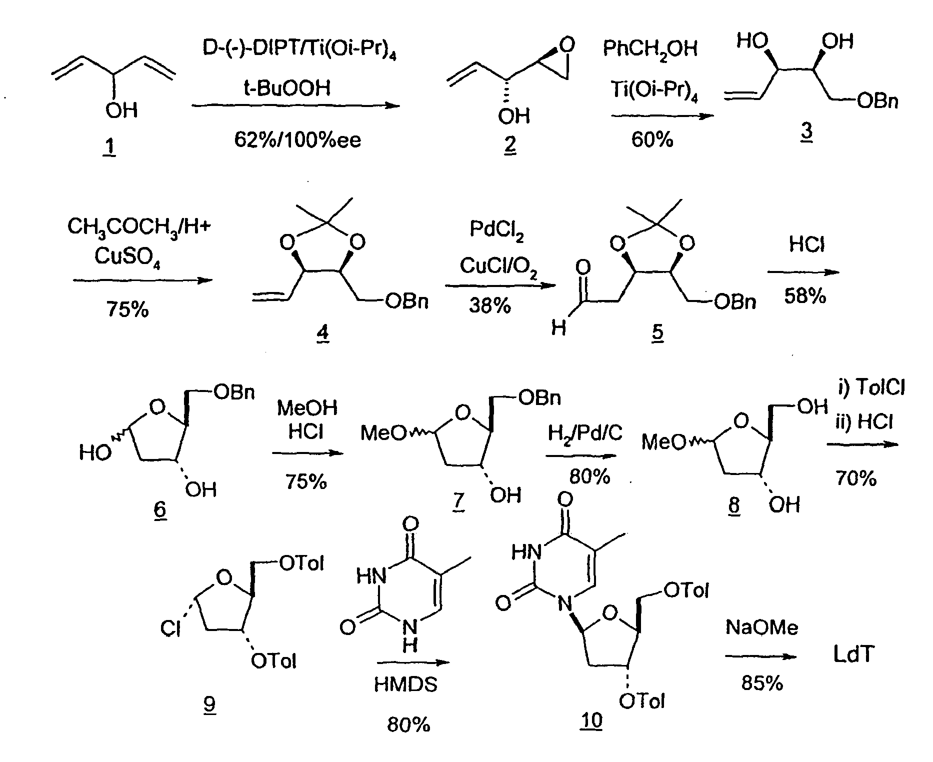

### Example 12

Epoxyalcohol **1** is protected as the benzyl ether **2** and opening the epoxide with sodium benzylate in benzyl alcohol, then protecting the resulting alcohol **3** to give the tris-benzyl ether **4**. The conversion of compound **4** to aldehyde **6** (which is a protected 2-deoxy-L-ribose) is carried out using hydroboration/H₂O₂ oxidation followed by Swern oxidation of the resulting alcohol **5**. Deprotection of the benzyl ethers of **6** using palladium hydroxide on carbon gives a mixture of the three ethyl 2-deoxy-L-ribosides **7a, 7b** and **7c** in a 2:2:1 ratio. Protecting **7a** and **7b** using toluoyl chloride and treating the resulting di-toluoyl derivative with hydrogen chloride gives the desired chlorosugar **8**. See M. E. Jung, and C. J. Nichols, Tetrahedron Lett. 1998, 39, 4615-4618.

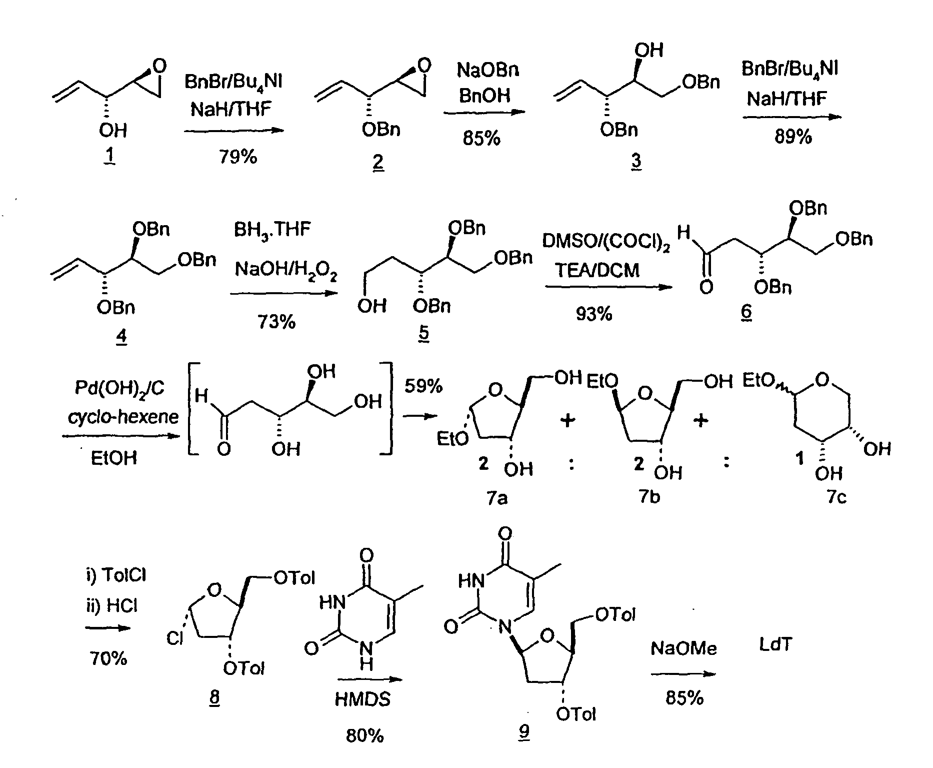

### Example 13

1,2-O-Isopropytidine-L-glyceraldehyde **1** is treated with allyl bromide in presence of zinc and aqueous ammonium chloride to give the corresponding homoallyl alcohol **2**. The isopropylidine group of **2** is removed using aqueous acetic acid to give intermediate **3.** Successive ozonolysis and reduction, by dimethylsulfide, of **3** affords 2-deoxy-L-ribofuranose **4** which is converted to the protected chlorosugar **7** in three steps. See J. S. Yadav, and C. Srinivas, Tetrahedron Lett. 2002, 43, 3837-3839; and T. Harada, and T. Mukaiyama, Chem. Lett. 1981, 1109-1110.

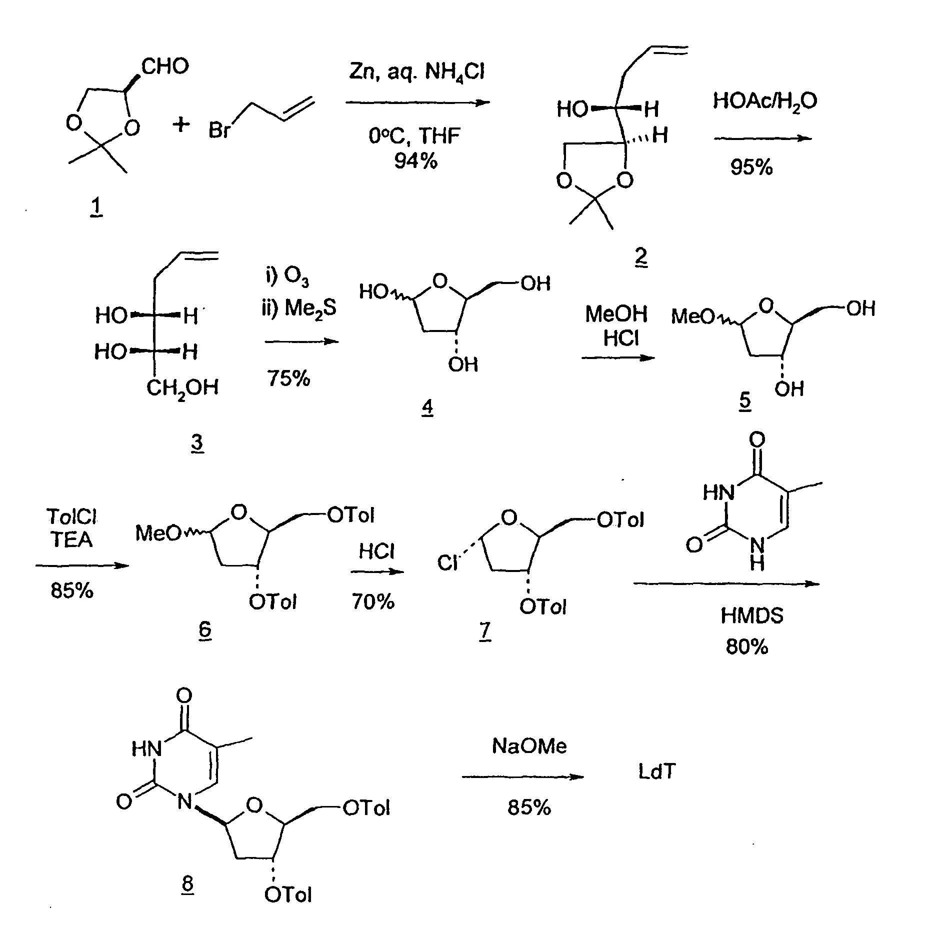

### Example 14

The present example utilizes 2-bromomethyl-[1,3]dioxolane instead of allyl bromide, as used in **Example 13,** which eliminates the need for ozonolysis and subsequent reduction of intermediate **3.** See J. S. Yadav, and C. Srinivas, Tetrahedron Lett. 2002, 43, 3837-3839; and T. Harada, and T. Mukaiyama, Chem. Lett. 1981, 1109-1110.

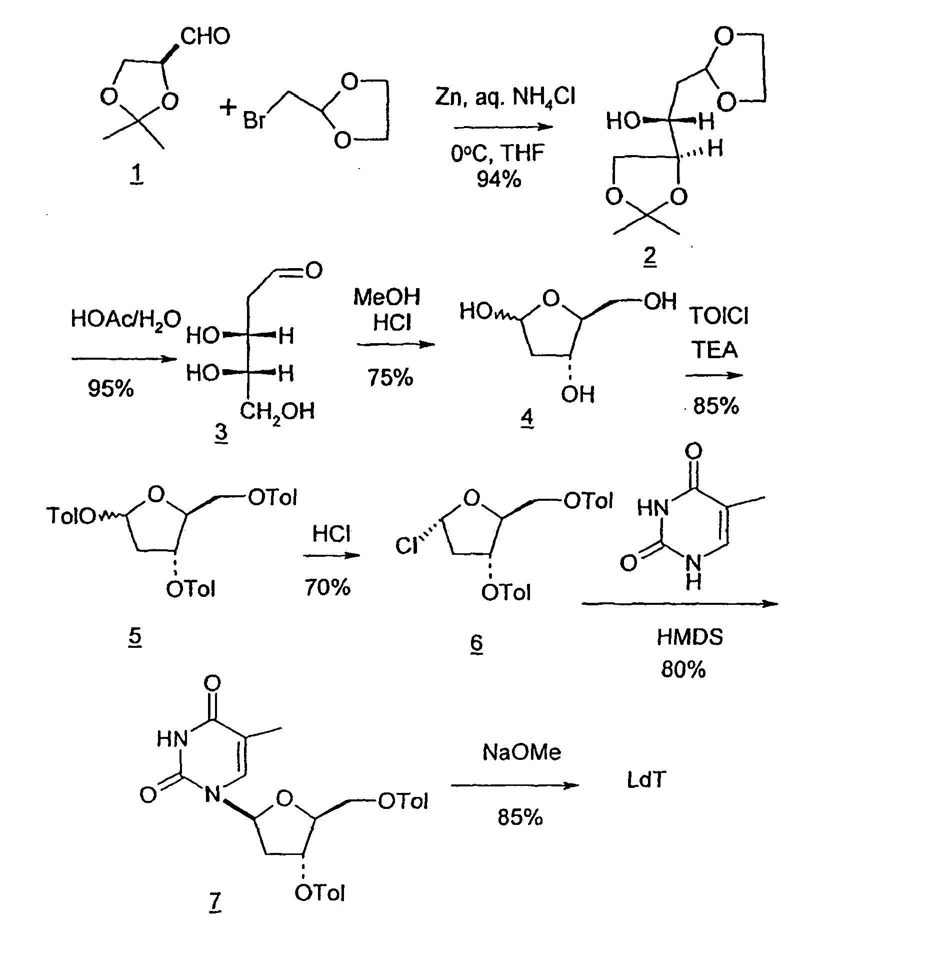

### Example 15

Glycal **1** (which can be prepared from L-ribose in four steps) is treated with acidic methanol to produce the 2-deoxy-L-ribose **3,** which is converted to the protected chlorosugar **5** in two steps. See H. Ohrui, and J. J. Fox, Tetrahedron Lett. 1973, 1951-1954; W. Abramski, and M. Chmielewski., J. Carbohydr. Chem. 1994, 13, 125-128; and J. C.-Y. Cheng, U. Hacksell, and G. D. Daves, Jr., J. Org. Chem. 1985, 50, 2778-2780.

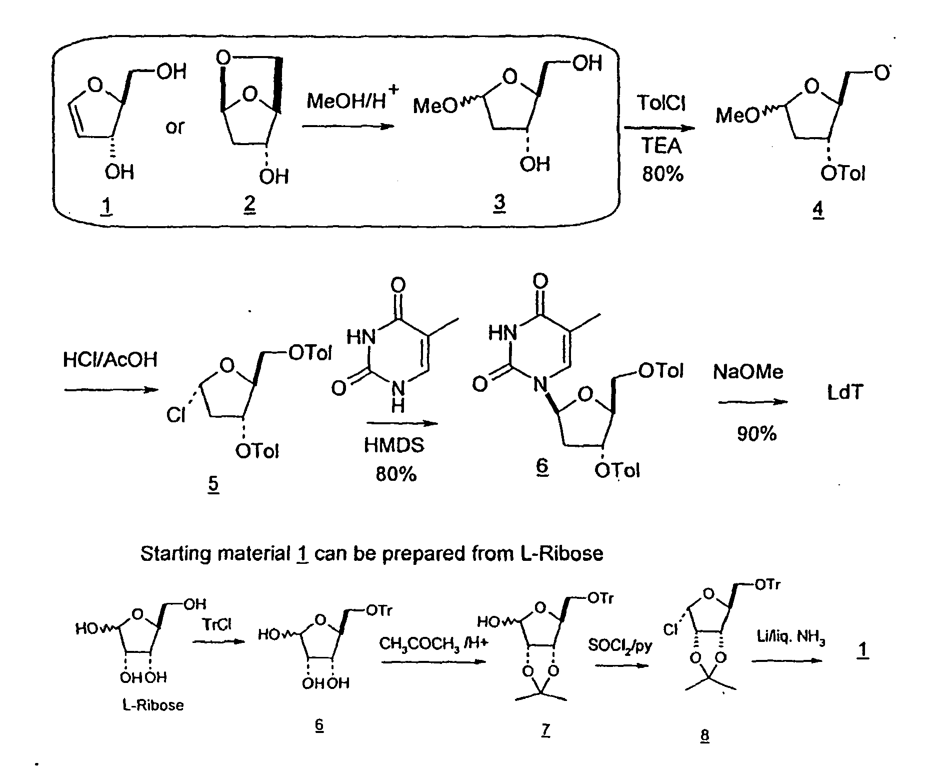

### Example 16

L-arabinose is reacted with cyanamide to give 1,2-oxazoline derivative **1.** When allowed to react with 2-methyl-3-oxo-propionic acid ethyl ester, compound **1** affords O^{2,2'}-anhydro-L-thymidine **2**. Compound **2** is benzoylated and the resulting di-O-benzoyl derivative **3** is subjected to reductive cleavage conditions to produce 3',5'-di-O-benzoyl LdT **4**. Compound **4** is treated with methanolic sodium methoxide to afford LdT. See A. Holy, Coll. Czech. Chem. Commun. 1972, 37, 4072-4087; and P. V. P. Pragnacharyulu, C. Vargeese, M. McGregor, and E. Abushanab, J. Org. Chem. 1995, 60, 3096-3099.

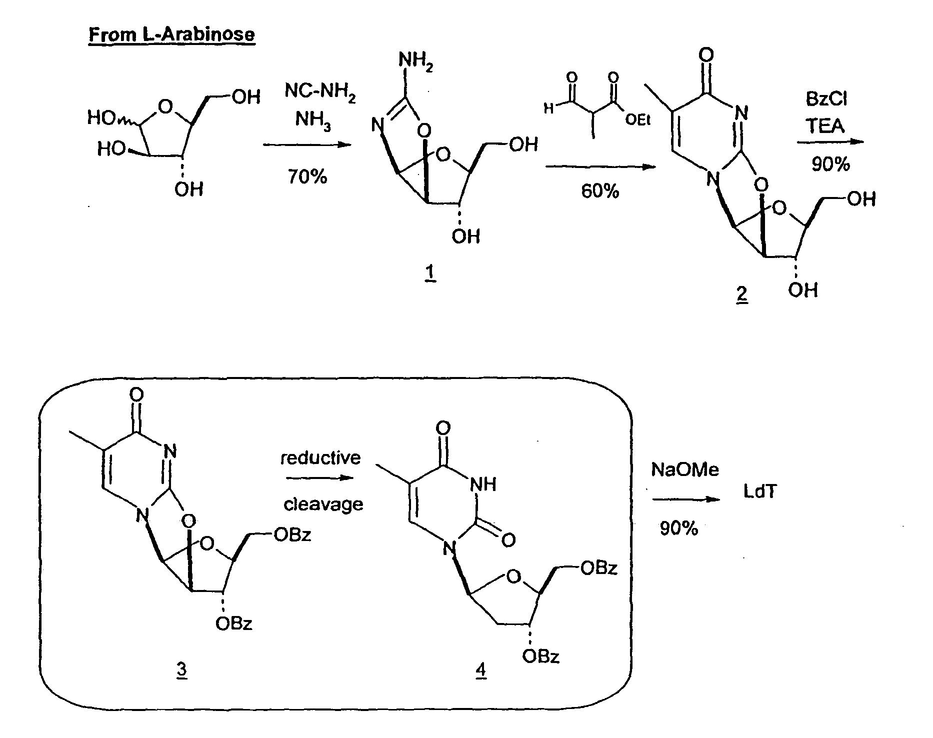

### Example 17

The present example employs a different method to open the O²,^{2'}-linkage of compound **3** by using hydrogen chloride. The resulting 2'-deoxy-2'-chloro derivative **4** is treated with TBTH/AIBN to obtain 2'-deoxy protected nucleoside **5**, which on deacylation gives LdT. See P. V. P. Pragnacharyulu, C. Vargeese, M. McGregor, and E. Abushanab, J. Org. Chem 1995, 60, 3096-3099.

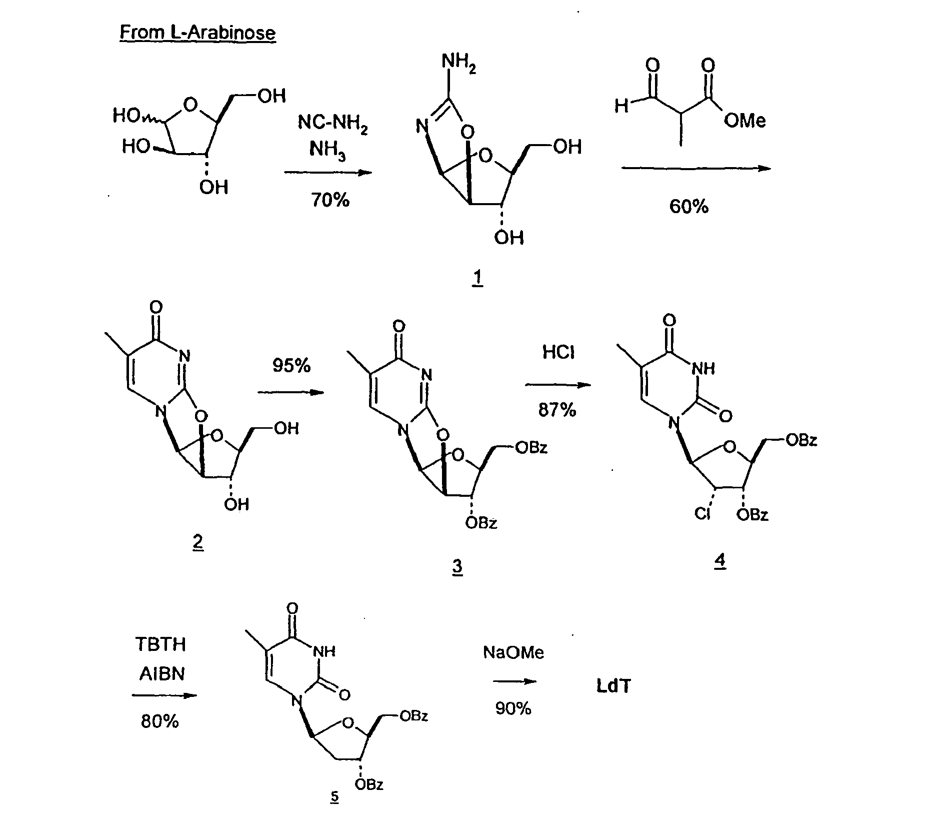

### Example 18

The present example differs from Example 17 in that 1,2-oxazoline derivative 1 is reacted with different compounds to obtain the O²,^{2'} -anhydro-L-thymidine **2.** See D. McGee, Boehringer Ingelheim Proposal to Novirio Pharmaceuticals, Inc., May 17, 2002**;** and C. W. Murtiashaw, *Eur. Patent,* 0,351,126 B1, January 18, 1995**.**

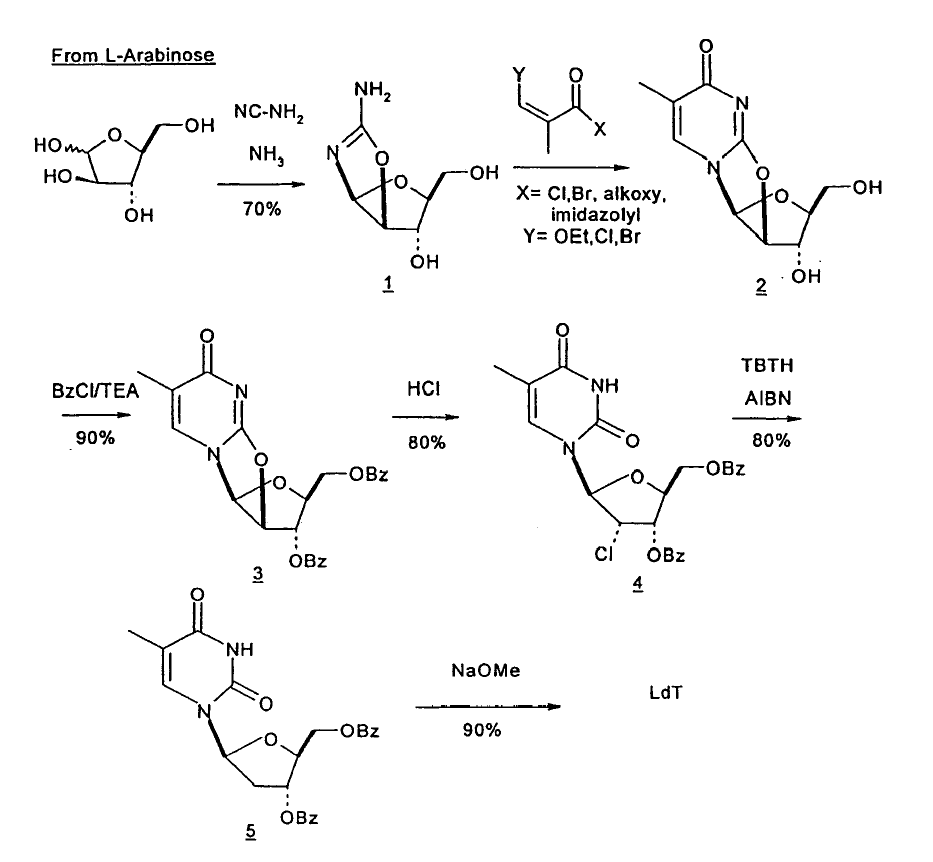

### Example 19

Hydrogen iodide is used to open the O^{2,2'}-linkage of compound **3** to obtain the 2'-deoxy-2'-iodo derivative **4.** Compound **4** is treated with potassium iodide to give the 3',5'-di-O-benzoyl-2'-deoxy-L-thymidine **5.** Compound **5** is subjected to methanolic solution of sodium methoxide to give LdT. See H. Sawai, A. Nakamura, H. Hayashi, and K. Shinozuka, Nucleosides & Nucleotides 1994, 13, 1647-1654; and H. Sawai, H. Hayashi, and S. Sekiguchi, Chemistry Lett. 1994, 605-606.

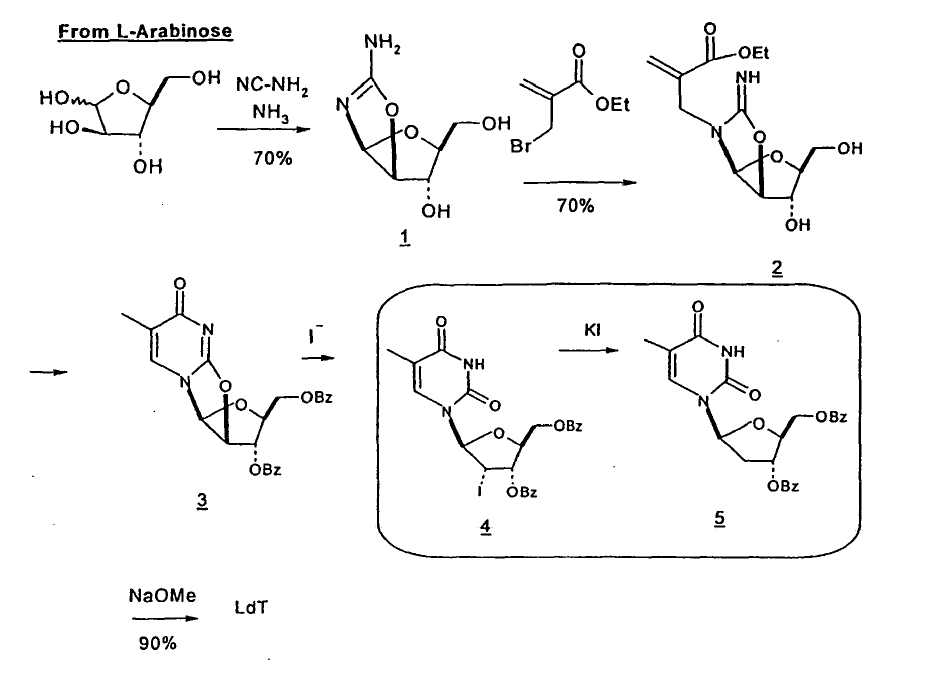

### Example 20

2-Methyl-oxirane-2-carboxylic ester is reacted with 1,2-oxazoline **1** to produce O^{2,2'}-anhydro-L-thymidine derivative **2.** Compound **2** is treated with pivaloyl chloride to protect the 3'- and 5'-hydroxyl groups and also cleave the O^{2,2'}-linkage to give the 2'-deoxy-2'-chloro nucleoside **3.** Compound **3** is treated with thionyl chloride to eliminate the hydroxyl group of the base moiety and the resulting compound is reduced using TBTH/AIBN to remove the 2'-chloro and produce the protected LdT **5**. Compound **5** is treated with sodium methoxide in methanol to afford LdT. See E. Abushanab, and P. V. P Pragnacharyula, US. Patent 5,760,208, June 2, 1998**.**

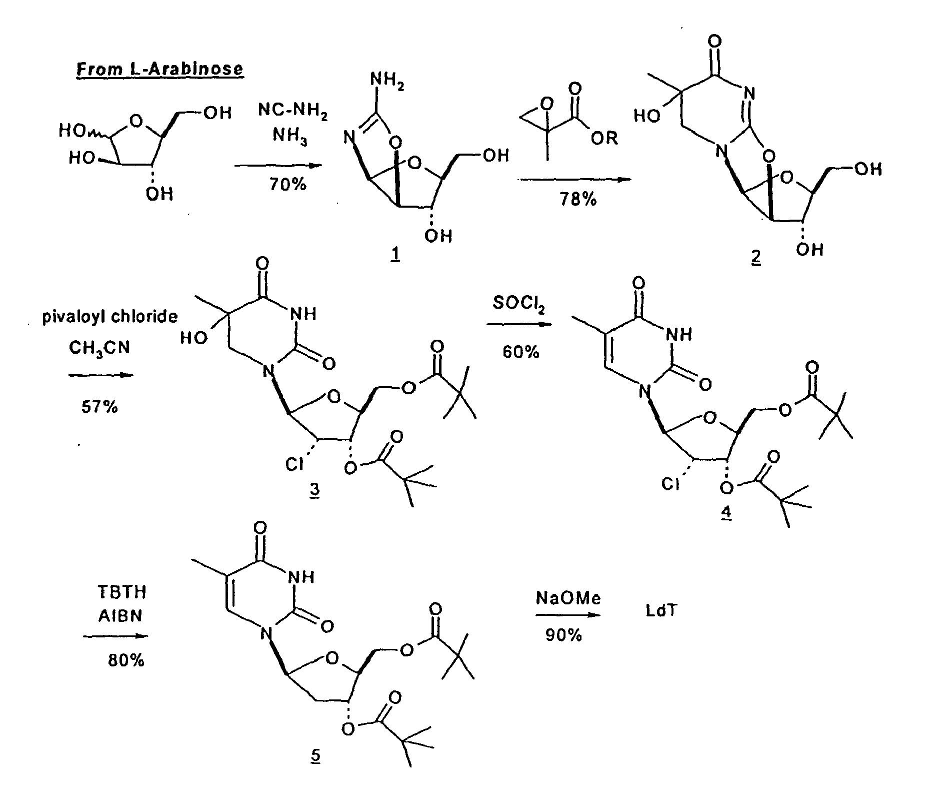

### Example 21

Ethyl propiolate is reacted with 1,2-oxazoline **1** to give O^{2,2'}-anhydro-L-uridine **2.** Compound **2** is protected and the resulting 3',5'-di-O-benzoyl derivative **3** is treated with hydrogen chloride to give the 2'-deoxy-2'-chloro nucleoside **4**. Compound **4** is then treated with TBTH/AIBN to remove the 2'-chloro and the 2'-deoxy derivative is then subjected to iodination conditions to give the 5-iodo nucleoside derivative **6.** The 5-iodo group of compound **6** is replaced by a methyl group using AlMe₃ and (Ph₃P)₄Pd to give 3',5'-di-O-benzoyl LdT **7,** which on treatment with sodium methoxide in methanol affords LdT. See A. Holy, Coll. Czech. Chem. Commun. 1972, 37, 4072-4087; J.-I. Asakura, and M. J. Robins, J. Org. Chem. 1990, 55, 4928-4933; J.-I. Asakura, and M. J. Robins, Tetrahedron Lett. 1988, 29, 2855-2858; and K. Hirota, Y. Kitade, Y. Kanbe, Y. Isobe, and Y. Maki, Synthesis, 1993, 210, 213-215.

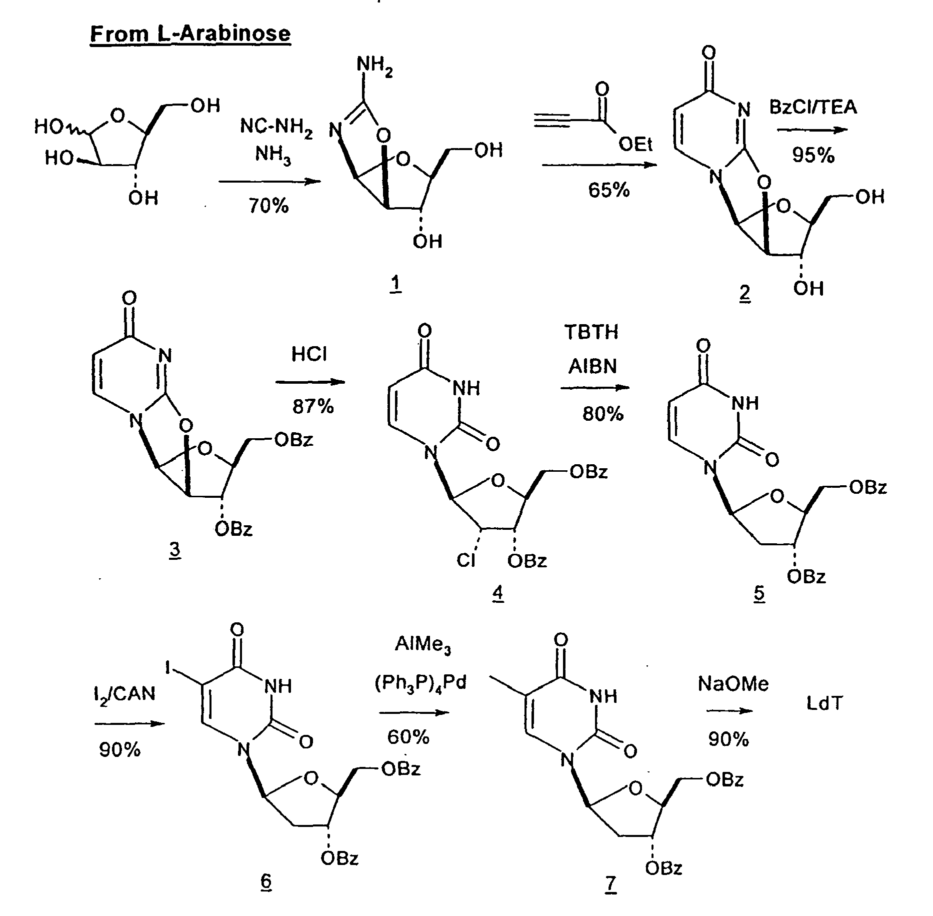

### Example 22

The present example is different from **Example 21,** only in the way of introducing a methyl group to the 5 position of the 2'-deoxy-L-uridine derivative **5.** Compound **5** is treated with formaldehyde in an alkaline medium to give 5-hydroxymethyl derivative **6,** which on subjection to acidic ethanol produces the 2'-deoxy-5-ethoxymethyl-L-uridine **7**. Compound **7** gives LdT under catalytic hydrogenation conditions. See A. Holy, Coll. Czech. Chem. Commun. 1972, 37, 4072-4087.

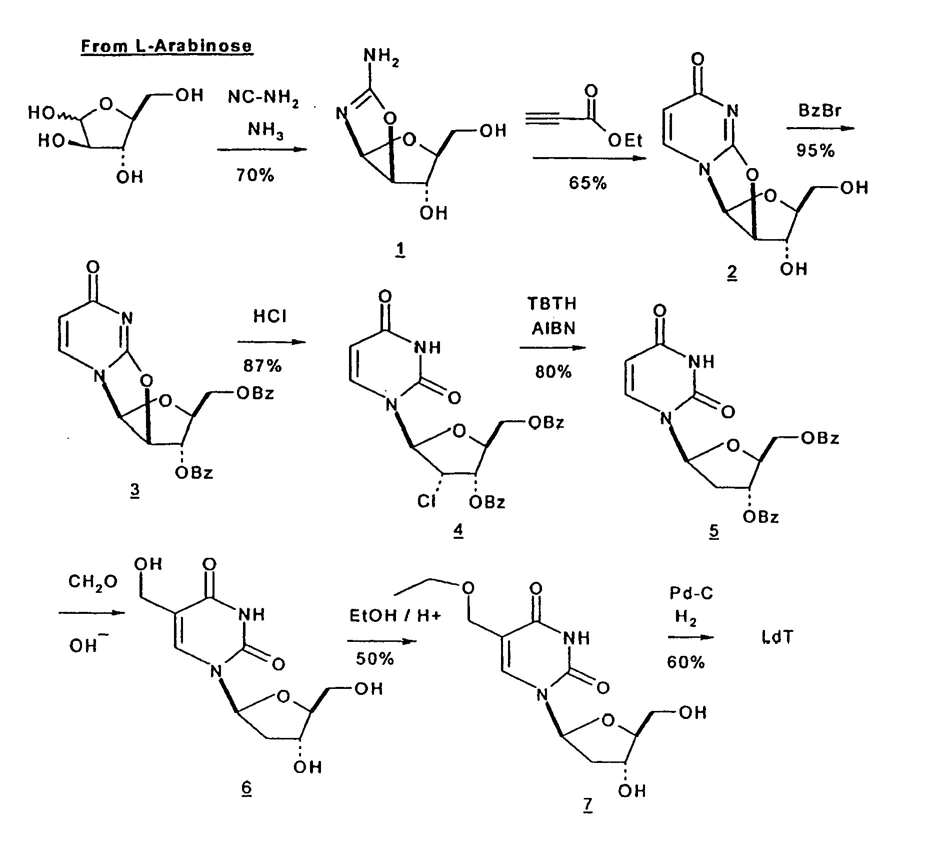

### Example 23

### Synthesis of the key intermediate 2-deoxy-3,5-di-O-para-toluoyl-□-L-erythro-pentofuranosyl chloride from D-xylose

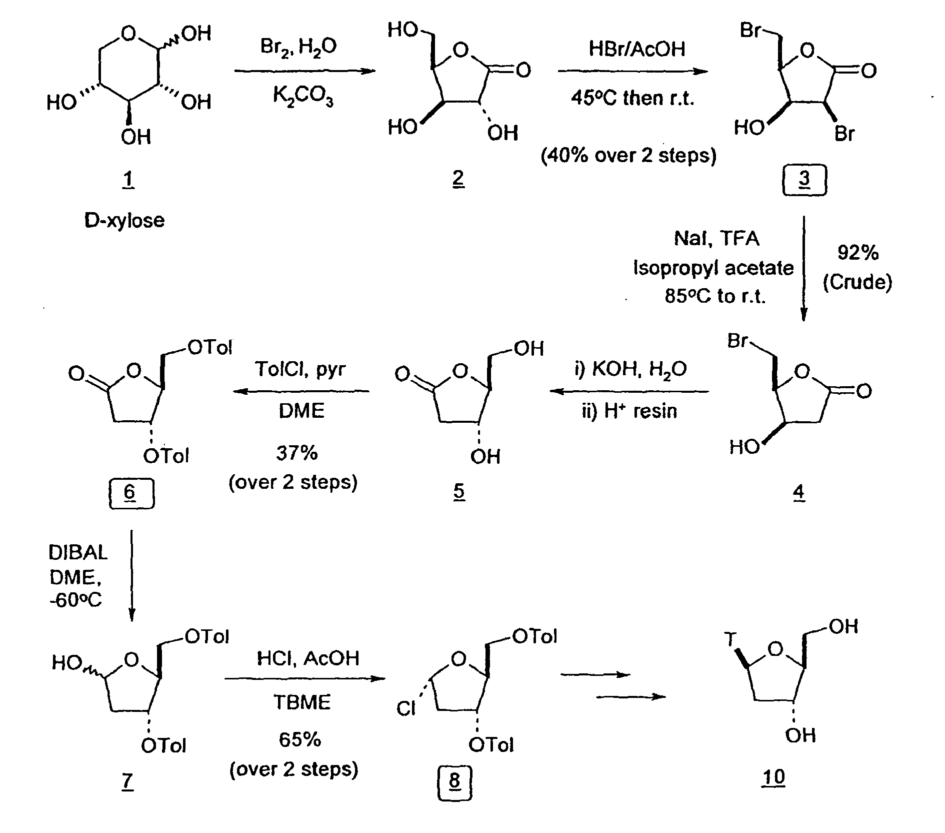

### Example 23(a): D-Xylono-1,4-lactone (2) from D-xylose (1) via bromine oxidation

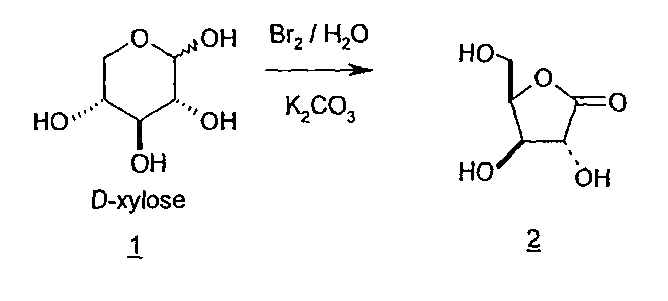

D-Xylose **1** (100 g, 666.1 mmol) was dissolved in distilled water (270 mL) and cooled to 0°C under overhead stirring in a lL three-necked round-bottomed flask. Potassium carbonate (113.2g, 819.3 mmol) was added portion-wise maintaining the temperature below 20°C. Bromine (39.4 mL, 766.0 mmol) was then added drop-wise at 0 to 5°C over a period of 2 hours whilst maintaining the temperature below 10°C. The reaction mixture was maintained at about 5-10°C for a further 30 minutes and then warmed to room temperature and stirred overnight. After about 8 hours, t.l.c. analysis (10% methanol in ethyl acetate, visualized using vanillin) indicated no starting material (R_{f} 0.0) and a new product (R_{f} 0.3). The reaction mixture was stirred with formic acid (6.6 mL) for about 15 minutes and then concentrated *in vacuum* at 45°C to a volume of approximately 50 mL. Co-evaporation with acetic acid (200 mL) and concentration *in vacuum* at about 45°C to a volume of 60 mL was performed and the crude D-xylono-1,4-lactone 2 transferred to be used as is in the next step.

Advantages of this synthetic step include switching from BaCO₃ known in the prior art to K₂CO₃ provided superior loading ratio (50 g of D-xylose **1** in 135 mL of water compared to 400 mL for BaCO₃); lactone can be used without further purification/removal of KBr salt in the next step; residual KBr can be used for the next reaction; and co-evaporation with acetic anhydride to remove residual water leads to formation of less polar products by t.l.c. analysis.

### Example 23(b): 2,5-Dibromo-2,5-dideoxy-D-lyxo-1,4-lactone (3) from D-xylono-1,4-lactone (2)

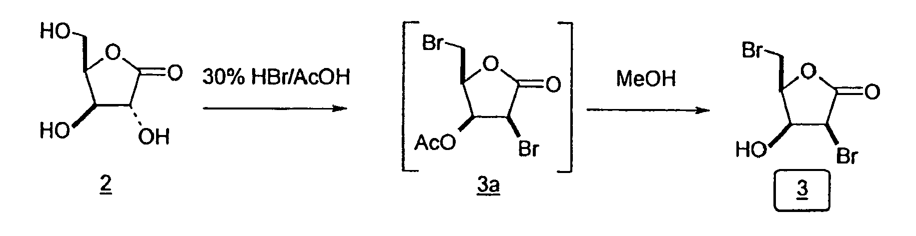

D-Xylono-1,4-lactone **(2)** (crude in acetic acid, 666.08 mmol) was transferred into a 3L flask using acetic acid (200 mL) whilst warm and 30% HBr-AcOH (662 mL, 3330 mmol) was added slowly to the stirred suspension. The reaction mixture was heated to 45°C for 1 hour and then cooled and stirred for 1.5 hours at room temperature. After this time, t.l.c. analysis (1:1, ethyl acetate:hexane) indicated two major products (R_{f} 0.63 [3-*O*-acetyl-2,5-dibromo-2,5-dideoxy-D-lyxo-1,4-lactone 3a] and R_{f} 0.5 [2,5-dibromo-2,5-dideoxy-D-lyxo-1,4-lactone 3]) and some remaining starting material (t.l.c. analysis, 10% methanol in ethyl acetate, R_{f} 0.0, visualized using vanillin). The reaction was cooled to 0°C and methanol (850 mL) was added over 1 hour whilst maintaining the temperature below 20°C. The reaction mixture was then allowed to warm to room temperature and stirred overnight. After this time, t.l.c. analysis (1:1, ethyl acetate:hexane) indicated conversion of one product (R_{f} 0.63) to the other product (R_{f} 0.44). The reaction mixture was filtered through a Buchner funnel to remove the residual KBr (173.89g) and then concentrated *in vacuum* and co-evaporated with water (2 x 250 mL). Ethyl acetate (800 mL) and water (250 mL) were added and the layers separated. The aqueous layer was further extracted with ethyl acetate (2 x 300 mL) and the combined organic extracts were washed with aqueous saturated sodium hydrogen carbonate solution (400 mL) and water (100 mL). The layers were separated, the aqueous layer extracted with ethyl acetate (2 x 300 mL) and the combined organic extracts dried with sodium sulfate (125 g), filtered and concentrated *in vacuum* at 50°C. Before concentrating to dryness, the organic extract was coevaporated with heptane (200 mL) to give a brown semi-solid. Trituration of this solid was carried out using 20% heptane in isopropyl ether (100 mL heptane and 500 mL of isopropyl ether) and dried in vacuum at 30-35°C overnight to yield 2,5-dibiomo-2,5-dideoxy-D-lyxo-1,4-lactone 3 as a pure light brown solid (71.9 g, 40% over 2 steps).
M.p. 92-94°C [Lit. 92-93°C]; δ_{H} (d⁶-DMSO, 400 MHz): 3.65 (1H, dd, *J*_{4,5'} 8.1 Hz, *J*_{5,5'} 10.7 Hz, H-5'), 3.73 (1H, dd, *J*_{4,5} 5.9 Hz, *J*_{5,5'} 10.7 Hz), 4.4 (1H, m, H-3 or H-4), 4.73 (1H, m, H-4 or H-3), 5.31 (1H, d, *J*_{2,3} 4.4 Hz, H-2), 6.38 (1H, br-s, 3-OH); δ_{H} (CDCl₃, 400 MHz): 3.65 (1H, dd, *J*_{5,5'} 10.3 Hz, *J*_{4,5} 5.9 Hz, H-5'), 3.72 (1H, a-t, *J*_{5,5'} 9.88 Hz, H-5), 4.63 (1H, m, H-3), 4.71 (1H, m, H-4), 4.86 (1H, d, *J*_{2,3} 4.4 Hz, H-2).

Advantages to this synthetic step included the ability to carry out the reaction at 45°C dramatically shortened the reaction time from 24 hours as known in the prior art; removal of KBr salt by filtration was possible after treatment with methanol and this is essential to allow easy extraction of the product; and careful temperature control of the quenching reaction was very important to prevent formation of byproducts.

### Example 23(c): 5-Bromo-2,5-dideoxy-D-threo-pentono-1,4-lactone (4)

### Method 1- Sodium Iodide

2,5-Dibromo-2,5-dideoxy-D-lyxo-1,4-lactone 3 (35 g, 127.8 mmol) was dissolved in isopropyl acetate (300 mL) and sodium iodide (76.6 g, 511.2 mmol) and trifluoroacetic acid (14.8 mL) were added at room temp. The reaction mixture was heated to about 85°C (internal temp.) for 1.5 hours. After this time, t.l.c. analysis (1:1, ethyl acetate:hexane) indicated little remaining starting material (R_{f} 0.44) and a new product (R_{f} 0.19). The reaction mixture was cooled to room temperature and stirred for 4 hours. T.l.c. analysis indicated no starting material therefore, the reaction mixture was concentrated *in vacuum* to 20 mL to remove trifluoroacetic acid and diluted with isopropyl acetate (200 mL). The reaction mixture was washed with saturated aqueous sodium hydrogen carbonate solution (200 mL) and the layers were separated. The aqueous layer was further extracted with isopropyl acetate (3 x 200 mL). The combined organic extracts were treated with aqueous sodium thiosulfate solution (48 g in 160 mL water). The aqueous layer was extracted with isopropyl acetate (2 x 200 mL) and the combined organic extracts were dried with sodium sulfate (20g), filtered and concentrated *in vacuum* to yield 5-bromo-2,5-dideoxy-D-threo-pentono-1,4-lactone 4 (16.38 g, crude yield 92%) as an oily brown residue. The obtained product was dissolved in water and used as is for the subsequent reaction. In other runs, the isopropyl acetate was swapped with water and the aqueous solution was used as is for the KOH reaction.
δ_{H} (D₂O, 400 MHz): 2.64 (1H, d, *J*_{2,2'} 18.3 Hz, H-2'), 3.12 (1H, dd, *J*_{2,2'} 18.0 Hz, *J*_{2,3} 5.49 Hz, H-2), 3.45 (0.125H, dd, H-5' and H-5 for iodide 41); 3.70 (2H, a-d, *J* 6.71 Hz, H-5, H-5'), 4.74 (1H, a-t, H-3), 4.87 (1H, m, H-4). δ_{C} (D₂O, 100 MHz): 27.1 (C-5), 39.0 (C-2), 67.9 (C-3), 84.6 (C-4), 178.8 (C-1); δ_{H} (d⁶-DMSO, 400 MHz): 2.34 (1H, a-d, *J*_{2,2'} 17.1 Hz, *J*_{2',3} 6.3 Hz, H-2'), 2.95 (1H, dd, *J*_{2,2'} 17.1 Hz, *J*_{2,3} 5.4 Hz, H-2), 3.39 (0.125H, dd, *J* 7.3 Hz, *J* 6.8 Hz, *J* 11.2 Hz, H-5' and H-5 for iodide 4I), 3.60 (1H, dd, *J*_{5,5} 10.7 Hz, *J*_{4,5'} 8.3 Hz, H-5'), 3.70 (1H, dd, *J*_{5,5} 10.7 Hz, *J*_{4,5} 5.4 Hz, H-5), 4.39 (1H, m, H-3), 4.63 (1H, m, H-4), 5.61 (1H, d, *J*_{3,OH} 4.4 Hz, 3-OH); *m*/*z* (ES -ve): 253 (M+AcOH)⁻; Found: C, 30.69, H, 3.55, Br, 41.22%; C₅H₇BrO₃ requires C, 30.80, H, 3.62, Br, 40.97%.

### Method 2 - Hydrogenation

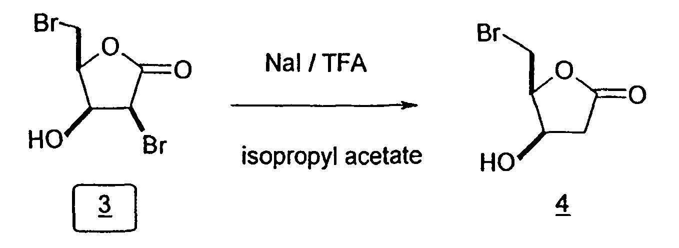

2,5-Dibromo-2,5-dideoxy-D-lyxo-1,4-lactone 3 (7.5 g, 27.6 mmol) was dissolved in ethyl acetate (120 mL) and triethylamine (4 mL, 28.7 mmol) was added to the stirred solution. The reaction mixture was stirred at room temperature under atmosphere of hydrogen (atmospheric pressure) in the presence of 5% dry palladium on carbon (1 g) for about 1.5 hours. After this time, t.l.c. analysis (1:1, ethyl acetate:hexane) indicated a new product (R_{f} 0.16), residual starting material (R_{f} 0.44). Therefore, the reaction mixture was purged with argon (three times) and then stirred under an atmosphere of hydrogen for a further 2 hours. After this time, t.l.c. analysis indicated little starting material therefore, the reaction mixture was filtered through celite (ethyl acetate as eluant), washed with 4M HCl (30 mL) and the aqueous layer further extracted with ethyl acetate (2 x 20 mL). The combined organic extracts were dried with sodium sulfate (10 g), filtered and concentrated *in vacuum* to yield 5-bromo-2,5-dideoxy-D-*threo*-pentono-1,4-lactone 4 (5.15g, crude yield 96%) as a crude pale yellow oil.
δ_{H} (D₂O, 400 MHz): 2.65 (1H, d, *J*_{2,2'} 18.3 Hz, H-2'), 3.09 (1H, dd, *J*_{2,2'} 18.3 Hz, *J*_{2,3} 5.8 Hz, H-2), 3.71 (2H, a-d, *J* 7.01 Hz, H-5, H-5'), 4.74 (1H, a-t, *J* 4.9 Hz, J 4.6 Hz, H-4), 4.87 (1H, m, J 3.7 Hz, H-3); δ_{C} (D₂O, 100 MHz): 27.7 (C-5), 39.5 (C-2), 68.5 (C-3), 85.2 (C-4), 179.5 (C-1); δ_{H} (d⁶-DMSO, 400 MHz): 2.34 (1H, d, *J*_{2,2'} 17.1 Hz, H-2'), 2.93 (1H, dd, *J*_{2,2'} 17.1 Hz, *J*_{2,3} 5.4 Hz, H-2), 3.60 (1H, dd, *J*_{5,5} 10.7 Hz, *J*_{4,5'} 8.3 Hz, H-5'), 3.70 (1H, dd, *J*_{5,5} 10.7 Hz, *J*_{4,5} 5.4 Hz, H-5), 4.40 (1H, m, H-3), 4.65 (1H, m, H-4), 5.58-5.64 (1H, br-s, 3-OH); δ_{C} (d⁶-DMSO, 100 MHz): 29.6 (C-5), 39.5 (C-2), 67.2 (C-3), 83.2 (C-4), 175.4 (C-1).

These steps in the process provided the advantages of the hydrogenation reaction that required significantly less solvent for the extraction, a change to sodium from potassium iodide and heating the reaction shortened reaction time from 6 hours to 2 hours, and the change from acetone to isopropyl acetate improved the extraction of product from the aqueous layer.

### Example 23(d): 2-Deoxy-L-ribono-1,4-lactone 5 from 5-bromo-2,5-dideoxy-D-threo-pentono-1,4-lactone 4:

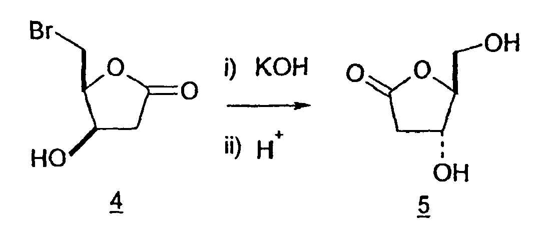

Potassium hydroxide (14.9 g, 230.7 mmol) was dissolved in water (124 mL) and cooled to 15°C. This solution was added to a stirred solution of 5-bromo-2,5-dideoxy-D-threo-pentono-1,4-lactone **4** (15 g, 76.9 mmol) in water (62 mL) at room temperature. After about 3 hours, t.l.c. analysis (2% methanol in ethyl acetate) indicated no remaining starting material (R_{f} 0.55) and a new product (R_{f} 0.0). The reaction mixture was heated to 80°C (internal temp.) for 30 minutess, cooled to room temperature and no change was observed by t.l.c. analysis. Amberlite IR-120 Plus acidic resin (50g) was added and the reaction mixture stirred at room temperature for 30 minutes at which point, the pH was measured as 3. Additional resin (40g) was added and the reaction mixture stirred at room temperature for a further 30 minutes, at which point the pH was measured as 1. The reaction mixture was stirred at room temperature overnight, after which time t.l.c. analysis indicated formation of a new product (R_{f} 0.21). The resin was removed by filtration through a sinter funnel (water as eluant, 200 mL) and concentrated *in vacuum.* Before taking to dryness, co-evaporation with 1,2-dimethoxyethane (2 x 100 mL) was performed. The red residue was dissolved in 1,2-dimethoxyethane (200 mL) and stirred with MgSO₄ (10 g) for 40 minutes at room temperature. Filtration, washing with 1,2-dimethoxyethane (75 mL) and concentration *in vacuum* at 45°C yielded 2-deoxy-L-ribono-1,4-lactone 5 (10.47 g, crude yield 90%) as a crude red solid. In other runs, product was kept in DME and used as is for the subsequent reaction.
δ_{H} (d⁶-DMSO, 400 MHz): 2.22 (1H, dd, *J*_{2,2'} 17.6 Hz, *J*_{2',3} 2.0 Hz, H-2'), 2.80 (1H, dd, *J*_{2,2'} 18.1 Hz, *J*_{2,3} 6.3 Hz, H-2), 3.50 (1H, dd, *J*_{5,5'} 12.2 Hz, *J*_{4,5'} 3.9 Hz, H-5'), 3.54 (1H, dd, *J*_{5,5'} 12.2 Hz, *J*_{4,5} 4.2 Hz, H-5), 4.26 (2H, m, H-3 and H-4), 4.7-5.0 (2H, br-s, OH).

### Example 23(e): 2-Deoxy-3,5-di-O-p-toluoyl-L-ribono-1,4-lactone 6 from 2-deoxy-L-ribono-1,4-lactone 5

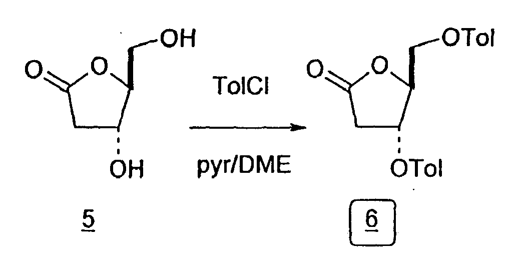

A solution of 2-deoxy-L-ribono-1,4-lactone **5** (10.47 g, 76.9 mmol) and pyridine (31.1 mL, 384.4 mmol) in 1,2-dimethoxyethane (100 mL) was cooled to between 0 and - 5°C under argon. *para-*Toluoyl chloride (21.4 g, 138 mmol) was added from an addition funnel over 20 minutes maintaining the temperature between 0 and -5°C. After 3.5 hours maintaining the temperature between 0 and -5°C, t.l.c. analysis (30% ethyl acetate in hexane) indicated a new product (R_{f} 0.76) and no remaining starting material (R_{f} 0.36) and HPLC analysis indicated the reaction had reached completion. The reaction mixture was cooled (0°C) and quenched with a solution of sodium hydrogen carbonate solution (25 g in 300 mL). A brown oil separated from the reaction mixture which gradually solidified on stirring at room temperature. After 1.5 hours, the solid was filtered, washed with water (150 mL) and the crude solid (25.84 g) was dried overnight. The crude lactone was dissolved in dichloromethane (150 mL) and stirred with MgSO₄ (10 g) for 1 hour. The solid was filtered, washed with dichloromethane (50 mL) and the filtrate concentrated at 40°C to approx. 50 mL TBME (100 mL) was added and the mixture concentrated at 40°C to approximately 50 mL. The residual concentrated solution was then stirred at room temperature and it formed a thick slurry. TBME (50 mL) was added and stirring continued at room temperature for 2 hours. The solid was filtered, washed with TBME (50 mL) and dried under vacuum at 30-35°C overnight to yield 2-deoxy-3,5-di-*O*-p-toluoyl-L-ribono-1,4-lactone **6** (10.46 g, 37% over 3 steps) as a pale brown solid.
δ_{H} (CDCl₃, 400 MHz): 2.42,2.43 (2 x s, 2 x CH₃Ar, 2 x 3H), 2.82 (1H, dd, *J*_{2,2'} 18.7 Hz, *J*_{2,3} 1.8 Hz, H-2'), 3.16 (1H, dd, *J*_{2,2'} 18.7 Hz, *J*_{2,3} 7.3 Hz, H-2), 4.61 (1H, dd, *J*_{5,5'} 12.4 Hz, *J*_{4,5'} 3.3 Hz, H-5'), 4.71 (1H, dd, *J*_{5,5'} 12.1 Hz, *J*_{4,5} 3.7 Hz, H-5), 4.95 (1H, m, H-4), 5.61 (1H, a-d, *J* 7.69 Hz, H-3), 7.25-7.28 (4H, m, 2 x ArH), 7.86-7.93 (2 x 2H, 2 x d, *J* 8.4 Hz, 2 x ArH); δ_{C} (CDCl₃, 100 MHz): 21.9, 35.3, 63.9, 71.8, 82.8, 125.1, 126.5, 129.4, 129.5, 129.6, 130.0, 130.4, 144.6, 145.0, 166.0, 166.1 (2 x ArCO₂), 174.2 (C-1).

Advantages to this step of the overall process were that toluoyl anhydride was removed from the process via treatment with TBME, and a column chromatography step was eliminated from process.

### Example 23(f): 2-Deoxy-3,5-di-O-para-toluoyl-L-ribose 7 from 2-deoxy-3,5-di-O-para-toluoyl-L-ribono-1,4-lactone 6:

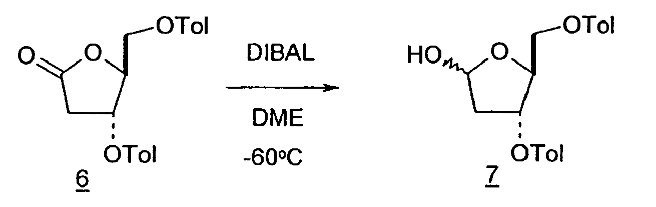

A solution of 2-deoxy-3,5-di-*O*-*p*-toluoyl-L-ribono-1,4-lactone **6** (9.0 g, 24.42 mmol) in 1,2-dimethoxyethane (90 mL) was cooled to approximately -60°C under argon with overhead stirring. A 1M solution of diisobutylaluminium hydride in toluene (32.4 mL, 32.4 mmol) was added drop-wise *via* an addition funnel over 15 minutes. The internal temperature was maintained at -60°C for 1 hour and HPLC analysis indicated completion of the reaction. The reaction mixture was quenched *via* addition of acetone (10 mL) over 2 minutes and then addition of 5N HCl (30 mL) over 5 minutes. The mixture was stirred at room temperature over 30 minutes and concentrated *in vacu*um at 35°C to approximately 30 mL. The residual oil was combined with brine (24 g in 60 mL) and extracted with ethyl acetate (3 x 100 mL). The combined organic extracts were dried with sodium sulfate (10 g), concentrated to a volume of 50 mL and co-evaporated with TBME to yield 2-deoxy-3,5-di-*O*-*p*-toluoyl-□,□-L-ribose **7**. A portion was concentrated *in vacuum* to dryness for ¹H NMR analysis. This product was not further characterized as it was used crude in the next step.
δ_{H} (CDCl₃, 400 MHz, ratio of anomers is 0.75:1): 2.2-2.6 (m, 2 x CH₃Ar and H-2/H-2' for □ and □-anomers), 4.4-4.75 (m, H-4 and H-5/H-5' for □ and □-anomers), 5.4-5.8 (4 x m, H-1 and H-3 for A and □), 7.18-8.02 (m, 8H, aromatic protons for A and B).

### Example 23(g): 2-Deoxy-3,5-di-O-para-toluoyl-□-L-erythro-pentofuranosyl chloride 8

### Method 1: Directly from Lactol 7

A solution of 2-deoxy-3,5-di-O-*p*-toluoyl-□,□-L-ribose 7 (approx. 7.0g, 18.91 mmol) in TBME (30 mL) was diluted with TBME (15 mL) and stirred for 20 minites at room temperature. Acetic acid was added in three portions of 1 mL with stirring until a clear brown solution was obtained. This was cooled to 0°C under argon and dry HCl gas was passed into it in a steady stream for 25 minutes. After 10 minutes at 0°C, an aliquot was quenched with anhydrous ethanol (1.2 mL) and allowed to sit at room temperature with occasional shaking for 10 minutes when a clear solution was obtained. The reaction mixture was maintained between 0-5°C and after 65 minutes the reaction mixture was filtered. The solid product was washed with TBME (30 mL) and dried in vacuum for 5 hours to yield 2-deoxy-3,5-di-O-*p*-toluoyl-□-L-erythro-pentofuranosyl chloride **8** (4.79 g, 65%) as a white crystalline solid. m.p. 118-121°C.
δ_{H} (CDCl₃, 400 MHz): 2.41, 2.43 (2 x s, 2 x CH₃Ar, 2 x 3H), 2.74 (1H, a-d, *J*_{2,2'} 14.6 Hz, H-2'), 2.87 (1H, ddd, *J*_{2,2'} 12.7 Hz, *J*_{2,3} 7.3 Hz, *J*_{1,2} 5.3 Hz, H-2), 4.59 (1H, dd, *J*_{5,5'} 12.2 Hz, *J*_{4,5'} 4.4 Hz, H-5'), 4.68 (1H, dd, *J*_{5,5'} 12.2 Hz, *J*_{4,5} 3.4 Hz, H-5), 4.86 (1H, m, *J*_{3.4} Hz, H-3/H-4), 5.56 (1H, a-dd, *J* 1.95 Hz, *J* 6.3 Hz, H-3/H-4), 6.47 (1H, d, *J*_{1,2} 5.4 Hz, H-1), 7.23-7.28 (4H, m, 2 x ArH), 7.89, 7.99 (2 x 2H, 2 x d, *J* 8.3 Hz, 2 x ArH). [α]_{D}²⁵-117 (c,1.0 in CHCl₃)[CMS Chemicals Ltd: [α]_{D}²⁰ -118.9 (c, 1 in DCM)]

### Method 2: via methoxide 7-OMe

A solution of 1% HCl in methanol was prepared *via* addition of acetyl chloride (0.2 mL) to methanol (10 mL) previously cooled to 5°C. 2-Deoxy-3,5-di-*O*-*p*-toluoyl-□,□-L-ribose 7 (470 mg, 1.27 mmol) was dissolved in anhydrous methanol (9 mL) and cooled to about 10°C. A portion of a solution of 1% HCl in methanol (1 mL) was added and the reaction mixture maintained at 10-15°C for 1.5 hours. After this time, HPLC analysis indicated unreacted starting material, therefore, an additional portion of 1% HCl in methanol (1 mL) was added and stirring continued at room temperature for a further 1.5 hours. HPLC analysis indicated the reaction was close to completion and the reaction was concentrated *in vacuum* at 30°C and co-evaporated with TBME (10 mL). The residue was dissolved in TBME (10 mL) and suspended white solids were observed. Ethyl acetate (15 mL) was added to dissolve the suspension and the solution was dried with sodium sulfate (2 g), filtered and concentrated *in vacuum* to yield methyl 2-deoxy-3,5-di-O-*p*-toluoyl-□,□-L-riboside 7-OMe (480 mg, crude yield 98%) as a brown oil.
δ_{H} (CDCl₃, 400 MHz, ratio of anomers is 1:1): 2.2-2.6 (m, 2 x CH₃Ar and H-2/H-2' for □ and □-anomers), 3.36 (s, 3H, OCH₃ for □), 3.42 (s, 3H, OCH₃ for □), 4.4-4.6 (m, H-4 and H-5/H-5' for □ and □-anomers), 5.19 (d, 1H, H-1 for □), 5.21 (dd, 1H, H-1 for □), 5.41 (m, 1H, H-3 for □), 5.59 (m, 1H, H-3 for □), 7.18-8.02 (m, 8H, aromatic). δ_{C} (CDCl₃, 100 MHz): 21.9, 39.5, 55.3, 55.4, 64.5, 65.3, 74.8, 75.6, 81.2, 82.1, 105.3, 105.8, 127.1, 127.2, 127.3, 127.4, 129.3, 129.3, 129.9, 130.0, 130.0, 143.9, 144.0, 144.1, 144.2, 166.3, 166.5, 166.6, 166.7.

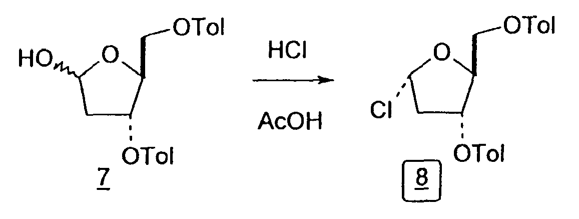

Methyl 2-deoxy-3,5-di-O-*p*-toluoyl-□,□-L-riboside 7-OMe (480 mg, 1.25 mmol) was dissolved in TBME (3 mL) and acetic acid (1 mL) and the solution cooled to 0°C under argon. Dry HCl gas was bubbled into this solution for 15 minutes and the reaction mixture allowed to stir at 0-5°C for an additional 10 minutes. HPLC analysis indicated remaining starting material, even after the reaction had continued for a further 1 hour and 10 minutes. The white solid that crystallized out of the reaction mixture was collected by filtration, washed (TBME) and dried under vacuum to yield 2-deoxy-3,5-di-O-*p*-toluoyl-□-L-erythro-pentofuranosyl chloride 8 (228 mg, 47% over 3 steps) as a white crystalline solid. The compound isolated was identical in all respects to that reported above for **Method 1.**

### Example 23(h): 2'-Deoxy-3',5'-di-O-para-toluoyl L-thymidine 9

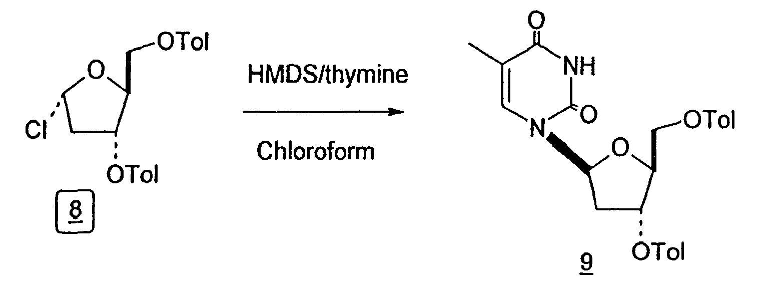

A mixture of thymine (1.0g, 7.92 mmol), HMDS (1.66g, 10.28 mmol) and ammonium sulfate (100 mg, 0.76 mmol) was heated at about 145°C for 2 hours at which point the thymine had dissolved. After a further 4 hours at 145°C, the reaction mixture was concentrated *in vacuum* at 60°C. The silylated thymine thus obtained (7.92 mol) was suspended in anhydrous chloroform (15 mL) and cooled to 15°C. 2-Deoxy-3,5-di-O-*p-*toluoyl-□-L-erythro-pentofuranosyl chloride **8** (1.48g, 3.8 mmol) was added portion-wise over 2-3 minutes and the reaction mixture (yellow solution) stirred at room temperature under argon. After 2 hours, HPLC analysis indicated no starting material. The reaction was cooled to about 5°C and quenched with 190% proof ethanol (0.25 mL). Precipitation of a white solid occurred and the reaction was stirred at room temperature for 20 minutes. The reaction mixture was filtered through celite (10g) and washed with dichloromethane (60 mL). The filtrate was washed with water (2 x 25 mL) and allowed to stand to break the emulsion. The organic layer was further washed with aq. sodium bicarbonate solution (2g in 25 mL of water) and brine (10g NaCl in 30 mL of water). The organic layer was dried with sodium sulfate (5g) and filtered through celite (8g). The filtrate was concentrated *in vacuum* at 40°C and the residue was suspended in hexane (20 mL) which was stirred at room temperature for 1.5 hours to get a uniform dispersion. The mixture was filtered, filter cake washed with hexane (10 mL) and dried briefly under vacuum to get a white solid. This solid was stirred in ethanol (30 mL) at 60°C for 40 minutes, concentrated *in vacuum* (removed 15 mL) and the residual slurry cooled to room temperature. The slurry was filtered, washed with cold ethanol (10 mL) and TBME (5 mL) and dried under vacuum at 55°C overnight to yield 2'-deoxy-3',5'-di-O-*para-*toluoyl-L-thymidine **9** (1.38g, 76%) as a white solid. HPLC: □:□ ratio = 268:1.
δ_{H} (CDCl₃, 400 MHz): 1.61 (3H, s, 5-Me), 2.31 (1H, m, H-2"), 2.42, 2.43 (2 x 3H, 2 x s, CH₃Ar), 2.71 (1H, dd, *J* 4.8 Hz, *J*_{2',2''} 14.3 Hz, H-2'), 4.52 (1H, m, H-4'), 4.64 (1H, dd, *J*_{4',5''} 3.3Hz, *J*_{5',5''} 12.5Hz, H-5"), 4.77 (1H, dd, *J*_{4',5'} 2.6 Hz, *J*_{5',5''} 12.5 Hz, H-5'), 5.64 (1H, a-d, *J* 6.6 Hz, H-3'), 6.47 (1H, dd, *J*_{1,2} 5.5 Hz, *J*_{1,2} 8.8 Hz, H-1'), 7.2-7.3 (5H, m, H-6 and ArH), 7.91-7.96 (4H, m, Ar-H), 8.6 (1H, br-s, NH); δ_{C} (CDCl₃, 100 MHz): 12.3, 21.5, 21.9, 38.2, 64.4, 75.1, 83.0, 85.0, 111.9, 126.3, 126.7, 129.5, 129.7, 129.7, 130.0, 134.6, 144.8, 150.6, 163.8, 166.2, 166.3.

### Example 23(i): 2'-Deoxy-L-thymidine 10

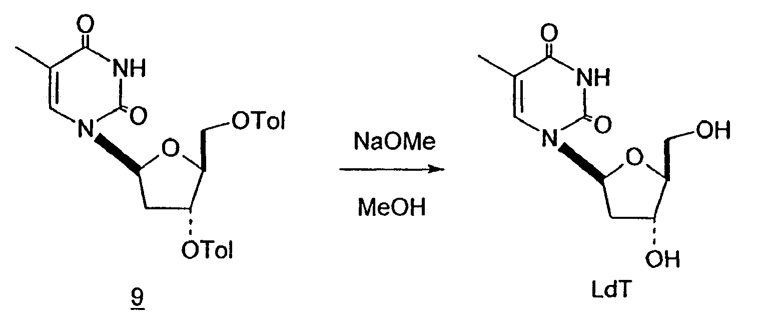

A stirring suspension of 2'-deoxy-3',5'-di-O-*para*-toluoyl-L-thymidine **9** (500 mg, 1.05 mmol) in anhydrous methanol (6 mL) was cooled to 5°C under argon. Sodium methoxide (64 mg, 1.19 mmol) was added in one portion. After 5 minutes, the cooling bath was removed and the reaction mixture stirred at room temperature for 30 minutes. The reaction mixture was heated to 45-50°C for 1 hour and it remained mostly as an insoluble suspension. Anhydrous tetrahydrofuran (4 mL) was added and a clear solution was obtained. The temperature was maintained at 45-50°C for a further 30 minutes at which point HPLC analysis indicated remaining starting material. Therefore, after a further 30 minutes (total 2.5 hours), an additional portion of sodium methoxide (31 mg, 0.57 mmol) was added and the reaction maintained at 45-50°C. After 2.5 hours (total 5 hours), HPLC analysis indicated the reaction had not reached completion, therefore, after a further 1 hour (total 6 hours) an additional portion of sodium methoxide (25 mg, 0.46 mmol) was added and the reaction mixture stirred overnight at 40-45°C. After this time, HPLC and t.l.c analysis (10% methanol in ethyl acetate) indicated complete conversion of starting material (R_{f} 0.73) to product (R_{f} 0.15)[sample prep. for HPLC and t.l.c. analysis = aliquot with Dowex-H⁺ resin, diluted with methanol, filtered and analyzed]. The reaction mixture was cooled to room temperature and DOWEX 50W x 2-200(H) ion-exchange resin (previously washed with methanol (3 x 10 mL)) was added. After stirring for 30 minutes at room temperature, the pH was 3 and the reaction mixture was filtered using a frittered glass funnel, washed with methanol (5 mL) and the filtrate concentrated *in vacuum* at 45°C. The residual methanol was co-evaporated with a mixture of TBME and dichloromethane (1:1, 10 mL) and the residue was dissolved in TBME (10 mL). The solids were dispersed at 40-45°C for 1 hour, cooled to room temperature and filtered. The resulting solid was washed with TBME (5 mL) and dried under vacuum to yield 2-deoxy-L-thymidine **10** (225 mg, 88%) as a white solid.

This compound was found to be identical in all respects to an authentic sample of 2-deoxy-L-thymidine **10.**

### Example 24

### 2,2'-anhydro-1-(5-O-dimethoxytrityl-□-D-arabinofuranosyl)thymine (2) from 2,2'-anhydro-1-□-D-arabinofuranosyl)thymine (1)

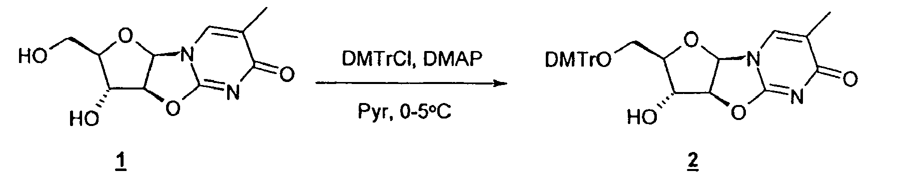

To a previously cooled (0-5°C) mixture of 2,2'-anhydro-1-□-D-arabinofuranosyl)-thymine **1** (2.40 g, 10.0 mmol) and DMAP (111 mg, 0.9 mmol) in anhydrous pyridine (15 mL), was added 4,4'-dimethoxytrityl chloride (3.56 g, 10.5 mmol) portionwise over a period of 3 minutes. The resulting mixture was kept stirring at 0-5°C under argon and after 1.5 h, t.l.c. analysis (silica plate, 1:9 methanol:dichloromethane) indicated no remaining starting material. The reaction mixture was concentrated *in vacuo* at 45°C. The residue was taken into dichloromethane (50 mL) and sat. sodium bicarbonate solution (20 mL). After stirring at room temperature for 10 minutes, the layers were separated and the organic layer was washed with distilled water (2 x 20 mL) and dried with anhydrous sodium sulfate. The reaction mixture was concentrated *in vacuo* at 50°C and residue was co-evaporated with toluene (2 x 10 mL). The resulting crude residue was triturated with dichloromethane (5 mL) and TBME (25 mL). After stirring at room temperature for 1 hour, the solid was collected by filtration under reduced pressure and washed with TBME (15 mL). The yellow solid was dried under vacuum affording 2,2'-anhydro-1-(5-*O*-dimethoxytrityl-□-D-arabinofuranosyl)thymine **2** (5.3 g, 97.8% yield, 91% AUC (area under curve) by HPLC analysis).
δ_{H} (d₆-DMSO, 400 MHz): 1.78 (3H, s, Me), 2.76 and 2.90 (2H, ABX, H-5' and H-5"), 3.72 (6H, s, 2 x OMe), 4.22-4.28 (2H, 2 x m, H-3' and H-4'), 5.17 (1H, d, *J*_{1',2'} 5.9 Hz, H-2'), 5.93, (1H, d, *J*_{3',OH} 4.4 Hz, 3'-OH), 6.30 (1H, d, *J*_{1',2'} 5.9 Hz, H-1'), 6.79-7.28 (13H, m, Ar-H), 7.84 (1H, s, H-6).

### Example 25

### 2'-Deoxy-5'-O-dimethoxytrityl-□-D-thymidine (3) from 2,2'-aahydro-1-(5-O-dimethoxytrityl-□-D-arabinofuranosyl)thymine (2)

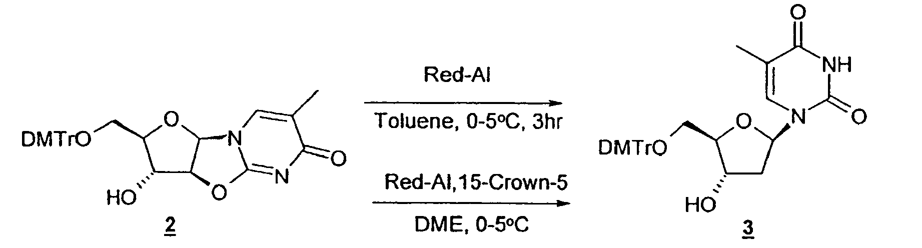

**Example 25** illustrates a comparison between the percent yield of the 5'-DMTrO-protected 2'-deoxy-thymidine (**3**) obtained from **Method 1,** which utilizes Red-Al in toluene, with the % yield of product (**3**) obtained from **Method 2,** which uses Red-Al in combination with 15-Crown-5 ether in DME. **Method 1** was carried out using known methods (e.g., U.S. Patent No. 6,090,932) and provided the product (**3**) in 21% yield. **Method 2** was carried out according to the process of the present invention that utilized Red-Al in combination with 15-Crown-5 ether in DME, which provided the product (**3**) in 35% yield.

### Method 1: Red-Al reaction of 2,2'-anhydro-1-(5-O-dimethoxytrityl-□-D-arabino-furanosyl)thymine 2 in Toluene

To a previously cooled (0-5°C) solution of 2,2'-anhydro-1-(5-*O*-dimethoxytrityl-□-D-arabinofuranosyl)thymine **2** (1.08 g, 2.0 mmol) in anhydrous toluene (50 mL) was added Red-Al (65 wt % in toluene, 0.90 mL, 3.0 mmol) dropwise over a period of 10 minutes. The mixture was kept stirring at 0-5°C under argon. The reaction was monitored by t.l.c. (silica, 5:95 methanol in dichloromethane) and HPLC analysis. After stirring for 2 hours at 0-5°C, additional Red-Al (0.5 equiv, 65 wt% in toluene, 0.30 mL, 1.0 mmol) was added to the reaction mixture. After stirring for 45 minutes, an aliquot from the reaction mixture was taken into HPLC grade THF (ca. 1 mL), quenched with drops of distilled water and injected on an HPLC instrument. The result indicated a 1:1 ratio of product (37.4% AUC) vs. starting material (36%AUC). The reaction was quenched by adding brine (30 mL) at 5°C. After stirring for a further 30 minutes, the mixture was filtered through a celite pad and washed with ethyl acetate (60 mL). The filtrate was partitioned in a separation funnel. The organic layer was washed with sat. aq. NH₄Cl solution (30 mL)and brine (2 x 25mL) and dried using anhydrous sodium sulfate. The reaction mixture was concentrated *in vacuo* at 40°C. The crude residue (1.01 g, yellow foamy solid) was purified by column chromatography (silica gel, 5% methanol in dichloromethane) to yield 2'-deoxy-5'-*O*-dimethoxytrityl-□-D-thymidine **3** (0.23g, 21% yield) as a light yellow solid.
δ_{H} (d₆-DMSO, 400 MHz): 1.43 (3H, s, Me), 2.14 and 2.22 (2 x 1H, 2 x m, H-2' and H-2"), 3.18 (2H, m, H-5" and H-5'), 3.72 (s, 6H, 2 x OMe), 3.87 (1H, m, H-4'), 4.30 (1H, m, H-3'), 5.32 (1H, d, *J*_{3',OH} 4.4 Hz, 3'-OH), 6.19 (1H, m, H-1'), 6.85-7.39 (13H, m, DMTr), 7.50 (1H, s, H-6), 11.38 (1H, s, NH); MS (ESI+, M+1 = 545.3, M+Na⁺= 567.3).

### Method 2: Red-Al reaction of 2,2'-anhydro-1-(5-O-dimethoxytrityl-□-D-arabino-furanosyl)thymine 2 in DME in the presence of 15-crown-5

To a previously cooled (0-5°C) solution of 2,2'-anhydro-1-(5-*O*-dimethoxytrityl-□-D-arabinofuranosyl)thymine **2** (120 mg, 0.22 mmol) and 15-crown-5 (65µl, 0.33 mmol) in anhydrous DME (5ml) was added Red-Al (65 wt% in toluene, 0.10 mL, 0.33 mmol) dropwise over a period of 4 minutes. The reaction mixture was maintained at 0-5°C under argon. The reaction was monitored by HPLC analysis. After stirring for 3.5 hours, additional Red-Al (0.5 equiv, 65 wt% in toluene, 0.030 mL, 0.10 mmol) was added to the reaction mixture. After stirring for 30 minutes, HPLC analysis results indicated the product:starting material ratio increased from 1.7:1 to 2.4:1. The reaction mixture was allowed to warm to room temperature and was maintained at room temperature for 16 hours. HPLC analysis indicated the product to starting material ratio increased slightly to 2.8:1. The reaction mixture was then cooled to 0-5°C and a further 0.5 equivalent, of Red-Al (65 wt% in toluene, 0.030 mL, 0.10 mmol) was added to the reaction mixture. After stirring at 0-5°C for 1 h, HPLC results indicated that the product to starting material ratio increased to 4.0:1 (62.7% AUC of product vs. 15.8% AUC starting material). Further addition of 15-crown-5 and Red-Al did not result in improvement in the product formation. To the reaction mixture was added a small amount of acetone (ca. 0.1 mL). After stirring for 10 minutes, the reaction mixture was concentrated *in vacuo* at 40°C and the residue was co-evaporated with isopropyl acetate (10 mL). The residue was partitioned between isopropyl acetate (20 mL) and distilled water (5 mL). The organic layer was washed with saturated aqueous NH₄Cl solution (5 mL) and brine (5 mL) and dried with anhydrous sodium sulfate. After concentration in vacuo at 40°C, the crude residue (120 mg, yellow foamy solid) was purified by column chromatography (silica gel, 5% methanol in dichloromethane) to yield 2'-deoxy-5'-*O*-dimethoxytrityl-□-D-thymidine **3** as light yellow solid (45mg, 35% yield). The ¹H-NMR spectrum conforms to the structure obtained in **Method 1.**

### Example 26

### 2,2'-anhydro-1-(5-O-trityl-□-D-arabinofuranosyl)thymine (4) from 2,2'-anhydro-1-□-D-arabinofuranosyl)thymine (1)

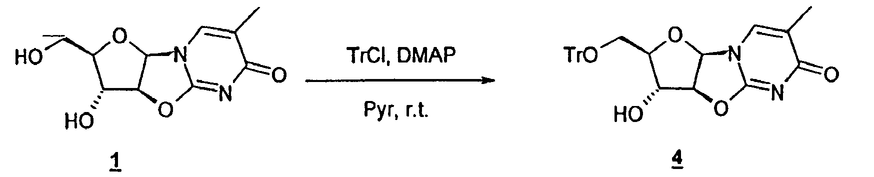

2,2'-anhydro-1-□-D-arabinofuranosyl)thymine **1** (500 mg, 2.08 mmol) was dissolved in anhydrous pyridine (5 mL) and DMAP (12.5 mg, 0.1 mmol) was added to the stirred solution. Trityl chloride (1.28 g, 2.29 mmol) was added portionwise over 3 minutes at room temperature. The resulting reaction mixture was stirred at room temperature for 2 hours and then at 40°C overnight under argon. After this time, t.l.c. analysis (silica plate, 2:8 methanol:dichloromethane) indicated no remaining starting material (R_{f} 0.3) and formation of a new product (silica plate, 1:9 methanol:dichloromethane, R_{f} 0.17). The reaction mixture was cooled to 0°C using an ice bath, saturated NaHCO₃ solution (15 mL) was slowly added portionwise and a white solid precipitated from solution. The resulting suspension was stirred at room temperature for 30 minutes, the white solid was filtered and then washed with distilled water (25 mL). The crude solid (3 g) was taken into TBME (18 mL) and stirred at room temperature for 30 minutes. The white solid was filtered, washed with TBME (8 mL) and then dried under vacuum to yield 2,2'-anhydro-1-(5-*O*-trityl-□-D-arabinofuranosyl)thymine **4** (844 mg, 84%) as a white solid.
δ_{H} (d₆-DMSO, 400 MHz): 1.77 (3H, s, Me), 2.73 (1H, dd, *J*_{4',5"} 7.4 Hz, *J*_{5',5"} 10.2 Hz, H-5"), 2.92 (1H, dd, *J*_{4',5'} 4.3 Hz, *J*_{5',5"} 10.2 Hz, H-5'), 4.24-4.28 (2 x 1H, 2 x m, H-3' and H-4'), 5.16 (1H, d, *J*_{1',2'} 5.86 Hz, H-2'), 5.94 (1H, d, *J*_{3',OH} 4.23 Hz, 3'-OH), 6.29 (1H, d, *J*_{1',2'} 5.45 Hz, H-1'), 7.2-7.27 (15H, m, Tr), 7.83 (1H, br-s, H-6).

### Example 27

### 2'-Deoxy-5'-O-trityl-□-D-thymidine (5) from 2,2'-anhydro-1-(5-O-trityl-□-D-arabino-furanosyl)thymine (4)

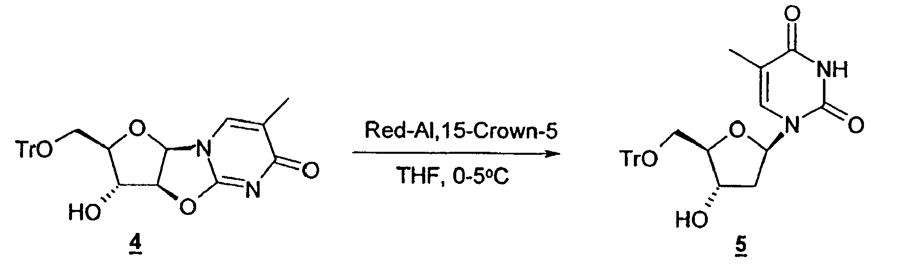

To a previously cooled (0-5°C) mixture of 2,2'-anhydro-1-(5-*O*-trityl-□-D-arabinofuranosyl)thymine **4** (241 mg, 0.5 mmol) and 15-crown-5 (0.15 mL, 0.75 mmol) in anhydrous THF (10 mL) was added Red-Al (65% wt. in toluene, 0.23 mL, 0.75 mmol) dropwise over a period of 5 minutes. The mixture was maintained at 0-5°C under argon. The reaction was monitored by t.l.c. (silica, 5:95 methanol in dichloromethane) and HPLC analysis. After stirring at 0-5°C for 1 hour, an aliquot from reaction mixture was taken into HPLC grade THF (*ca.* 1 mL), quenched with drops of distilled water and injected on an HPLC instrument. The result indicated only 8% AUC (area under curve) of starting material remained and 70.8% of product was present. The reaction was quenched by adding saturated aqueous NH₄Cl solution (5 mL) at 5°C and stirred for 15 minutes. After this time, the layers were separated and the aqueous layer was further extracted with isopropyl acetate (10 mL). The organic layers were combined, washed with brine (5 mL) and dried with anhydrous sodium sulfate. After concentration *in vacuo* at 40°C, the crude residue (287 mg, white foamy solid) was purified by column chromatography (silica gel, 5% methanol in dichloromethane) to yield 2'-deoxy-5'-O-trityl-□-D-thymidine **5** (106 mg, 44% yield) as a white solid.
δ_{H} (d₆-DMSO, 400 MHz): 1.45 (3H, s, Me), 2.15 (1H, m, H-2"), 2.22 (1H, m, H-2'), 3.15 (1H, dd, *J*_{4',5"} 2.6 Hz, *J*_{5',5"} 10.5 Hz, H-5"), 3.22 (1H, dd, *J*_{4',5'} 4.8 Hz, J_{5',5"} 10.5 Hz, H-5'), 3.87 (1H, m, H-4'), 4.31 (1H, m, H-3'), 5.33 (1H, d, _{J3,OH} 4.83 Hz, 3'-OH), 6.19 (1H, a-t, *J* 6.6 Hz, *J* 7.0 Hz, H-1'), 7.25-7.39 (15H, m, Tr), 7.49 (1H, br-s, H-6), 11.35 (1H, s, NH).
δ_{C} (d₆-DMSO, 100 MHz): 11.7, 54.9, 70.4, 83.7, 85.4, 86.4, 109.6, 127.2, 128.0, 128.3, 135.7, 143.5, 150.4, 163.7

Product compound (**5**) was deprotected using acetic acid at about 50 °C to produce 2'-deoxy-D-thymidine as a final product, which was identical in all respects to an authenticated sample of known 2'-deoxy-D-thymidine.

### Example 28

### Formation of 2'-deoxy-D-thymidine (4) from 2,2'-anhydro-1-(β-D-arabinofuranosyl) thymine (1)

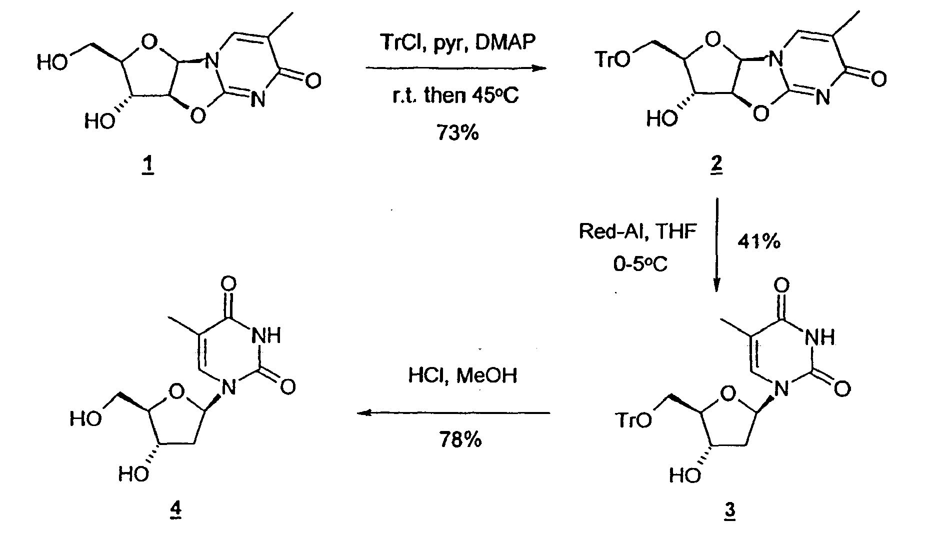

### Example 28(a) 2,2'-anhydro-1-(5-O-trityl-□-D-arabinofuranosyl) thymine (2) from 2,2'-anhydro-1-(□-D-arabinofuranosyl) thymine (1)

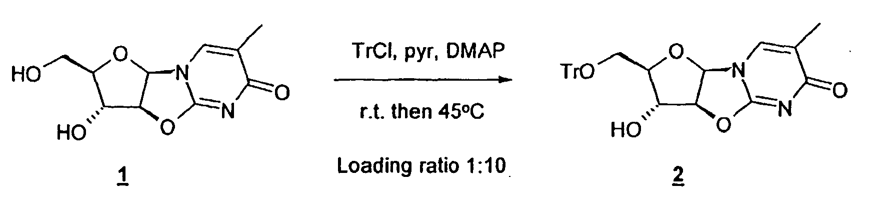

2,2'-Anhydro-1-(□-D-arabinofuranosyl) thymine (**1**) (10.0g, 41.62 mmol) was suspended in pyridine (100 mL) and DMAP (254 mg, 2.08 mmol) and trityl chloride (25.48 g, 91.56 mmol) were added portionwise at room temp. The reaction mixture was maintained at room temperature for about 1 hour and then heated to about 45°C (internal temp.). After about 5 hours, t.l.c. analysis (10% methanol in dichloromethane, visualized using 1% KMnO₄ and UV) indicated significant starting material (R_{f} 0.15) and formation of product (R_{f} 0.43). Therefore, the reaction mixture was maintained at about 45°C for about a further 15 hours (overnight). After this time, t.l.c. analysis indicated no remaining starting material (R_{f} 0.15). The reaction mixture was cooled to about 0°C and saturated aqueous NaHCO₃ solution (320 mL) was slowly added over a 15 minute time period (no change in internal temperature). A white solid immediately precipitated from solution and the white suspension was stirred for about 30 minutes at room temp. The solid was isolated by filtration through a Buchner funnel and washed with water (3 x 100 mL). The residual solid was taken into dichloromethane (150 mL) and stirred for about 30 minutes at room temperature. The remaining residue was isolated by filtration through a Buchner funnel, washed with dichloromethane (20 mL) and dried under vacuum overnight to yield 2,2'-anhydro-1-(5-*O*-trityl-□-D-arabinofuranosyl) thymine (**2**) (14.66g, 73%) as a white solid.
δ_{H} (d⁶-DMSO, 400 MHz): 1.77 (3H, s, 5-Me), 2.76 (1H, dd, *J*_{5',5"} 10.3 Hz, *J*_{4',5"} 7.8 Hz, H-5"), 2.94 (1H, dd, *J*_{5',5"} 10.3 Hz, *J*_{4',5'} 3.9 Hz, H-5'), 4.26 (1H, m, H-4'), 4.29 (1H, m, H-3'), 5.17 (1H, a-d, *J* 5.9 Hz, H-2'), 5.98 (1H, br-s, 3-OH), 6.30 (1H, d, *J*_{1',2'} 5.37 Hz, H-1'), 7.2-7.27 (15H, m, Tr), 7.83 (1H, s, H-6); δ_{C} (d⁶-DMSO, 125 MHz): 13.5 (5-Me), 63.2 (C-5'), 74.8 (C-3'), 85.9 (TrC), 86.7 (C-4'), 88.1 (C-2'), 89.9 (C-1'), 116.9 (C-6), 127.0, 127.7, 127.9, 128.0 (Tr), 132.1 (C-5), 143.3 (Tr), 158.8 (C-2), 171.3 (C-4).

### Example 28(b) 2'-Deoxy-5'-O-trityl-□-D-thymidine 3 from 2,2'-anhydro-1-(5-O-trityl-□-D-arabinofuranosyl) thymine 2

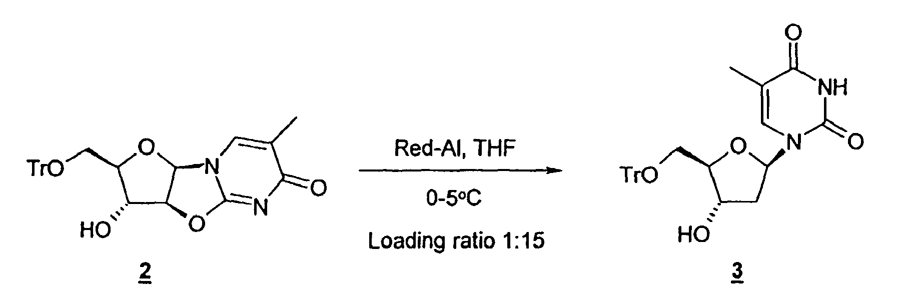

2,2'-Anhydro-1-(5-*O*-trityl-□-D-arabinokranosyl) thymine (**2**) (4.30g, 8.91 mmol) was suspended in anhydrous tetrahydrofuran (43 mL) and cooled to about 0-5°C using an ice-bath. In a separate flask immersed in an ice-bath at about 0-5°C, a 65% wt solution of Red-Al in toluene (3.26 mL, 10.69 mmol) was diluted by addition to anhydrous tetrahydrofuran (21.5 mL). This diluted Red-Al solution was cooled to about 0-5°C and added dropwise *via* syringe to the suspension of 2,2'-anhydro-1-(5-*O*-trityl-□-D-arabinofuranosyl) thymine (**2**) in tetrahydrofuran. The rate of dropwise addition of the Red-Al solution is critical to the reaction and was completed in about 1 hour. The resulting clear solution was maintained at about 0-5°C for 1 hour after which time, t.l.c. analysis (10% methanol in dichloromethane) indicated the presence of starting material (R_{f} 0.34), required product (R_{f} 0.47) and impurities (R_{f} 0.42 and 0.26). HPLC analysis indicated presence of starting material (11.35 mins, 36.5% AUC), product (12.60 mins, 24%) and little of the major impurity (11.7 mins, 2.9%). After a total of about 2 hours at about 0-5°C, an additional portion of an "undiluted" 65% wt solution of Red-Al in toluene (1.63 mL, 5.35 mmol) was added dropwise *via* syringe over a period of about 20 minutes to the reaction mixture, which was maintained at about 0-5°C. After about a further 1 hour, t.l.c. and HPLC analysis indicated presence of starting material (11.35 mins, 3.2%). A further portion of a 65% wt solution of Red-Al in toluene (0.26 mL, 0.85 mmol) was added dropwise and the reaction mixture maintained at about 0-5°C for a further 45 minute period. After this time, t.l.c. analysis indicated only a trace amount of remaining starting material. The reaction was quenched by addition of saturated NH₄Cl solution (40 mL) and the tetrahydrofuran layer was decanted. The aqueous layer was extracted with isopropylacetate (50 mL) and the resulting emulsion was broken by slow addition of 5N HCl solution (15 mL). The organic layer was separated, combined with the tetrahydrofuran layer and washed with saturated NH₄Cl solution (30 mL) and then brine (30 mL). The pH of the brine layer was 6.5 to 7 at this point, and the organic layer was dried with Na₂SO₄, filtered and concentrated *in vacuo* to yield a foamy solid (4.4g). The crude residue was co-evaporated with toluene (30 mL), concentrated *in vacuo* and the resulting residue was taken into toluene (25 mL) by heating to about 45°C. The mixture was cooled to room temp. and stirred at this temperature until a white solid began to precipitate. Water (8.5 mL) was added dropwise and the resulting mixture stirred at room temperature for about 3 hours. The solid was isolated by filtration and the filter cake washed with water (5 mL) and toluene (3 mL). The solid was dried at about 45°C under high vacuum for approximately 1 hour and then at room temperature under vacuum overnight to yield 2'-deoxy-5'-*O*-trityl-□-D-thymidine (**3**) (1.77g, 41%) as a white solid.
δ_{H} (d₆-DMSO, 400 MHz): 1.45 (3H, s, Me), 2.15 (1H, m, H-2"), 2.22 (1H, m, H-2'), 3.15 (1H, dd, *J*_{4',5"} 2.6 Hz, *J*_{5',5"} 10.5 Hz, H-5"), 3.22 (1H, dd, *J*_{4'5'} 4.8 Hz, *J*_{5',5"} 10.5 Hz, H-5'), 3.87 (1H, m, H-4'), 4.31 (1H, m, H-3'), 5.33 (1H, d, J_{3',OH} 4.83 Hz, 3'-OH), 6.19 (1H, a-t, *J* 6.6 Hz, *J* 7.0 Hz, H-1'), 7.25-7.39 (15H, m, Tr), 7.49 (1H, br-s, H-6), 11.35 (1H, s, NH). δ_{C} (d₆-DMSO, 100 MHz): 11.7, 54.9, 70.4, 83.7, 85.4, 86.4, 109.6, 127.2, 128.0, 128.3, 135.7, 143.5, 150.4, 163.7.

### Example 28(c) 2'-Deoxy-D-thymidine (4) from 2'-deoxy-5'-O-trityl-□-D-thymidine (3)

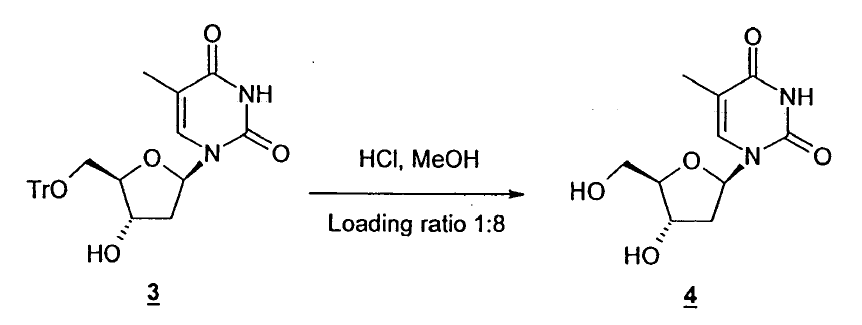

2'-Deoxy-5'-*O*-trityl-□-D-thymidine (**3**) (1.215 g, 2.5 mmol) was suspended in methanol (9.6 mL) and the reaction mixture was heated to about 45°C in a water bath until dissolved. The flask was then cooled to room temp. and concentrated HCl (200 µL, 2.5 mmol) was added to the mixture and stirred at room temp. After about 25 minutes, a white solid began to precipitate from the solution. After about 1 hour, t.l.c. analysis (10%methanol in dichloromethane, visualized by vanillin and UV) indicated no remaining starting material (R_{f} 0.53) and formation of major product (R_{f} 0.21). A portion of n-heptane (10 mL) was added to the reaction mixture and stirred at room temp. for about .15 minutes. The white solid was isolated by filtration (405 mg of solid). The filtrate was split into two layers and the methanol layer was extracted with n-heptane (10 mL) and then concentrated *in vacuo* to a volume of 2 mL. The residue was combined with the 405 mg of white solid, suspended in TBME (10 mL) and stirred at room temperature for about 1 hour. The white solid was isolated by filtration, washed with TBME (3 mL) and dried under vacuum in an oven to yield 2'-deoxy-D-thymidine (**4**) (471 mg, 78%). This product was identical by ¹H NMR and HPLC analysis to an authentic sample.

The invention has been described with reference to various specific and preferred embodiments and techniques. However, it should be understood that many variations and modifications will be obvious to those skilled in the art from the foregoing detailed description of the invention and may be made while remaining within the spirit and scope of the invention.

## Claims

1. A process for preparing a 2'-deoxynucleoside or 2'- substituted nucleoside that comprises:
a) obtaining an optionally protected 2,2'-anhydro-1-furanosyl-nucleoside;
b) reacting the 2,2'-aohydro-1-furanosyl-nucleoside from step (a) with a reducing agent and a sequestering agent to afford an optionally protected 2'-deoxynucleoside or 2'-substituted nucleoside; and
c) deprotecting the one or more protected hydroxyl groups, if necessary or desired.

2. The process of claim 1, wherein the optional protecting group is selected from the group consisting of trityl, silyl, or dimethoxytrityl.

3. The process of claim 2, wherein the optional protecting group is trityl.

4. The process of claim 2, wherein the optional protecting group is dimethoxytrityl.

5. The process of any one of claims 1-4, wherein in step (c), the deprotection occurs via the addition of an acid or acid resin at a temperature of about 50 °C.

6. The process of claim 1, wherein in step (b), the reducing agent is Red-Al.

7. The process of claim 1, wherein in step (b), the sequestering agent is 15-crown-5 ether.

8. The process of claim 1, wherein in step (b), the reaction is carried out in a polar solvent

9. The process of claim 8, wherein the polar solvent is THF and/or DME.

10. The process of claim 1, wherein in step (b), the reaction temperature is from about 0-5 °C.

11. A process for preparing a 2'-deoxynucleoside or 2'- substituted nucleoside that comprises:
a) optionally protecting one or more hydroxyl groups on a furanosyl ring by reaction with a protecting group;
b) condensing the furanosyl ring from step (a) with an optionally substituted natural or non-natural pyrimidine nucleoside base to form a nucleoside;
c) reacting the nucleoside from step (b) with a condensing agent to afford a 2,2'-anhydro-1-furanosyl-nucleoside;
d) reacting the 2,2'-anhydro-1-furanosyl-nucleoside from step (c) with a reducing agent and a sequestering agent to afford an optionally protected optionally protected 2'-deoxynucleoside or with an appropriate nucleophic reagent or organo-metallic to afford a 2'-substituted nucleoside; and
e) deprotecting the one or more protected hydroxyl groups, if necessary or desired.

12. The process of claim 11, wherein the optional protecting group is selected from the group consisting of trityl, silyl, or dimethoxytrityl.

13. The process of claim 12, wherein the optional protecting group is trityl.

14. The process of claim 12, wherein the optional protecting group is dimethoxytrityl.

15. The process of any one of claims 11-14, wherein in step (e), the deprotection occurs via the addition of an acid or acid resin at a temperature of about 50 °C.

16. The process of claim 11, wherein in step (b), the condensation occurs in the presence of a solvent and optionally a catalyst.

17. The process of claim 11, wherein in step (c), the condensing agent is a dialkyl or diaryl carbonate in the presence of a base and an organic solvent.

18. The process of claim 17, wherein the condensing agent is PhOCOOPh/NaHCO₃ and the organic solvent is DMF.

19. The process of claim 11, wherein in step (c), the reaction occurs at elevated temperatures.

20. The process of claim 19, wherein the temperature is from about 140-150 °C.

21. The process of claim 11, wherein in step (d), the reducing agent is Red-Al.

22. The process of claim 11, wherein in step (d), the sequestering agent is 15-crown-5 ether.

23. The process of claim 11, wherein in step (d), the reaction is carried out in a polar solvent.

24. The process of claim 23, wherein the polar solvent is THF and/or DME.

25. The process of claim 11, wherein in step (d), the reaction temperature is from about 0-5 °C.

26. The process of claim 11, wherein the furanosyl ring is an α- or β-, D- or L-arabinofuranosyl, xylofuranosyl, or ribofuranosyl ring.

27. A process for preparing a 2'-deoxythymidine that comprises:
a) optionally protecting one or more hydroxyl groups on a furanosyl ring by reaction with a protective group;
b) reacting the optionally protected furanosyl ring with cyanamide to form an optionally protected furanosylaminooxazoline;
c) reacting the optionally protected furanosylaminooxazoline with a cyclization or condensation agent to afford an optionally protected 2,2'-anhydro-1-furanosyl-thymidine;
d) reacting the optionally protected 2,2'-anhydro-1-furanosyl-thymidine with a reducing agent and a sequestering agent to provide an optionally protected, 2'-deoxythymidine; and
e) deprotecting the optionally protected 2'-deoxythymidine, if necessary or desired.

28. The process of claim 27, wherein the optional protecting group is selected from the group consisting of trityl, silyl, or dimethoxytrityl.

29. The process of claim 28, wherein the optional protecting group is trityl.

30. The process of claim 28, wherein the optional protecting group is dimethoxytrityl.

31. The process of any one of claims 27-30, wherein in step (e), the deprotection occurs via the addition of an acid or acid resin at a temperature of about 50 °C.

32. The process of claim 27, wherein in step (c), the cyclization or condensation agent is selected from the group consisting of:

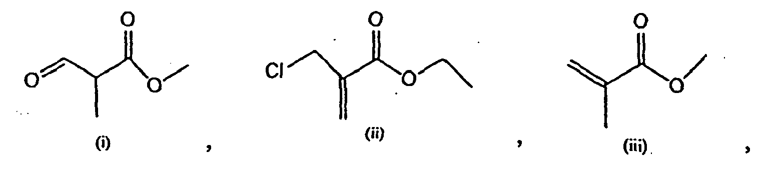

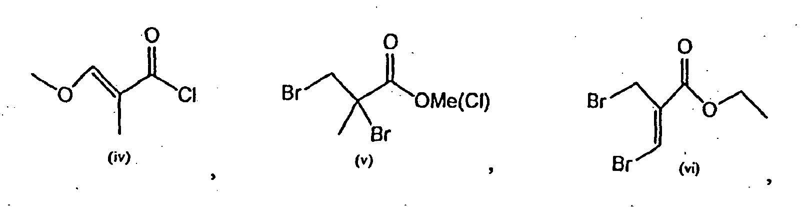

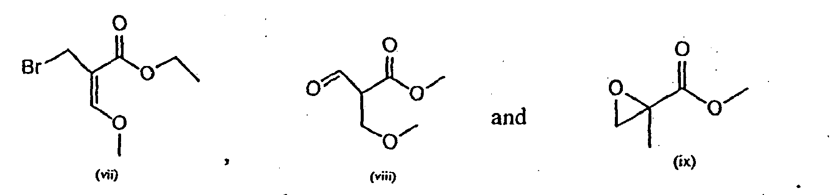

33. The process of claim 27, wherein in step (d), the reducing agent is Red-Al.

34. The process of claim 27, wherein in step (d), the sequestering agent is 15-crown-5 ether.

35. The process of claim 27, wherein in step (d), the reaction is carried out in a polar solvent.

36. The process of claim 35, wherein the polar solvent is THF and/or DME.

37. The process of claim 27, wherein in step (d), the reaction temperature is from about 0-5 °C.

38. The process of claim 27, wherein the furanosyl ring is an α- or β-, D- or L-arabinofuranosyl, xylofuranosyl, or ribofuranosyl ring.

39. A process for preparing a 2'-deoxythymidine that comprises:
a) optionally protecting one or more hydroxyl groups on a furanosyl ring by reaction with a protective group;
b) reacting the optionally protected furanosyl ring with cyanamide to form an optionally protected furanosylaminooxazoline;
c) reacting the optionally protected furanosylaminooxazoline with a cyclization or condensation agent to afford an optionally protected 2,2'-anhydro-1-furanosyl-thymidine;
d) reacting the optionally protected 2,2'-anhydro-1-furanosyl-thymidine with a reducing agent to provide an optionally protected, 2'-deoxythymidine; and
e) deprotecting the optionally protected 2'-deoxythymidine, if necessary or desired.

40. The process of claim 39, wherein the optional protecting group is selected from the group consisting of trityl, silyl, or dimethoxytrityl.

41. The process of claim 40, wherein the optional protecting group is trityl.

42. The process of claim 40, wherein the optional protecting group is dimethoxytrityl.

43. The process of claim 39, wherein in step (e), the deprotection occurs via the addition of an acid or acid resin at a temperature of about 50 °C.

44. A process for preparing an intermediate of Formula (B) comprising reducing a lactone of Formula (A) with Red-Al to obtain a compound of Formula (B):

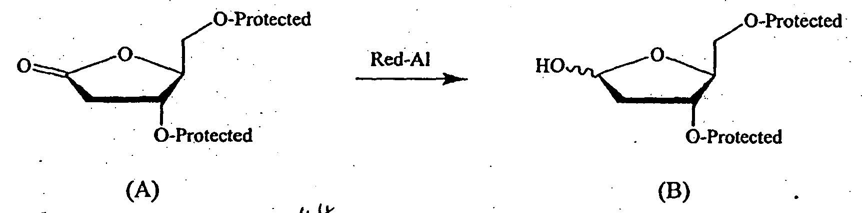

45. The process of claim 44, wherein the oxygen protecting groups are toluoyl.

46. A process for preparing an intermediate of Formula (F) comprising
a) reacting an optionally protected alcohol of Formula (C) with mesyl chloride to obtain a mesylate of Formula (D), 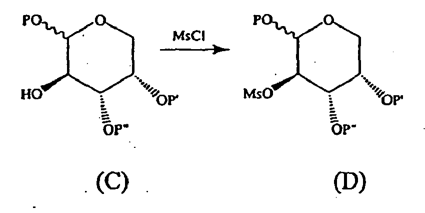 wherein P, P', and P" are hydrogen, alkyl, or a suitable oxygen protecting group;
b) reducing the compound of Formula (D) to obtain a compound of Formula (E), 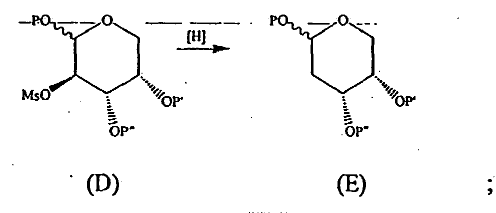 and
c) deprotecting if necessary to obtain a compound of Formula (F)

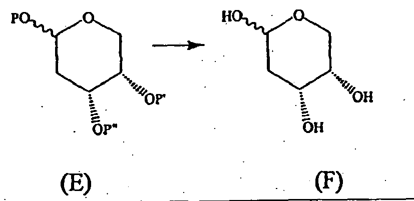

47. A process for preparing an intermediate of Formula (F) comprising
a) reacting an optionally protected alcohol of Formula (C') with a mesylate to obtain a mesylate of Formula (D'), 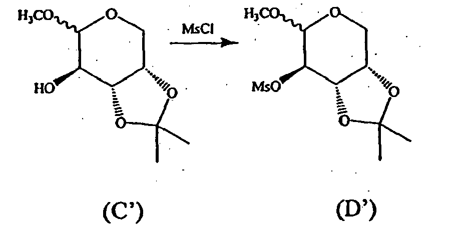 wherein P, P', and P" are hydrogen, alkyl, or a suitable oxygen protecting group;
b) reducing the compound of Formula (D') to obtain a compound of Formula (E'), 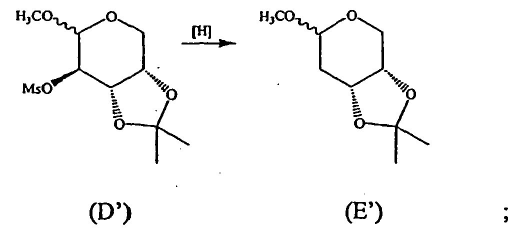 and
c) deprotecting the compound for Formula (E') to obtain a compound of Formula (F)

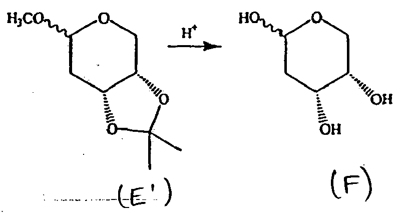

48. A process for preparing an intermediate of Formula (H) comprising reacting an alcohol of Formula (G) with an acid to obtain an intermediate of formula (H):

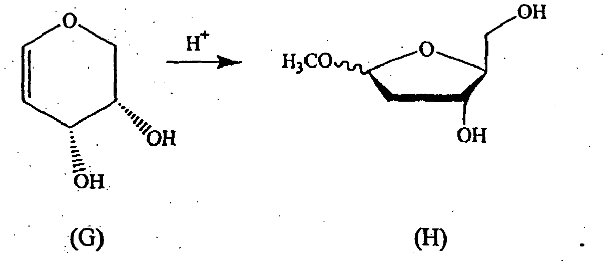

49. A process for preparing an intermediate of Formula (J) comprising reacting an alcohol of Formula (I) with an oxidizing agent, OsO₄, to obtain an intermediate of formula (J):

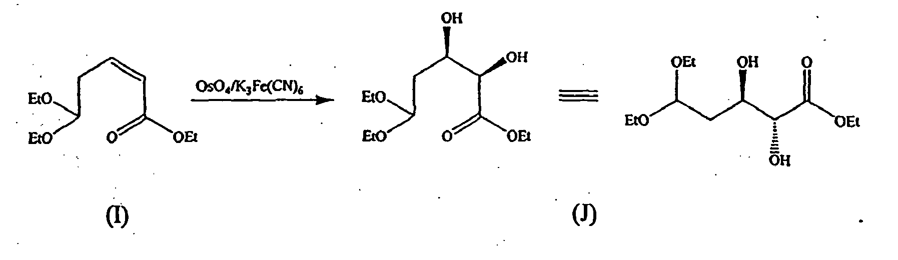

50. A process for preparing an intermediate of Formula (Q) comprising
a) reducing an ester of Formula (K) with an reducing agent, DIBAL to obtain an aldehyde of formula (L): 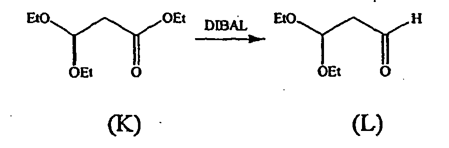
b) reacting the aldehyde of Formula (L) with a phosphate (i), followed by reduction with a reducing agent, DIBAL, to obtain an alkene of formula (M): 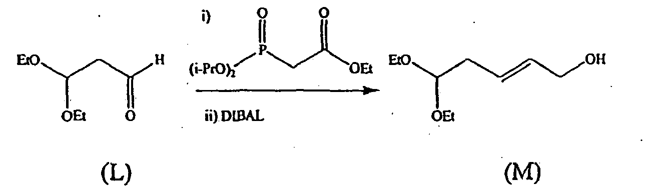
c) reacting the alkene of Formula (M) with an oxidizing agent, Ti(OPr)₄ (+) DET, to obtain an epoxide of formula (N): 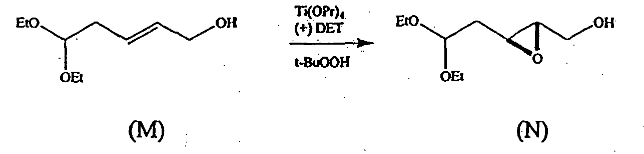
d) optionally protecting the free alcohol in the epoxide of formula (N) to obtain an optionally protected epoxide of formula (O); 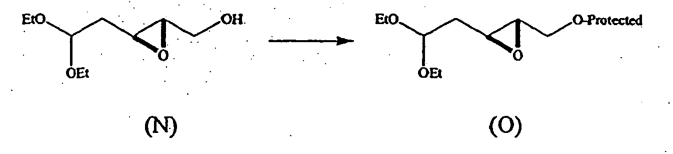
e) reacting the optionally protected epoxide of formula (O) with an acid to form a diol of formula (P); 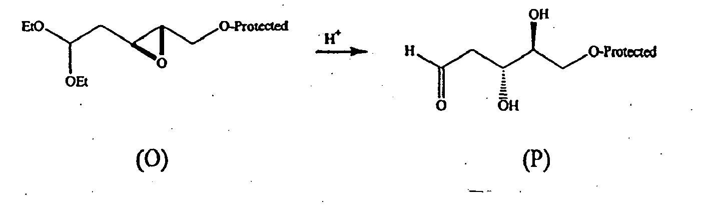
f) cyclizing the diol of formula (P) to form intermediate (Q);

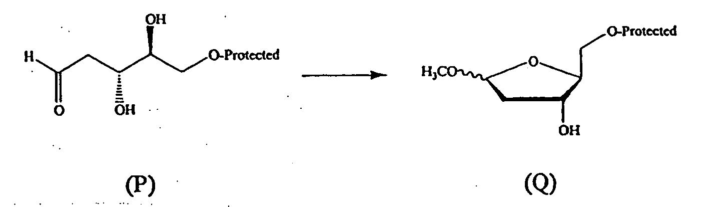

51. A process for preparing an intermediate of Formula (S) comprising reacting an carboxylic acid of Formula (R) with NaNO₂ and HCl, to obtain an intermediate of formula (S):

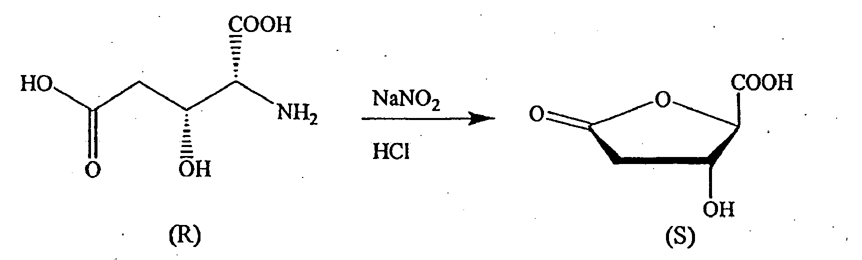

52. A process for preparing an intermediate of Formula (U) comprising reacting an sugar of Formula (T) with methanol in acid, to obtain an intermediate of formula (U)

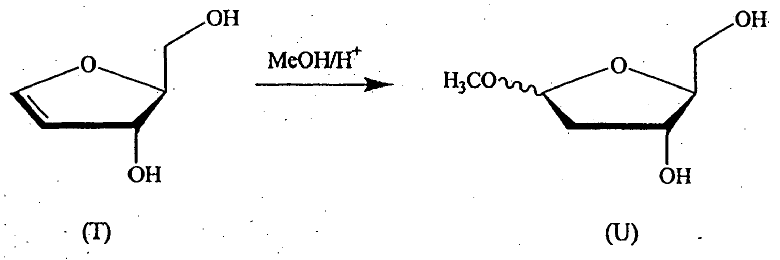

53. A process for preparing an intermediate of Formula (U) comprising reacting an sugar of Formula (V) with methanol in acid, to obtain an intermediate of formula (U)

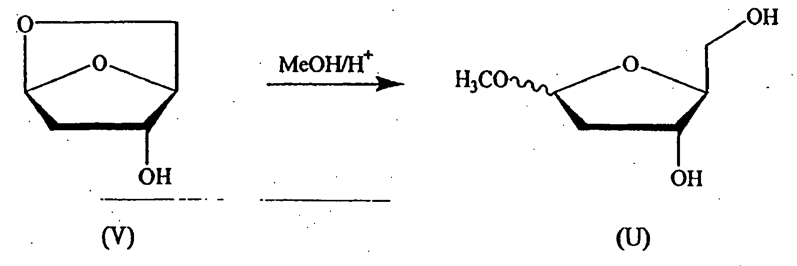

54. A process for preparing a nucleoside, nucleoside analog, or a pharmaceutically acceptable salt or prodrug thereof, comprising;
a) reacting D-xylose in the presence of bromine/water to form a 1,4-lactone;
b) reacting the 1,4-lactone from step a) with HBr/acetic acid to provide 2,5-dibromo-2,5-dideoxy-D-lyxono-1,4-lactone;
c) treating the 2,5-dibromo-2,5-dideoxy-D-lyxono-1,4-lactone from step b) with KI in TFA to produce 5-iodo-2-deoxylactone;
d) reacting the 5-iodo-2-deoxcylactone from step c) with aqueous KOH to provide 4,5-epoxy-3-hydroxy-butyl-potassium ester;
e) treating the ester compound product of step d) with aqueous acid to provide 2-deoxy L-ribonolactone;
f) reducing the 2-deoxy L-ribonolactone from step e) with Red-A1 to provide the corresponding lactol;
g) reacting the lactol from step f) with toluoyl chlorine and TEA to produce 1-, 3-, 5-tri-O-toluoyl-2-deoxy-ribofuranose;
h) reacting 1-, 3-, 5-tri-O-toluoyl-2-deoxy-ribofuranose from step g) with HCI to provide 1-chloro-2-deoxy-3-, 5-di-O-tohioyl-ribofuranose;
i) reacting the 1-chloro-3-, 5-di-O-toluoyl-ribofuranose from step h) with a nucleoside base in the presence of HMDS to produce 1-nucleoside base-2'-deoxy-3'-, 5'-di-O-toluoyl-ribofuranose; and
j) deprotecting the 3'-, 5'-di-O-toluoyl substituents on the product of step i), by treating the 1-nucleoside base-2'-deoxy-3'-, 5'-di-O-toluoyl-ribofuranose from step i) with NaOMe, thereby producing a final product nucleoside.

55. The process of claim 54, wherein the nucleoside is a β-D or β-L 2'-deoxy-ribonucleoside.

56. The process of claim 55, wherein the nucleoside is β-L 2'-deoxy-thymidine.

57. The process ot claim 39, wherein in step (c), the cyclization or condensation agent is selected from the group consisting of:

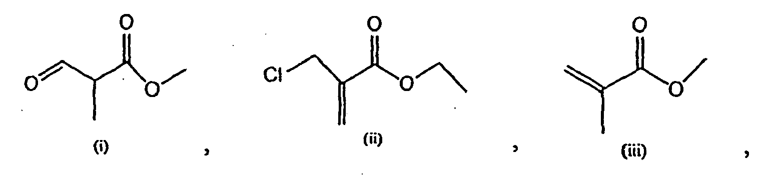

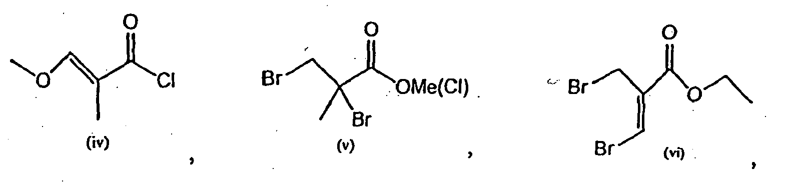

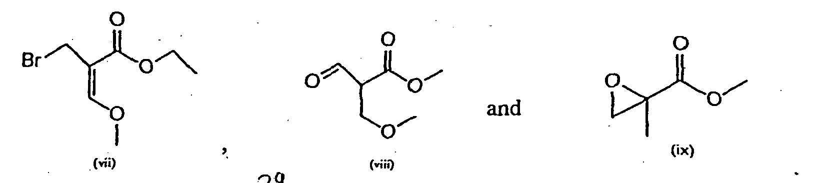

58. The process of claim 39, wherein in step (d), the reducing agent is Red-Al.

59. The process of claim 39, wherein in step (d), the reaction is carried out in a polar solvent.

60. The process of claim 39, wherein the polar solvent is THF and/or DME.

61. The process of claim 39, wherein in step (d), the reaction temperature is from about 0-5 °C:

62. the process of claim 39, wherein the furanosyl ring is an α- or β-, D- or L-arabinofuranosyl, xylofuranosyl, or ribofuranosyl ring.
